(19) Europäisches Patentamt
European Patent Office
Office européen des brevets

(11) EP 3 431 474 A1

(12) **EUROPEAN PATENT APPLICATION**

(43) Date of publication:
**23.01.2019 Bulletin 2019/04**

(21) Application number: **17305973.4**

(22) Date of filing: **21.07.2017**

(51) Int Cl.:
***C07D 417/12*** *(2006.01)* ***C07D 277/30*** *(2006.01)*
***A61K 31/426*** *(2006.01)* ***A61K 31/427*** *(2006.01)*
***A61P 31/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Antabio SAS**
**31670 Labège (FR)**

(72) Inventor: **The designation of the inventor has not yet been filed**

(74) Representative: **J A Kemp**
**14 South Square**
**Gray's Inn**
**London WC1R 5JJ (GB)**

Remarks:
Claims 16-30 are deemed to be abandoned due to non-payment of the claims fees (Rule 45(3) EPC).

(54) **CHEMICAL COMPOUNDS**

(57) The invention relates to a compound which is a thiazole derivative of Formula (I), or a pharmaceutically acceptable salt thereof,

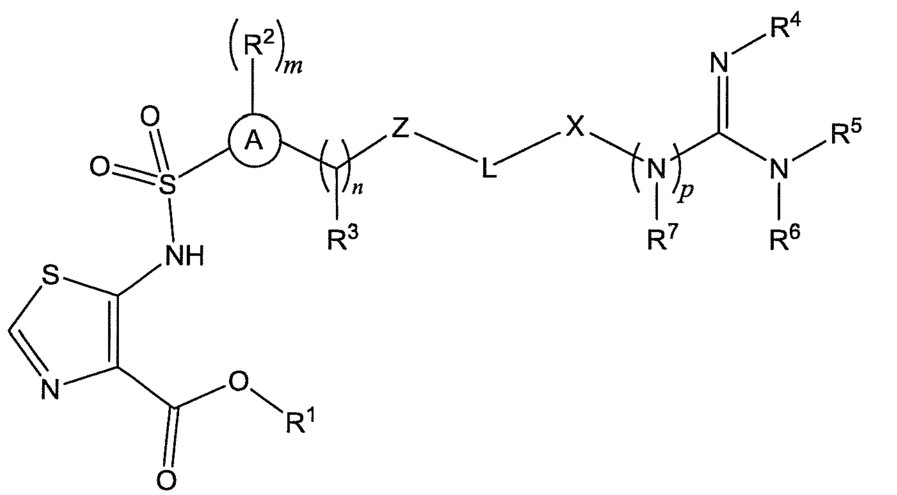

[FORMULA (I)]

wherein $R^1$, $R^2$, $R^3$, $R^4$, $R^5$, $R^6$, $R^7$, Ⓐ, Z, L, X, *m*, *n* and *p* are as defined herein. The compounds are useful in the treatment and prevention of bacterial infection.

EP 3 431 474 A1

## Description

### Field of the Invention

The present invention relates to compounds which are thiazole derivatives. The compounds of the invention find use in the prevention or treatment of bacterial infection. The invention also provides such compounds *per se* and pharmaceutical compositions comprising such compounds. The compounds of the invention are useful as inhibitors of metallo-β-lactamase (MBL) enzymes.

### Background

Bacteria in both clinical and non-clinical settings are becoming increasingly resistant to conventional antibiotics, and this resistance is becoming a serious clinical and epidemiological problem for human health. In Gram-negative bacteria, resistance to antibiotics often arises from the production by the organism of β-lactamases, especially metallo-β-lactamases (MBL).

MBL are resistance determinants of increasing clinical relevance. In fact, because of their broad range, potent carbapenemase activity and resistance to inhibitors, these enzymes can confer resistance to almost all β-lactam antibiotics.

MBLs were first detected in the mid-1960s as carried by mobile DNA elements in species with only low pathogenic potential. However, genes encoding MBL spread among major Gram-negative bacteria during the 1990s and this has led to a health crisis arising from the international dissemination of carbapenem-resistant *Enterobacteriaceae* producing the VIM-type and NDM-type metallo-β-lactamases.

Functional features of these *Enterobacteriaceae* include potent carbapenemase activity and resistance to clinical β-lactamase inhibitors (clavulanate and sulfones). The activity against β-lactams differs between the different metallo-β-lactamases, and substrate specificity might vary from a narrow range (eg, the CphA metallo-β-lactamase of *Aeromonas hydrophila*), to an extended range (eg, the VIM-type metallo-β-lactamases, which can degrade almost all classes of β-lactams apart from the monobactams).

There are three major structural subclasses of MBL which share substantial internal diversity. Members of the different subclasses differ not only in their high degree of sequence diversity, but also in the structure of their active sites. In enzymes of subclasses B1 and B3, the active site contains two zinc ions; in members of subclass B2, the active site contains only one zinc ion.

Acquired metallo-β-lactamases have been detected in strains of *Enterobacteriaceae, Pseudomonas aeruginosa, Acinetobacter baumannii,* and other Gram-negative bacteria. Among acquired MBL, almost all the enzymes belong to subclass B1, which indicates an overall higher propensity for members of this subclass to be captured and spread with mobile genetic elements than for members of subclasses B2 and B3.

As an example, the subclass B1 comprises the IMP-type, the VIM-type, and the NDM-type enzymes.

The IMP-type enzymes, including IMP-1, were first detected in Japan in the late 1980s, and have since been reported worldwide in *Enterobacteriaceae* and in Gram-negative bacteria. The IMP-type enzymes have broad substrate specificity with a high affinity for cephalosporins and carbapenems, but they have little activity against Temocillin.

The VIM-type enzymes, including VIM-2, were first discovered in Europe in the late 1990s and have since been reported worldwide. VIM-type enzymes were initially detected in *P. aeruginosa* and in other Gram-negative bacteria, but have since emerged in *Enterobacteriaceae,* and have become a major problem in some settings. More than 20 different VIM allotypes are known, each with a defined geographical distribution except for VIM-1 and VIM-2, which share a broader distribution than the IMP-type enzymes. The VIM-type metallo-β-lactamases show even broader substrate specificities than the IMP-types, being able to hydrolyse 6-α-methoxy-penicillins. Furthermore, the VIM-type enzymes are unique in the metallo-β-lactamases in that they have a high affinity for carbapenems.

New Delhi metallo-β-lactamase 1 (NDM-1) is a novel metallo-β-lactamase identified initially in a patient hospitalized in New Delhi with an infection caused by *Klebsiella pneumoniae.* Subsequently, organisms in the *Enterobacteriaceae* family containing this new β-lactamase have been found widely distributed throughout India, Pakistan, and Bangladesh and are now occurring in the United Kingdom and in many other countries. The New Delhi metallo-β-lactamase 1 (NDM-1) is a polypeptide of 158 amino acids in length (Accession number AB571289) capable of hydrolyzing a wide range of β-lactam antibiotics including penicillins, cephalosporins and carbapenem antibiotics that are a mainstay for the treatment of antibiotic-resistant bacterial infections.

Accordingly, there is an urgent need for new antibacterial compounds and compositions and adjuvant therapies for treating bacterial infection, in particular bacterial infection caused by bacteria which express MBL enzymes.

## Summary of the Invention

Previously, the inventors reported in WO 2014/198849 that certain thiazole derivatives are inhibitors of metallo-β-lactamases, including NDM-1. The inventors have now surprisingly found that compounds of Formula (I) are potent inhibitors of metallo-β-lactamases, including NDM-1, and have improved properties compared to the compounds disclosed in WO 2014/198849. The compounds therefor are useful in treating and preventing bacterial infection, for example by use in combination with β-lactam antibiotics.

Accordingly, the invention provides a compound which is a thiazole derivative according to Formula (I), or a pharmaceutically acceptable salt thereof,

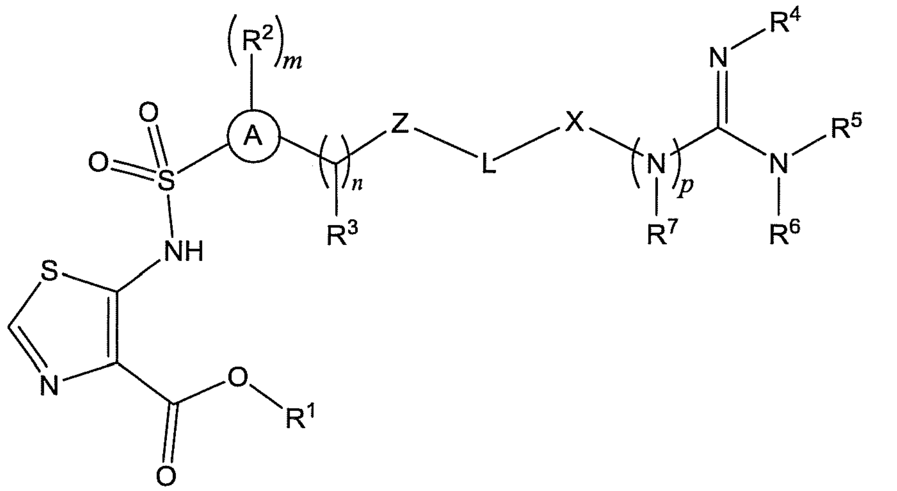

[FORMULA (I)]

wherein

◦ $R^1$ is selected from H, $R^{1a}$ and $-CH_2OC(O)R^{1a}$, wherein $R^{1a}$ is selected from an unsubstituted $C_1$ to $C_4$ alkyl group and phenyl;

◦ Ⓐ is a cyclic group selected from $C_6$ to $C_{10}$ aryl, 5- to 10-membered heteroaryl, and 4- to 10- membered carbocyclic and heterocyclic groups;

◦ each $R^2$ is independently selected from:

(i) halo or $R^8$;
(ii) $C_{1-3}$ alkyl, $O(C_{1-3}$ alkyl), $S(C_{1-3}$ alkyl), $SO(C_{1-3}$ alkyl) or $SO_2(C_{1-3}$ alkyl), any of which may optionally be substituted with 1, 2 or 3 halo substituents and/or one $R^8$ substituent; and
(iii) $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;
and

- each $R^8$ is independently selected from CN, OH, $-C(O)NR^fR^g$, $-NR^fR^g$, $-NR^{10}C(NR^{11})R^{12}$, $-C(NR^{10})NR^{11}R^{12}$, and $-NR^{10}C(NR^{11})NR^{12}R^{13}$; wherein each of $R^f$ and $R^g$ is independently H or unsubstituted $C_{1-2}$ alkyl;

◦ *m* is 0, 1, 2 or 3

◦ $R^3$ is selected from hydrogen and a $C_1$ to $C_3$ alkyl group which is unsubstituted or is substituted with 1, 2 or 3 substituents selected from halogen, $-OR^{10}$, and $-NR^{10}R^{11}$;

◦ *n* is 0 or 1

◦ Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$,

$-NR^{10}C(O)S-$, $-SC(O)NR^{10}$, $-NR^{10}C(NR^{11})-$, $-C(NR^{10})NR^{11}-$, $-NR^{10}C(NR^{11})NR^{12}-$, $-NR^{10}C(N^+R^{11}R^{12})-$, $-C(N^+R^{10}R^{11})NR^{12}-$, $-NR^{10}C(N^+R^{11}R^{12})NR^{13}-$, $-NR^{10}C(NR^{11})O-$, $-OC(NR^{10})NR^{11}$, $-NR^{10}C(N^+R^{11}R^{12})O-$, $-OC(N^+R^{10}R^{11})NR^{12}-$, $-NR^{10}C(NR^{11})S-$, $-SC(NR^{10})NR^{11}$ $-NR^{10}C(N^+R^{11}R^{12})S-$, and $-SC(N^+R^{10}R^{11})NR^{12-}$;

◦ L is a bond or is selected from $C_{1-4}$ alkylene, $C_{2-4}$ alkenylene, $C_{2-4}$ alkynylene, $C_{1-3}$ alkylene-($C_{3-6}$cycloalkylene)-$C_{1-3}$ alkylene, $C_{1-4}$ alkylene-($C_{3-6}$cycloalkylene) and ($C_{3-6}$cycloalkylene)-$C_{1-4}$ alkylene, wherein L is unsubstituted or is substituted with 1 or 2 substituents selected from halogen, $-OR^{10}$, and $-NR^{10}R^{11}$; or L is $-C(R^{10})=N-$;

◦ X is a bond or, when L is other than a bond or $-C(R^{10})=N-$, X is a bond or is selected from $-NR^{10}-$, -O-, $-NR^{10}C(NR^{11})-$, and $-C(NR^{10})-$;

◦ *p* is 0 or 1;

◦ $R^4$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;
or $R^4$ is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ $R^5$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;
or $R^5$ is joined together with $R^4$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;
or $R^5$ is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ $R^6$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;
or $R^6$ is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;
or $R^6$ is joined together with $R^7$ if present to form, together with the atoms to which they are attached, a 5- to 6-membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ $R^7$ if present is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;
or $R^7$ is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ each $R^{10}$, $R^{11}$, $R^{12}$, $R^{13}$ and $R^{14}$ is independently H or methyl.

The present invention also provides a pharmaceutical composition comprising a compound as described herein and optionally further comprising an antibiotic agent. Also provided is a product comprising a compound as described herein in combination with an antibiotic agent.

The invention also provides a compound as described herein for use in the treatment or prevention of bacterial infection in a subject in need thereof. Also provided is a method for treating or preventing bacterial infection in a subject, which method comprises administering to said subject an effective amount of a compound as described herein. Further provided is the use of a compound as described herein in the manufacture of a medicament for use in treating or

preventing bacterial infection in a subject.

## DETAILED DESCRIPTION OF THE INVENTION

*Definitions*

As used herein, a $C_1$ to $C_4$ alkyl group is a linear or branched alkyl group containing from 1 to 4 carbon atoms. A $C_1$ to $C_4$ alkyl group is often a $C_1$ to $C_3$ alkyl group. Examples of $C_1$ to $C_4$ alkyl groups include methyl, ethyl, n-propyl, iso-propyl, n-butyl, iso-butyl, sec-butyl, and tert-butyl. A $C_1$ to $C_3$ alkyl group is typically a $C_1$ to $C_2$ alkyl group. A $C_1$ to $C_2$ alkyl group is methyl or ethyl, typically methyl. For the avoidance of doubt, where two alkyl groups are present, the alkyl groups may be the same or different.

As used herein, a $C_2$-$C_4$ alkenyl group is a linear or branched alkenyl group containing from 2 to 4 carbon atoms and having one or more, e.g. one or two, typically one double bonds. Typically a $C_2$-$C_4$ alkenyl group is a $C_2$-$C_3$ alkenyl group. Examples of $C_2$-$C_4$ alkenyl groups include ethenyl, propenyl and butenyl. For the avoidance of doubt, where two alkenyl groups are present, the alkenyl groups may be the same or different.

As used herein, a $C_2$-$C_4$ alkynyl group is a linear or branched alkynyl group containing from 2 to 4 carbon atoms and having one or more, e.g. one or two, typically one triple bonds. Typically a $C_2$-$C_4$ alkynyl group is a $C_2$-$C_3$ alkynyl group. Examples of $C_2$ to $C_4$ alkynyl groups include ethynyl, propynyl and butynyl. For the avoidance of doubt, where two alkynyl groups are present, the alkynyl groups may be the same or different.

As used herein, a $C_1$ to $C_4$ alkylene group is an unsubstituted or substituted bidentate moiety obtained by removing two hydrogen atoms from a $C_1$ to $C_4$ alkane. The two hydrogen atoms may be removed from the same carbon atom or from different carbon atoms. Typically a $C_1$ to $C_4$ alkylene group is a $C_1$ to $C_3$ alkylene group. Examples of $C_1$ to $C_4$ alkylene groups include methylene, ethylene, n-propylene, iso-propylene, n-butylene, sec-butylene and tert-butylene. A $C_1$ to $C_4$ alkylene group is typically a $C_1$ to $C_2$ alkylene group. A $C_1$ to $C_2$ alkyl group is methylene or ethylene, typically methylene. For the avoidance of doubt, where two alkylene groups are present, the alkylene groups may be the same or different.

As used herein, a $C_2$ to $C_4$ alkenylene group is an unsubstituted or substituted bidentate moiety obtained by removing two hydrogen atoms from a $C_2$ to $C_4$ alkene. The two hydrogen atoms may be removed from the same carbon atom or from different carbon atoms. Typically a $C_2$ to $C_4$ alkenylene group is a $C_2$ to $C_3$ alkenylene group. Examples of $C_2$ to $C_4$ alkenylene groups include ethenylene, n-propenylene, iso-propenylene, n-butenylene, sec-butenylene and tert-butenylene. A $C_2$ to $C_3$ alkenylene group is typically a $C_2$ alkenylene, i.e. ethenylene. For the avoidance of doubt, where two alkenylene groups are present, the alkenylene groups may be the same or different.

As used herein, a $C_2$ to $C_4$ alkynylene group is an unsubstituted or substituted bidentate moiety obtained by removing two hydrogen atoms from a $C_2$ to $C_4$ alkyne. The two hydrogen atoms may be removed from the same carbon atom or from different carbon atoms. Typically a $C_2$ to $C_4$ alkynylene group is a $C_2$ to $C_3$ alkynylene group. Examples of $C_2$ to $C_4$ alkynylene groups include ethynylene, n-propynylene, iso-propynylene, n-butynylene, sec-butynylene and tert-butynylene. A $C_2$ to $C_3$ alkynylene group is typically a $C_2$ alkynylene, i.e. ethynylene. For the avoidance of doubt, where two alkynylene groups are present, the alkynylene groups may be the same or different.

An alkyl, alkenyl, alkynyl, alkylene, alkenylene or alkynylene group as used herein may be unsubstituted or substituted. Unless otherwise stated, substituted alkyl, alkenyl or alkynyl groups typically carry one or more, e.g. 1, 2, 3 or 4, such as one, two or three e.g. one, or two, e.g. one substituent selected from halogen, -CN, -$R^8$, -$OR^{10}$, and -$NR^{10}R^{11}$, wherein $R^{10}$ and $R^{11}$ are as defined herein. The substituents on a substituted alkyl, alkenyl or alkynyl group are typically themselves unsubstituted unless otherwise stated. Where more than one substituent is present, these may be the same or different.

As used herein, a halogen is typically chlorine, fluorine, bromine or iodine and is preferably chlorine, bromine or fluorine, especially chorine or fluorine, especially fluorine.

A 3- to 10- membered carbocyclic group is a cyclic hydrocarbon containing from 3 to 10 carbon atoms. A carbocyclic group may be saturated or partially unsaturated, but is typically saturated. A 3- to 10- membered partially unsaturated carbocyclic group is a cyclic hydrocarbon containing from 3 to 10 carbon atoms and containing 1 or 2, e.g. 1 double bond. A 3- to 10- membered carbocyclic group is typically a 4- to 10- membered carbocyclic group. Often, a 3- to 10- membered carbocyclic group is a 3- to 6- membered carbocyclic group, such as a 4- to 6- membered or 5- to 6- membered carbocyclic group. A 3- to 10- membered carbocyclic group may be a fused bicyclic group, as defined herein. A 3- to 10- membered carbocyclic group may be a saturated 4- to 6-membered, preferably 5- or 6- membered carbocyclic group. Examples of 3- to 6- membered saturated carbocyclic groups include cyclopropyl, cyclobutyl, cyclopentyl and cyclohexyl groups.

A 3- to 10- membered heterocyclic group is a cyclic group containing from 3 to 10 atoms selected from C, O, N and S in the ring, including at least one heteroatom, and typically one or two heteroatoms. The heteroatom or heteroatoms are typically selected from O, N, and S, most typically from S and N, especially N. For example, where the

heterocyclic group is denoted a nitrogen-containing heterocyclic group, it contains one nitrogen atom and optionally a further heteroatom selected from O, N and S. A heterocyclic group may be saturated or partially unsaturated. A 3- to 10- membered partially unsaturated heterocyclic group is a cyclic group containing from 3 to 10 atoms selected from C, O, N and S in the ring and containing 1 or 2, e.g. 1 double bond.

A 3- to 10- membered heterocyclic group is typically a 4- to 10- membered heterocyclic group. Sometimes a 3- to 10- membered heterocyclic group is a 3- to 6- membered heterocyclic group, such as a monocyclic 4- to 6- membered heterocyclic group or a monocyclic 5- or 6- membered heterocyclic group. Alternatively, a 3- to 10- membered heterocyclic group may be a 9- or 10- membered fused bicyclic heterocyclic group (i.e. a fused heterobicyclic group).

Examples of 5- and 6- membered saturated heterocyclic groups include piperazine, piperidine, morpholine, 1,3-oxazinane, pyrrolidine, imidazolidine, and oxazolidine, including quaternised derivatives thereof, as defined herein. Examples of 5- and 6-membered partially saturated heterocyclic groups include tetrahydropyrazine, tetrahydropyridine, dihydro-1,4-oxazine, tetrahydropyrimidine, dihydro-1,3-oxazine, dihydropyrrole, dihydroimidazole and dihydrooxazole, including quaternised derivatives thereof, as defined herein.

Examples of 9- and 10- membered fused heterobicyclic groups include 9-membered fused heterobicyclic groups such as indoline, 2,3-dihydrobenzofuran, 2,3-dihydrobenzo[b]thiophene, 2,3-dihydro-1H-benzo[d]imidazole, 2,3-dihydrobenzo[d]oxazole, 2,3-dihydrobenzo[d]thiazole, benzo[d][1,3]dioxole, 4,5,6,7-tetrahydrothiazolo[5,4-c]pyridine and 4,5,6,7-tetrahydrothiazolo[4,5-c]pyridine, including quaternised derivatives thereof, as defined herein; and 10-membered heterobicyclic groups such as 1,2,3,4-tetrahydroquinoline, 1,2,3,4-tetrahydroisoquinoline, chromane, isochromane, thiochromane, isothiochromane, 1,2,3,4-tetrahydroquinoxaline, 1,2,3,4-tetrahydroquinazoline, 1,4-dihydro-2H-benzo[d][1,3]oxazine, 3,4-dihydro-2H-benzo[b][1,4]oxazine, 3,4-dihydro-2H-benzo[b][1,4]thiazine, 1,4-dihydro-2H-benzo[d][1,3]thiazine, 4H-benzo[d][1,3]dioxine and 2,3-dihydrobenzo[b][1,4]dioxine, including quaternised derivatives thereof. Often, the fused heterobicyclic group comprises 1, 2 or 3, preferably 1 or 2 nitrogen atoms.

For the avoidance of doubt, references to a heterocyclic group also include fused polycyclic ring systems, including for instance fused bicyclic systems in which a heterocyclic group is fused to an aryl group. When the heterocyclic group is such a fused heterocyclic group, preferred examples are fused ring systems wherein a 5- to 6-membered heterocyclic group is fused to a phenyl group.

As used herein, a $C_3$ to $C_6$ cycloalkylene group (also referred to as a $C_{3-6}$cycloalkylene group) is an unsubstituted or substituted bidentate moiety obtained by removing two hydrogen atoms from a saturated $C_3$ to $C_6$ carbocyclic group as defined herein. The two hydrogen atoms may be removed from the same carbon atom or from different carbon atoms. Examples of $C_{3-6}$cycloalkylene groups include cyclopropylene, cyclobutylene, cyclopentylene and cyclohexylene.

As used herein, a $C_6$ to $C_{10}$ aryl group is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 10- membered heteroaryl group is a substituted or unsubstituted monocyclic or fused polycyclic aromatic group containing from 5 to 10 atoms in the ring portion, including at least one heteroatom, for example 1, 2 or 3 heteroatoms, typically selected from O, S and N. A heteroaryl group is typically a 5- or 6-membered heteroaryl group or a 9- or 10- membered heteroaryl group, preferably a 5- or 6- membered heteroaryl group. Preferably, the heteroaryl group comprises 1, 2 or 3, preferably 1 or 2 nitrogen atoms.

Examples of 5- and 6- membered heteroaryl groups include pyrrole, furan, thiophene, imidazole, oxazole, thiazole, pyridine, pyridazine, pyrimidine, and pyrazine. Examples of 9- and 10- membered heteroaryl groups include 9-membered heteroaryl groups such as indole, benzothiophene, benzofuran, benzoxazole, benzothiazole, benzimidazole, imidazo[1,2-a]pyridine, [1,2,4]triazolo[1,5-a]pyridine and imidazo[1,2-a]pyrazine, including quaternised derivatives thereof; and 10-membered heteroaryl groups such as quinoline, isoquinoline, quinazoline, and quinoxaline.

For the avoidance of doubt, references to a heteroaryl group also include fused polycyclic ring systems, including for instance fused bicyclic systems in which a heteroaryl group is fused to an aryl group. When the heteroaryl group is such a fused heteroaryl group, preferred examples are fused ring systems wherein a 5- to 6-membered heteroaryl group is fused to a phenyl group.

As used herein, a fused bicyclic group is a group comprising two cyclic moieties sharing a common bond between two atoms.

A carbocyclic, heterocyclic, aryl or heteroaryl group may be unsubstituted or substituted as described herein. For example, a carbocyclic, heterocyclic, aryl or heteroaryl group may be unsubstituted or substituted with 1, 2 or 3, typically 1 or 2 such as e.g. 1 substituent. Suitable substituents include, halogen; -CN; $OR^{10}$ and $-NR^{10}R^{11}$ (wherein $R^{10}$ and $R^{11}$ are as defined herein) unsubstituted $C_1$ to $C_2$ alkyl and $R^2$ as depicted in Formula (I) and defined herein. The substituents on a substituted carbocyclic, heterocyclic, aryl or heteroaryl group are typically themselves unsubstituted, unless otherwise stated.

Compounds of the invention may comprise heterocyclic or heteroaryl groups comprising at least one nitrogen atom. In such compounds, said nitrogen atom(s) are independently selected from secondary, tertiary and quaternary nitrogen atom(s). A quaternary nitrogen atom is present when the compound comprises a quaternised derivative of one

or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

As used herein, a pharmaceutically acceptable salt is a salt with a pharmaceutically acceptable acid or base. Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic or *p*-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts.

In Formula (I), the stereochemistry is not limited. In particular, compounds of Formula (I) containing one or more chiral centre may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound according to Formula (I) which is enantiomerically or diasteriomerically pure. Typically, a compound of the invention comprises by weight at least 60%, such as at least 75%, 90%, or 95% of a single enantiomer or diastereomer. Preferably, the compound is substantially optically pure.

For the avoidance of doubt, the terms 'thiazole derivative' and 'thiazolyl derivative' may be used interchangeably and unless otherwise indicated refer to compounds of the invention, such as compounds of Formula (I).

*Compounds of the Invention*

Typically, in Formula (I), $R^1$ is selected from H and $R^{1a}$. More preferably, $R^1$ is H. $R^{1a}$ is typically an unsubstituted $C_1$ to $C_4$ alkyl group, such as an unsubstituted $C_1$ to $C_2$ alkyl group. More preferably, $R^{1a}$ is methyl or t-butyl.

In Formula (I), Ⓐ is preferably a cyclic group selected from phenyl, 5- to 6-membered heteroaryl, and 5- to 6-membered carbocyclic and heterocyclic groups. Ⓐ is more preferably selected from phenyl and 5- to 6-membered heteroaryl groups. Still more preferably, Ⓐ is a phenyl.

When Ⓐ is a 5- to 10-membered heteroaryl group, it is preferably a 5- or 6- membered group. When Ⓐ is a 4- to 10-membered heterocyclic or carbocyclic group, it is preferably a 5- or 6- membered group. When Ⓐ is a heterocyclic or heteroaryl group, it preferably contains 1, 2 or 3, preferably 1 or 2 heteroatoms selected from O, N and S. When Ⓐ is a heterocyclic or heteroaryl group, it is preferably a nitrogen-containing group. When Ⓐ is a fused heteroaryl or heterocyclic group, Ⓐ preferably comprises a benzene ring fused to a 5- or 6- membered heterocyclic or heteroaryl group as defined herein.

Preferably, Ⓐ is selected from phenyl, cyclohexane, piperidine, pyridazine, pyridine and thiazole. More preferably, Ⓐ is selected from phenyl, pyridazine, pyridine and thiazole. Still more preferably, Ⓐ is phenyl.

In Formula (I), each $R^2$ is independently selected from:

(i) halo or $R^8$;
(ii) $C_{1-3}$ alkyl, $O(C_{1-3}$ alkyl), $S(C_{1-3}$ alkyl), $SO(C_{1-3}$ alkyl) or $SO_2(C_{1-3}$alkyl), any of which may optionally be substituted with 1, 2 or 3 halo substituents and/or one $R^8$ substituent; and
(iii) $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;

wherein $R^8$ is as defined herein.

When an $R^2$ group is according to option (i) above, preferably the group is a halo group. Fluorine is preferred.

When an $R^2$ group is according to option (ii) above, preferably the $C_{1-3}$ alkyl group in the $R^2$ moiety is a $C_{1-2}$ alkyl group, more preferably a $C_1$ alkyl (methyl) group. The $R^2$ group is preferably selected from $C_{1-2}$ alkyl, $O(C_{1-2}$ alkyl), $S(C_{1-2}$ alkyl) and $SO(C_{1-2}$ alkyl), more preferably from $C_{1-2}$ alkyl and $O(C_{1-2}$ alkyl), each of which may be unsubstituted or substituted as described above. When an $R^2$ group is according to option (ii) above, the $R^2$ group may optionally be substituted with 1, 2 or 3 halo substituents and/or one $R^8$ substituent; preferably with either 1, 2 or 3 halo substituents (of which one or more, preferably all are fluorine) or with one $R^8$ substituent. Preferred $R^2$ groups according to option (ii) above include $C_{1-2}$ alkyl and $O(C_{1-2}$ alkyl) each of which is unsubstituted or is substituted with 3 fluorine substitutents, such as $-CF_3$, $-OCF_3$ and $-OCH_3$. For the avoidance of doubt, when $R^2$ is according to option (ii) above and is substituted as described above, the one or more substituent(s) are each preferably present on the alkyl moiety of the $R^2$ group.

When an $R^2$ group is according to option (iii) above, each $R^a$ and $R^b$ is independently preferably selected from hydrogen and methyl. Each $R^c$ is preferably methyl. More preferably, each $R^a$ and $R^b$ is independently selected from hydrogen and methyl (preferably hydrogen) and $R^c$ is methyl.

Preferably, each $R^8$ group is independently selected from CN, OH, $-C(O)NR^fR^g$, $-NR^fR^g$, wherein each of $R^f$

and $R^g$ is independently H or methyl, preferably hydrogen. More preferably, each $R^8$ group is independently selected from CN and $-C(O)NR^fR^g$.

Accordingly, in Formula (I), each $R^2$ is preferably independently selected from:

- halo or $R^8$;
- $C_{1-2}$ alkyl, O($C_{1-2}$ alkyl), S($C_{1-2}$ alkyl), SO($C_{1-2}$ alkyl) or $SO_2$($C_{1-2}$ alkyl), any of which may optionally be substituted with 1, 2 or 3 halo substituents and/or one $R^8$ substituent; and
- $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;

wherein each $R^8$ is independently selected from CN, OH, $-C(O)NR^fR^g$, and $-NR^fR^g$; wherein each of $R^f$ and $R^g$ is independently H or unsubstituted $C_{1-2}$ alkyl.

More preferably, in Formula (I), each $R^2$ is independently selected from

- halo, CN, OH, $-C(O)NR^fR^g$, $-NR^fR^g$; wherein each of $R^f$ and $R^g$ is independently H or methyl; and
- $C_{1-2}$ alkyl, O($C_{1-2}$ alkyl), S($C_{1-2}$ alkyl), SO($C_{1-2}$ alkyl) any of which may optionally be substituted with 1, 2 or 3 halo substituents and/or one substituent selected from CN and OH.

In Formula (I), *m* is preferably 0, 1 or 2. More preferably, *m* is 1 or 2. Sometimes *m* is 1. Sometimes *m* is 2.

Therefore, in Formula (I), preferably:

- Ⓐ is a cyclic group selected from phenyl, 5- to 6-membered heteroaryl, and 5- to 6-membered carbocyclic and heterocyclic groups;
- each $R^2$ is independently selected from:
    - halo or $R^8$;
    - $C_{1-2}$ alkyl, O($C_{1-2}$ alkyl), S($C_{1-2}$ alkyl), SO($C_{1-2}$ alkyl) or $SO_2$($C_{1-2}$ alkyl), any of which may optionally be substituted with 1, 2 or 3 halo substituents and/or one $R^8$ substituent; and
    - $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;
- wherein each $R^8$ is independently selected from CN, OH, $-C(O)NR^fR^g$, and $-NR^fR^g$; wherein each of $R^f$ and $R^g$ is independently H or unsubstituted $C_{1-2}$ alkyl.

and

- *m* is 0, 1 or 2.

More preferably, in Formula (I):

- Ⓐ is selected from phenyl, cyclohexane, piperidine, pyridazine, pyridine and thiazole;
- each $R^2$ is independently selected from
    - halo, CN, OH, $-C(O)NR^fR^g$, $-NR^fR^g$; wherein each of $R^f$ and $R^g$ is independently H or methyl; and
    - $C_{1-2}$ alkyl, O($C_{1-2}$ alkyl), S($C_{1-2}$ alkyl), SO($C_{1-2}$ alkyl) any of which may optionally be substituted with 1, 2 or 3 halo substituents and/or one substituent selected from CN and OH;

  and
- *m* is 1 or 2.

Typically, in Formula (I), *n* is 0.

In Formula (I), if *n* is 1, $R^3$ is preferably selected from hydrogen and an unsubstituted $C_1$ to $C_3$ alkyl group such as methyl or ethyl, preferably methyl. More preferably, $R^3$ if present is hydrogen.

Typically, in Formula (I), Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$, $-NR^{10}C(O)S-$, $-SC(O)NR^{10}$, $-NR^{10}C(NR^{11})-$, $-C(NR^{10})NR^{11}-$, $-NR^{10}C(NR^{11})NR^{12}-$,$-NR^{10}C(NR^{11})O-$, $-OC(NR^{10})NR^{11}$, $-NR^{10}C(NR^{11})S-$, and $-SC(NR^{10})NR^{11}$, wherein $R^{10}$, $R^{11}$ and $R^{12}$ are as defined herein. More preferably, Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$, $-NR^{10}C(O)S-$, $-SC(O)NR^{10}$, $-NR^{10}C(NR^{11})-$, $-C(NR^{10})NR^{11}-$, and $-NR^{10}C(NR^{11})NR^{12}-$. Still

more preferably, Z is a bond or is selected from $-NR^{10}C(O)-$, $-C(O)NR^{10}-$, $-NR^{10}C(O)NR^{11}-$, $-NR^{10}C(O)O-$, $-NR^{10}C(O)S-$, and $-NR^{10}C(NR^{11})-$. Most preferably, Z is selected from $-NR^{10}C(O)-$, $-C(O)NR^{10}-$, and $-NR^{10}C(O)NR^{11}-$, preferably $-NR^{10}C(O)-$.

In Formula (I), L is a bond or is selected from $C_{1-4}$ alkylene, $C_{2-4}$ alkenylene, $C_{2-4}$ alkynylene, $C_{1-3}$ alkylene-($C_{3-6}$cycloalkylene)-$C_{1-3}$ alkylene, $C_{1-4}$ alkylene-($C_{3-6}$cycloalkylene) and ($C_{3-6}$cycloalkylene)-$C_{1-4}$ alkylene, wherein L is unsubstituted or is substituted with 1 or 2 substituents selected from halogen, $-OR^{10}$, and $-NR^{10}R^{11}$; or L is $-C(R^{10})=N-$. Typically, in Formula (I), L is unsubstituted or is substituted with 1 substituent selected from halogen, $-OR^{10}$, and $-NR^{10}R^{11}$; most typically L is unsubstituted. When L is other than a bond or $-C(R^{10})=N-$ and L is substituted by one or more substituents as described above, the one or more substituents are each preferably present on the alkylene, alkenylene or alkynylene group(s) of L. For the avoidance of doubt, when L is a bond L is unsubstituted.

L is preferably a bond or is selected from $C_{1-4}$ alkylene, $C_{2-4}$ alkenylene and $C_{2-4}$ alkynylene; or L is $-C(R^{10})=N-$. More preferably, L is a bond or is selected from $C_{1-3}$ alkylene and $C_{2-3}$ alkenylene or is $-C(R^{10})=N-$. Still more preferably, L is selected from $C_{1-3}$ alkylene and $C_{2-3}$ alkenylene.

Typically, in Formula (I), X is a bond or, when L is other than a bond or $-C(R^{10})=N-$, X is a bond or is selected from $-NR^{10}-$ and -O-. More preferably, X is a bond.

Preferably, therefore, in Formula (I):

- Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$ $-NR^{10}C(O)S-$, $-SC(O)NR^{10}$, $-NR^{10}C(NR^{11})-$, $-C(NR^{10})NR^{11}-$, and $-NR^{10}C(NR^{11})NR^{12}-$;
- L is a bond or is selected from $C_{1-4}$ alkylene, $C_{2-4}$ alkenylene and $C_{2-4}$ alkynylene; or L is $-C(R^{10})=N-$;

and

- X is a bond.

More preferably, in Formula (I):

- Z is selected from $-NR^{10}C(O)-$, $-C(O)NR^{10}-$, and $-NR^{10}C(O)NR^{11}-$;
- L is selected from $C_{1-3}$ alkylene and $C_{2-3}$ alkenylene each of which are preferably unsubstituted;

and

- X is a bond.

In Formula (I), $R^4$ is:

(i) selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or

(ii) $R^4$ is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN.

In Formula (I), $R^5$ is

(i) selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or

(ii) is joined together with $R^4$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

or

(iii) is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen,

$-OR^{10}$, $-NR^{10}R^{11}$ , and -CN;

When $R^4$ is $C_1$ to $C_3$ alkyl according to option (i) above, it is typically unsubstituted or is substituted with 1, 2 or 3 halo substituents or with 1 or 2 halo substituents and/or with one substituent selected from $-OR^{10}$, $-NR^{10}R^{11}$, and -CN. When $R^4$ is according to option (i) above, $R^4$ is preferably H or $C_1$ to $C_2$ alkyl which is unsubstituted or is substituted with 1, 2 or 3 halo substituents or with one $-OR^{10}$ substituent; more preferably $R^4$ is H or methyl, most preferably H.

When $R^5$ is $C_1$ to $C_3$ alkyl according to option (i) above, it is typically unsubstituted or is substituted with 1, 2 or 3 halo substituents or with 1 or 2 halo substituents and/or with one substituent selected from $-OR^{10}$, $-NR^{10}R^{11}$, and -CN. When $R^3$ is according to option (i) above, $R^5$ is preferably selected from H, -CN and $C_1$ to $C_2$ alkyl which is unsubstituted or is substituted with 1, 2 or 3 halo substituents or one $-NR^{10}R^{11}$ substituent; more preferably, $R^5$ is H or methyl, most preferably H.

When $R^4$ is according to option (ii) above and $R^5$ is according to option (ii) above so that $R^4$ and $R^5$ are joined together to form, together with the atoms to which they are attached, a 5- to 6- membered heterocyclic group comprising at least one saturated carbon atom in the ring, the heterocyclic group is preferably unsubstituted or is substituted with 1 substituent selected from unsubstituted $C_1$ to $C_2$ alkyl, halogen, and $-OR^{10}$; more preferably the heterocyclic group is unsubstituted or is substituted with 1 substituent selected from methyl and methoxy; most preferably the heterocyclic group is unsubstituted. Preferably, when $R^4$ is according to option (ii) above and $R^5$ is according to option (ii) above, $R^4$ and $R^5$ are joined together to form, together with the atoms to which they are attached, a 5- membered heterocyclic group, preferably 4,5-dihydro-1H-imidazole.

In Formula (I), $R^6$ is

(i) selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or

(ii) is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

or

(iii) is joined together with $R^7$ if present to form, together with the atoms to which they are attached, a 5- to 6-membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN.

When $R^6$ is $C_1$ to $C_3$ alkyl according to option (i) above, it is typically unsubstituted or is substituted with 1, 2 or 3 halo substituents or with 1 or 2 halo substituents and/or with one substituent selected from $-OR^{10}$, $-NR^{10}R^{11}$, and -CN. When $R^6$ is according to option (i) above, $R^6$ is preferably selected from H, -CN and $C_1$ to $C_2$ alkyl which is unsubstituted or is substituted with 1, 2 or 3 halo substituents or one $-NR^{10}R^{11}$ substituent; more preferably, $R^6$ is H or methyl, most preferably H.

When $R^5$ is according to option (iii) above and $R^6$ is according to option (ii) above so that $R^6$ and $R^6$ are joined together to form, together with the atoms to which they are attached, a 5- to 6- membered heterocyclic group comprising at least one saturated carbon atom in the ring, the heterocyclic group is preferably unsubstituted or is substituted with 1 substituent selected from unsubstituted $C_1$ to $C_2$ alkyl, halogen, and $-OR^{10}$; more preferably the heterocyclic group is unsubstituted or is substituted with 1 substituent selected from methyl and methoxy; most preferably the heterocyclic group is unsubstituted. Preferably, when $R^5$ is according to option (iii) above and $R^6$ is according to option (ii) above, $R^5$ and $R^6$ are joined together to form, together with the atoms to which they are attached, a 6- membered heterocyclic group, preferably morpholine or piperazine, more preferably morpholine.

In Formula (I), *p* is 0 or 1.

In Formula (I), $R^7$ if present is

(i) selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or

(ii) is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

When $R^7$ is present and is $C_1$ to $C_3$ alkyl according to option (i) above, it is typically unsubstituted or is substituted with 1, 2 or 3 halo substituents or with 1 or 2 halo substituents and/or with one substituent selected from $-OR^{10}$, $-NR^{10}R^{11}$, and -CN. When $R^7$ is present and is according to option (i) above, $R^7$ is preferably selected from H, -CN and $C_1$ to $C_2$ alkyl which is unsubstituted or is substituted with 1, 2 or 3 halo substituents or one $-NR^{10}R^{11}$ substituent; more preferably, $R^7$ if present is H or methyl, most preferably H.

When $R^7$ is present and $R^6$ is according to option (iii) above and $R^7$ is according to option (ii) above so that $R^6$ and $R^7$ are joined together to form, together with the atoms to which they are attached, a 5- to 6- membered heterocyclic group comprising at least one saturated carbon atom in the ring, the heterocyclic group is preferably unsubstituted or is substituted with 1 substituent selected from unsubstituted $C_1$ to $C_2$ alkyl, halogen, and $-OR^{10}$; more preferably the heterocyclic group is unsubstituted or is substituted with 1 substituent selected from methyl and methoxy; most preferably the heterocyclic group is unsubstituted. Preferably, when $R^7$ is present and is according to option (ii) above and $R^6$ is according to option (iii) above, $R^6$ and $R^7$ are joined together to form, together with the atoms to which they are attached, a 5- membered heterocyclic group, preferably imidazolidine.

Preferably, therefore, in Formula (I), *p* is 1 and $R^7$ is H or methyl or is joined together with $R^6$ to form, together with the atoms to which they are attached, an unsubstituted 5- to 6-membered heterocyclic group. Preferably, $R^4$ is H or is joined together with $R^5$ to form, together with the atoms to which they are attached, an unsubstituted 5- to 6-membered heterocyclic group. More preferably, in Formula (I), $R^5$ is selected from H, -CN and $C_1$ to $C_2$ alkyl which is unsubstituted or is substituted with 1, 2 or 3 halo substituents and/or one $-NR^{10}R^{11}$ substituent and $R^6$ is H or methyl. Most preferably, $R^4$, $R^5$, $R^6$ and $R^7$ if present are each selected from methyl and hydrogen, preferably hydrogen.

For the avoidance of doubt, a heterocyclic group comprising at least one saturated carbon atom in the ring comprises a $-CH_2-$ group within the ring, wherein one or both of the hydrogen atoms of the -CH2- group may be substituted as defined herein. Usually, the saturated carbon atom in the ring is unsubstituted; i.e., the a heterocyclic group comprising at least one saturated carbon atom in the ring usually comprises a -CH2- group within the ring. A heterocyclic group comprising at least one saturated carbon atom in the ring is therefore saturated or partially saturated. A heterocyclic group comprising at least one saturated carbon atom in the ring is not aromatic.

In some preferred compounds of Formula (I), therefore,

- $R^1$ is H;
- Ⓐ is a cyclic group selected from phenyl, 5- to 6-membered heteroaryl, and 5- to 6-membered carbocyclic and heterocyclic groups;
- *m* is 0, 1 or 2;
- each $R^2$ is independently selected from:
  - halo or $R^8$;
  - $C_{1-2}$ alkyl, $O(C_{1-2}$ alkyl), $S(C_{1-2}$ alkyl), $SO(C_{1-2}$ alkyl) or $SO_2(C_{1-2}$ alkyl), any of which may optionally be substituted with 1, 2 or 3 halo substituents and/or one $R^8$ substituent; and
  - $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;
- each $R^8$ is independently selected from CN, OH; $-C(O)NR^fR^g$, and $-NR^fR^g$; wherein each of $R^f$ and $R^g$ is independently H or unsubstituted $C_{1-2}$ alkyl;
- *n* is 0; or *n* is 1 and $R^3$ is H
- Z is selected from $-NR^{10}C(O)-$, $-C(O)NR^{10}-$, $-NR^{10}C(O)NR^{11}-$, $-NR^{10}C(O)O-$, $-OC(O)NR^{10}$, $-NR^{10}C(O)S-$, $-SC(O)NR^{10}$, $-NR^{10}C(NR^{11})-$, $-C(NR^{10})NR^{11}-$, and $-NR^{10}C(NR^{11})NR^{12}-$;
- L is a bond or is selected from $C_{1-4}$ alkylene, $C_{2-4}$ alkenylene and $C_{2-4}$ alkynylene; or L is $-C(R^{10})=N-$;

- X is a bond;
- i) *p* is 0;
  $R^4$ is H and $R^5$ is selected from H, -CN and $C_1$ to $C_2$ alkyl which is unsubstituted or is substituted with 1, 2 or 3 halo substituents and/or one-$NR^{10}R^{11}$ substituent; or $R^4$ is joined together with $R^5$ to form, together with the atoms to which they are attached, an unsubstituted 5- to 6- membered heterocyclic group; and
  $R^6$ is H or methyl;
  ii) *p* is 1; and
  or $R^4$ is H; $R^5$ is selected from H, -CN and $C_1$ to $C_2$ alkyl which is unsubstituted or is substituted with 1, 2 or 3 halo substituents and/or one-$NR^{10}R^{11}$ substituent; $R^6$ is H or methyl and $R^7$ is H or methyl; or $R^4$ is joined together with $R^5$ to form, together with the atoms to which they are attached, an unsubstituted 5- to 6- membered heterocyclic group; $R^6$ is H or methyl and $R^7$ is H;

In some even more preferred compounds of Formula (I),

- $R^1$ is H;
- Ⓐ is selected from phenyl, cyclohexane, piperidine, pyridazine, pyridine and thiazole;
- *m* is 1 or 2;
- each $R^2$ is independently selected from:

  ◦ halo, CN, OH, -C(O)$NR^fR^g$, -$NR^fR^g$; wherein each of $R^f$ and $R^g$ is independently H or methyl; and
  ◦ $C_{1-2}$ alkyl, O($C_{1-2}$ alkyl), S($C_{1-2}$ alkyl), SO($C_{1-2}$ alkyl) any of which may optionally be substituted with 1, 2 or 3 substituents selected from halo, CN, OH;

- *n* is 0;
- Z is selected from -$NR^{10}$C(O)-, -C(O)$NR^{10}$-, and -$NR^{10}$C(O)$NR^{11}$-;
- L is selected from $C_{1-3}$ alkylene and $C_{2-3}$ alkenylene.
- X is a bond;
- *p* is 0; or *p* is 1 and $R^7$ is H;
- $R^4$ is H;
- $R^5$ is selected from H, -CN and $C_1$ to $C_2$ alkyl which is unsubstituted or is substituted with 1, 2 or 3 halo substituents and/or one -$NR^{10}R^{11}$ substituent H; and
- $R^6$ is H.

Particularly preferred compounds of the invention are

- 5-[[4-[(2-guanidinoacetyl)amino]-3-(trifluoromethoxy)phenyl]sulfonylamino] thiazole-4-carboxylic acid;
- 5-[[3-fluoro-4-[[(2-guanidinoacetyl)amino]methyl]phenyl]sulfonylamino]thiazole-4-carboxylic acid;
- 5-[[3-fluoro-4-(guanidinomethyl)phenyl]sulfonylamino]thiazole-4-carboxylic acid;
- 5-[[3-fluoro-4-(2-guanidinoethylsulfanylcarbonylamino)phenyl]sulfonylamino] thiazole-4-carboxylic acid;
- 5-[[4-[2-[(2-amino-2-imino-ethyl)amino]-2-oxo-ethyl]-3-fluoro-phenyl] sulfonylamino]thiazole-4-carboxylic acid;
- 5-[[3-carbamoyl-4-[(2-guanidinoacetyl)amino]phenyl]sulfonylamino]thiazole-4-carboxylic acid;
- 5-[[3-cyano-4-[(2-guanidinoacetyl)amino]phenyl]sulfonylamino]thiazole-4-carboxylic acid;
- 5-[[3-fluoro-4-(2-guanidinoethoxycarbonylamino)phenyl]sulfonylamino]thiazole-4-carboxylic acid;
- 5-[(4-guanidinophenyl)sulfonylamino]thiazole-4-carboxylic acid;
- 5-[[4-[2-(2-carbamimidoylhydrazino)-2-oxo-ethyl]-3-fluoro-phenyl]sulfonylamino] thiazole-4-carboxylic acid;
- 5-[[3-chloro-4-[(2-guanidinoacetyl)amino]phenyl]sulfonylamino]thiazole-4-carboxylic acid;
- 5-[[4-[(2-guanidinoacetyl)amino]-3-methoxy-phenyl]sulfonylamino]thiazole-4-carboxylic acid;
- 5-[[4-[[2-(2-carbamimidoylhydrazino)acetyl]amino]-3-fluoro-phenyl]sulfonylamino] thiazole-4-carboxylic acid;
- 5-[[4-[[(2E)-2-(carbamimidoylhydrazono)acetyl]amino]-3-fluoro-phenyl] sulfonylamino]thiazole-4-carboxylic acid;
- 5-[[4-[[2-(4,5-dihydro-1H-imidazol-2-ylamino)acetyl]amino]-3,5-difluoro-phenyl] sulfonylamino]thiazole-4-carboxylic acid;
- 5-[[6-[(2-guanidinoacetyl)amino]pyridazin-3-yl]sulfonylamino]thiazole-4-carboxylic acid;
- 5-[[4-[(2-amino-2-imino-ethyl)carbamoylaimino]-3-fluoro-phenyl]sulfonylamino] thiazole-4-carboxylic acid;
- 5-[[3,5-difluoro-4-(guanidinocarbamoylamino)phenyl]sulfonylamino]thiazole-4-carboxylic acid;
- 5-[[4-[(3-amino-3-imino-propanoyl)amino]-3,5-difluoro-phenyl]sulfonylarmino] thiazole-4-carboxylic acid;
- 5-[[4-[[3-(dimethylamino)-3-imino-propanoyl]amino]-3-fluoro-phenyl] sulfonylamino]thiazole-4-carboxylic acid;
- 5-[[3-fluoro-4-[(2-guanidinooxyacetyl)amino]phenyl]sulfonylamino]thiazole-4-carboxylic acid;
- 5-[[3-fluoro-4-[[3-imino-3-(methylamino)propanoyl]amino]phenyl]sulfonylamino] thiazole-4-carboxylic acid;

- 5-[[4-[3-(4,5-dihydro-1H-imidazol-2-yl)propanoylamino]-3-fluoro-phenyl] sulfonylamino]thiazole-4-carboxylic acid ;
- 5-[[2-[(2-guanidinoacetyl)amino]thiazol-5-yl]sulfonylamino]thiazole-4-carboxylic acid;
- 5-[[4-[[2-[(N-cyanocarbamimidoyl)amino]acetyl]amino]-3-fluoro-phenyl] sulfonylamino]thiazole-4-carboxylic acid;
- 5-[[3-fluoro-4-(guanidinocarbamoylamino)phenyl]sulfonylamino]thiazole-4-carboxylic acid;
- 5-[[3-fluoro-4-[[2-(morpholine-4-carboximidoylamino)acetyl]amino]phenyl] sulfonylamino]thiazole-4-carboxylic acid;
- 5-[[4-[(3-amino-3-imino-2-methyl-propanoyl)amino]-3-fluoro-phenyl] sulfonylamino]thiazole-4-carboxylic acid;
- 5-[[4-[[2-(4,5-dihydro-1H-imidazol-2-yl)acetyl]amino]-3-fluoro-phenyl] sulfonylamino]thiazole-4-carboxylic acid;
- 5-[[4-(carbamimidoylcarbamoylamino)-3-fluoro-phenyl]sulfonylamino]thiazole-4-carboxylic acid;
- 5-[[4-[[(2R)-2-guanidinopropanoyl]amino]phenyl]sulfonylamino]thiazole-4-carboxylic acid;
- 5-[[3,5-difluoro-4-[(2-guanidinoacetyl)amino]phenyl]sulfonylamino]thiazole-4-carboxylic acid;
- 5-[[4-[(4-amino-4-imino-butanoyl)amino]-3-fluoro-phenyl]sulfonylamino]thiazole-4-carboxylic acid;
- 5-[[4-[[2-(4,5-dihydro-1H-imidazo1-2-ylamino)acetyl]amino]-2,5-difluoro-phenyl] sulfonylamino]thiazole-4-carboxylic acid;
- 5-[[2,5-difluoro-4-[(2-guanidinoacetyl)amino]phenyl]sulfonylamino]thiazole-4-carboxylic acid;
- 5-[[3-fluoro-4-[[2-[(N-methylcarbamimidoyl)amino]acetyl]amino]phenyl] sulfonylamino]thiazole-4-carboxylic acid;
- 5-[[3-fluoro-4-[[2-(2-iminoimidazolidin-1-yl)acetyl]amino]phenyl]sulfonylamino] thiazole-4-carboxylic acid;
- 5-[[4-[[2-[carbamimidoyl(methyl)amino]acetyl]amino]-3-fluoro-phenyl] sulfonylamino]thiazole-4-carboxylic acid;
- 5-[[4-[[2-[[N-(2-aminoethyl)carbamimidoyl]amino]acetyl]amino]-3-fluoro-phenyl] sulfonylamino]thiazole-4-carboxylic acid;
- 5-[[5-fluoro-6-[(2-guanidinoacetyl)amino]-3-pyridyl]sulfonylamino]thiazole-4-carboxylic acid;
- 5-[[3-fluoro-4-(3-guanidinopropanoylamino)phenyl]sulfonylamino]thiazole-4-carboxylic acid;
- 5-[[4-[(3-amino-3-imino-propanoyl)amino]-3-fluoro-phenyl]sulfonylamino]thiazole-4-carboxylic acid;
- 5-[[3,5-difluoro-4-(guanidinocarbamoylamino)phenyl]sulfonylamino]thiazole-4-carboxylic acid;
- 5-[[3-fluoro-4-[(2-guanidinoacetyl)amino]phenyl]sulfonylamino]thiazole-4-carboxylic acid; and
- 5-[[4-[(2-guanidinoacetyl)amino]phenyl]sulfonylamino]thiazole-4-carboxylic acid; and pharmaceutically acceptable salts thereof.

*Synthesis*

The compounds of the invention can be prepared by any suitable method. Detailed general synthetic routes for representative compounds of the invention are set out below and in the Examples.

In summary, compounds of the invention can typically be prepared in a reaction according to the following scheme:

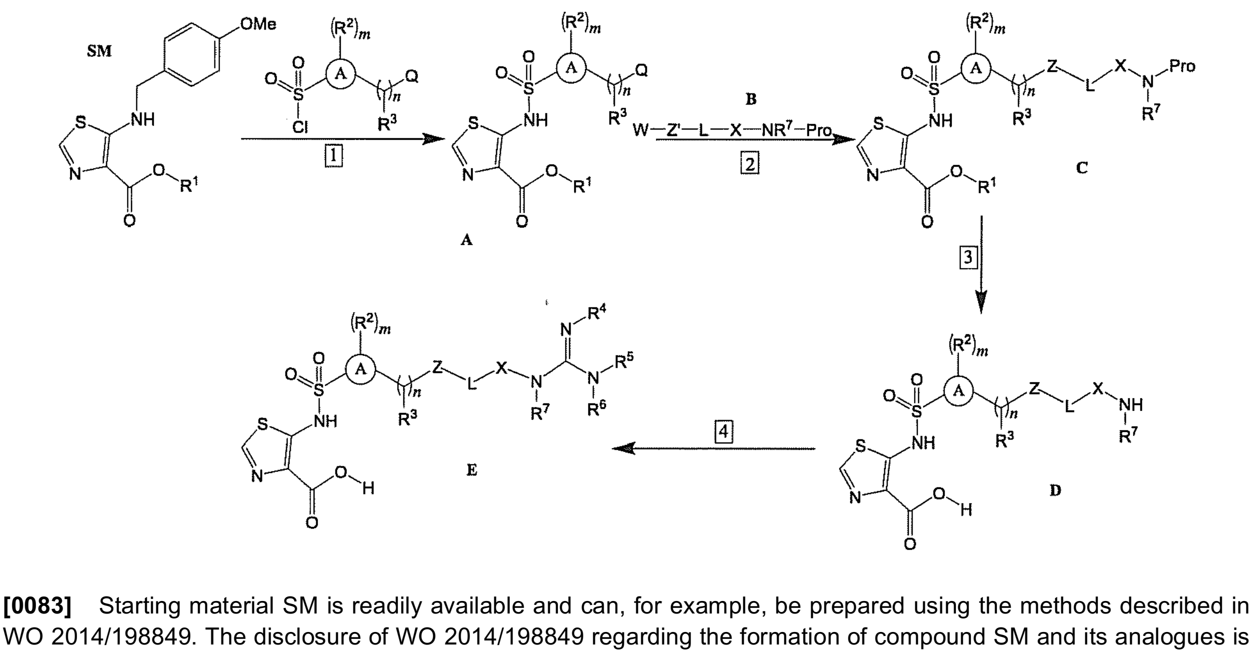


Starting material SM is readily available and can, for example, be prepared using the methods described in WO 2014/198849. The disclosure of WO 2014/198849 regarding the formation of compound SM and its analogues is incorporated by reference. Reaction of SM with a sulphonyl chloride derivative of Ⓐ (reaction step 1) yields a thiazole sulphonamide derivative of Ⓐ (**A**). Reaction of **A** with a W-Z-L-X-Pro moiety (**B**) yields intermediate **C**. In the above

scheme, Q and W are complementary reactive groups which react together to couple **A** to **B** to yield compound **C**. For example, Q can be bromine and -Z'-W can be -C(O)$NH_2$ so that Q and W react together via Buchwald chemistry (this is particularly suited to when *n* is 0). Alternatively, Q can be -$NH_2$ and -Z'-W can be -C(O)OH so that Q and W react together in a standard peptide coupling reaction using reagents such as HATU. Other methods of coupling compounds are well known to those skilled in the art. In compounds **B** and **C** the moiety -$NR^7$-Pro represents a protected amine moiety which can be deprotected to yield the amine via standard methods such as acid-catalysed deprotection (compounds **D**). Suitable amine protecting groups are well known to those skilled in the art and include Boc (*tert*-butoxycarbonyl) protecting groups The amine can then be reacted to form a guanidine group as in compound **E** by reaction with known guanidinylating agents such as 1H-pyrazole-1-carboximidamide. In compounds of the invention wherein *p* is zero such that an amidine rather than a guanidine group is present, the synthesis shown above can be modified so that compound **B** comprises a protected amidine group rather than protected amine $NR^7$-Pro. Suitable amidine protecting groups are well known to those skilled in the art and include Boc (*tert*-butoxycarbonyl) protecting groups. In these cases, reaction of **A** and **B** yields a compound **C'** which when deprotected yields the desired amidine product **E'**. Detailed synthetic routes to exemplary compounds of the invention are set out below.

*Therapeutic Efficacy*

The compounds of the present invention are therapeutically useful. The present invention therefore provides compounds as described herein, for use in medicine. The present invention provides compounds as described herein, for use in treating the human or animal body. For the avoidance of doubt, the compound of the invention may be administered in the form of a solvate.

Also provided is a pharmaceutical composition comprising a compound of the invention together with a pharmaceutically acceptable carrier or diluent and optionally further comprising an antibiotic agent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

The composition of the invention may be provided as a kit comprising instructions to enable the kit to be used in the methods described herein or details regarding which subjects the method may be used for.

As explained above, the compounds of the invention are useful in treating or preventing bacterial infection. In particular, they are inhibitors of metallo-P-lactamase (MBL) enzymes and are therefore useful for removing or reducing resistance of Gram-negative bacteria to antibiotics.

The compounds of the invention may be used as standalone therapeutic agents. For example, the compounds of the invention may be used as standalone adjuncts in antibacterial therapy, for example in chemotherapy regimes. Alternatively, they may be used in combination with antibiotic agents to enhance the action of the antibiotic agent.

The present invention therefore also provides a product comprising (i) a compound of the invention as described herein and (ii) an antibiotic agent. The compound of the invention and the antibiotic agent may be provided in a single formulation, or they may be separately formulated. Where separately formulated, the two agents may be administered simultaneously or separately. They may be provided in the form of a kit, optionally together with instructions for their administration.

Where formulated together, the two active agents may be provided as a pharmaceutical composition comprising (i) a compound of the invention as described herein and (ii) a further antibacterial compound; and (iii) a pharmaceutically acceptable carrier or diluent.

Preferably, the antibiotic agent is a β-lactam antibiotic. More preferably, the antibiotic agent is a β-lactam antibiotic is selected from carbapenems, penicillins, cephalosporins and penems. Examples of carbapenem antibiotics include Imipenem, Meropenem, Ertapenem, Doripenem and Biapenem. Examples of penicillins include Amoxicillin, Ampicillin, Ticarcillin, Piperacillin and Cloxacillin. Examples of cephalosporins include Cefazolin, Ceftriaxone, Ceftazidine and Ceftobiprole. Examples of penems include Faropenem. Other antibiotic agents include tobramycin, neomycin, streptomycin, gentamycin, tazobactam, rifampicin, ciprofloxacin, amikacin, colistin, aztreonam and levofloxacin.

The compounds of the invention are also useful in treating or preventing bacterial infection. The present invention therefore provides a compound of the invention for use in medicine. The invention also provides the use of a compound of the invention in the manufacture of a medicament. The invention also provides compositions and products comprising the compounds of the invention, as described here. Such compositions and products are also useful in treating or preventing bacterial infection. The present invention therefore provides a composition or product as defined herein for use in medicine. The invention also provides the use of a composition or product of the invention in the manufacture of a medicament.

As explained above, the compounds, compositions and products of the invention are useful in treating or preventing bacterial infection. The invention therefore also provides a method of treating or preventing bacterial infection

in a subject, which method comprises administering to said subject an effective amount of a compound, composition or product as described herein. Further provided is a compound, composition or product of the invention as described herein for the manufacture of a medicament for use in treating or preventing bacterial infection; the compound of the invention is often used in combination with an antibiotic agent.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

The compounds and combinations described herein are useful in the treatment of bacterial infection which occurs after a relapse following an antibiotic treatment. The compounds and combinations can therefore be used in the treatment of a patient who has previously received antibiotic treatment for the (same episode of) bacterial infection.

The bacterium causing the infection may be any bacterium expressing a metallo- β-lactamase enzyme or an analogue thereof. Typically the bacterium causing the infection expresses a MBL enzyme. The bacterium is typically Gram-negative. The bacterium may in particular be a pathogenic bacterium. Typically, the bacterial infection to be treated using the compounds of the invention is resistant to treatment with a conventional antibiotic when the conventional antibiotic is used alone.

The Gram-negative bacteria of which antibiotic resistance can be removed using the compounds of general formula (I) are bacteria which produce metallo-β-lactamases, which may be metallo-β-lactamases of subclasses B1, B2 or B3, for example IMP-type (including IMP-1), VIM-type (including VIM-1 and VIM-2) and NDM-type (including NDM-1) enzymes.

The bacterial infection may be caused by bacteria from the families Enterobacteriaceae, Pseudomonadaceae or Moraxellaceae. The bacterial infection may be caused by *Pseudomonas* (e.g. *Pseudomonas aeruginosa, Pseudomonas oryzihabitans,* or *Pseudomonas plecoglossicida), Klebsiella, Escherichia, Acinetobacter* or *Burkholderia.* For example, the bacterial infection may be caused by *Klebsiella pneumonia, Escherichia coli, Pseudomonas aeruginosa, Burkholderia cepacia* or *Acinetobacter baumannii.* The bacterium may be an opportunistic pathogen.

The compound or combination of the invention may be used to treat or prevent infections and conditions caused by any one or a combination of the above-mentioned bacteria. In particular, the compound or combination of the invention may be used in the treatment or prevention of pneumonia. The compound or combination may also be used in the treatment of septic shock, urinary tract infection, and infections of the gastrointestinal tract, skin or soft tissue.

The compound or combination of the invention may be used to treat patients with Carbapenem Resistant Enterobacteriaceae (CRE). CRE can be found in the community or in hospitals and other institutions which are commonly associated with long term patients and those that are undergoing significant medical interventions such as are commonly cared for in Intensive Care Units (ICUs).

A compound or combination of the invention can be administered to the subject in order to prevent the onset or reoccurrence of one or more symptoms of the bacterial infection. This is prophylaxis. In this embodiment, the subject can be asymptomatic. The subject is typically one that has been exposed to the bacterium. A prophylactically effective amount of the agent or formulation is administered to such a subject. A prophylactically effective amount is an amount which prevents the onset of one or more symptoms of the bacterial infection.

A compound or combination of the invention can be administered to the subject in order to treat one or more symptoms of the bacterial infection. In this embodiment, the subject is typically symptomatic. A therapeutically effective amount of the agent or formulation is administered to such a subject. A therapeutically effective amount is an amount effective to ameliorate one or more symptoms of the disorder.

The compound or combination of the invention may be administered in a variety of dosage forms. Thus, it can be administered orally, for example as tablets, troches, lozenges, aqueous or oily suspensions, dispersible powders or granules. Formulation composition of the invention may also be administered parenterally, whether subcutaneously, intravenously, intramuscularly, intrasternally, transdermally or by infusion techniques. The compound or combination may also be administered as a suppository. Preferably, compound or combination may be administered via inhaled (aerosolised) or intravenous administration, most preferably by inhaled (aerosolised) administration.

The compound or combination of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

The compound or combination of the invention may be formulated for inhaled (aerosolised) administration as

a solution or suspension. The compound or combination of the invention may be administered by a metered dose inhaler (MDI) or a nebulizer such as an electronic or jet nebulizer. Alternatively, the compound or combination of the invention may be formulated for inhaled administration as a powdered drug, such formulations may be administered from a dry powder inhaler (DPI). When formulated for inhaled administration, the compound or combination of the invention may be delivered in the form of particles which have a mass median aerodynamic diameter (MMAD) of from 1 to 100 μm, preferably from 1 to 50 μm, more preferably from 1 to 20 μm such as from 3 to 10 μm, e.g. from 4 to 6 μm. When the compound or combination of the invention is delivered as a nebulized aerosol, the reference to particle diameters defines the MMAD of the droplets of the aerosol. The MMAD can be measured by any suitable technique such as laser diffraction.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

A therapeutically or prophylactically effective amount of the compound of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific inhibitor, the age, weight and conditions of the subject to be treated, the type and severity of the disease and the frequency and route of administration. Preferably, daily dosage levels are from 5 mg to 2 g.

The antibacterial properties of the compounds described herein mean that they are also useful in the treatment of bacterial infection *in vitro,* i.e. other than by the treatment of human or animal subjects. Thus, the invention also provides a cleaning composition comprising a indane derivative of Formula (I) or a salt thereof. The cleaning composition may further comprise, for example, a detergent, a surfactant (including ionic and non-ionic surfactants), a diluent, a bleach (including a hypochlorite such as sodium hypochlorite or calcium hypochlorite, chlorine, chlorine dioxide, hydrogen peroxide or an adduct thereof, sodium perborate, and sodium percarbonate), an alcohol (such as ethanol or isopropanol), or a disinfectant. Typically, the disinfectant may be selected from benzyl-4-chlorophenol, amylphenol, phenylphenol, glutaraldehyde, alkyl dimethyl benzyl ammonium chloride, alkyl dimethyl ethylbenzyl ammonium chloride, iodine, peracetic acid and chlorine dioxide. Typically, the detergent may be an alkaline detergent such as sodium hydroxide, sodium metasilicate, or sodium carbonate, or an acid detergent such as hydrochloric acid, nitric acid, sulfuric acid, phosphoric acid, citric acid, or tartaric acid.

The following Examples illustrate the invention. They do not however, limit the invention in any way. In this regard, it is important to understand that the particular assay used in the Examples section is designed only to provide an indication of biological activity. There are many assays available to determine biological activity, and a negative result in any one particular assay is therefore not determinative.

**Experimental Details**

*General synthetic methodology*

There are several related synthetic methods to this class of compounds described by Formula 1 and which are described below, where R is taken to mean any substituent on the phenyl ring.

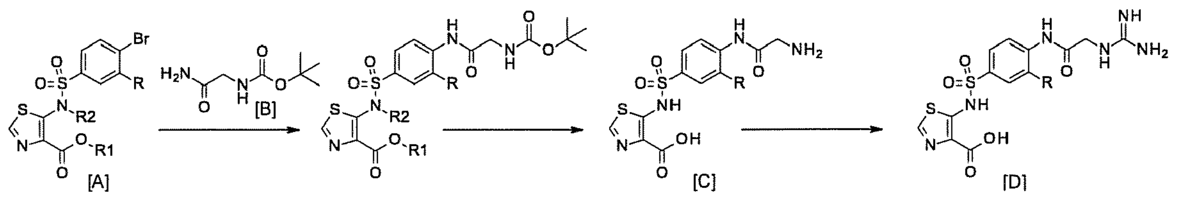

Scheme 1

The preparation of the key thiazole intermediate tert-butyl 5-{[(4-methoxyphenyl) methyl]amino}-1,3-thiazole-4-carboxylate has been described previously (WO2014/198849) and is easily prepared on a 100g scale. Reaction of this with a wide range of arylsulphonyl chlorides has been achieved using basic catalysis (such as pyridine, triethylamine or sodium hydride) giving sulphonamide intermediates such as [A]. Other versions of the thiazole starting material (eg R1 = ethyl; R2 = H) are also easily accessible or even commercially available. Many of the compounds described herein are accessible from the standard Buchwald reaction of bromophenylsulphonamide [A] with e.g. protected glycinamides such as [B]. Global acid-catalysed deprotection reveals the primary amine [C] which can be converted to the guanidine [D] if required (Scheme 1) using a guanidinylating reagent such as 1H-pyrazole-1-carboximidamide.

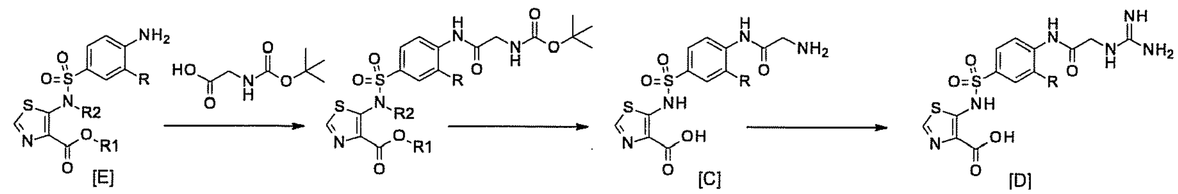

Scheme 2

Alternatively, instead of forming the aryl-nitrogen bond by using Buchwald chemistry on the aryl bromide, it is possible to react certain aniline intermediates such as [E] with the N-protected glycine acids using standard peptide coupling reagents such as HATU, (Scheme 2). Deprotection and guanidinylation then gives [C] and [D] again, respectively. The anilines [E] are available from the corresponding nitro compounds by standard reduction or from the bromo intermediates by a Buchwald reaction using ammonia (eg see Scheme 4).

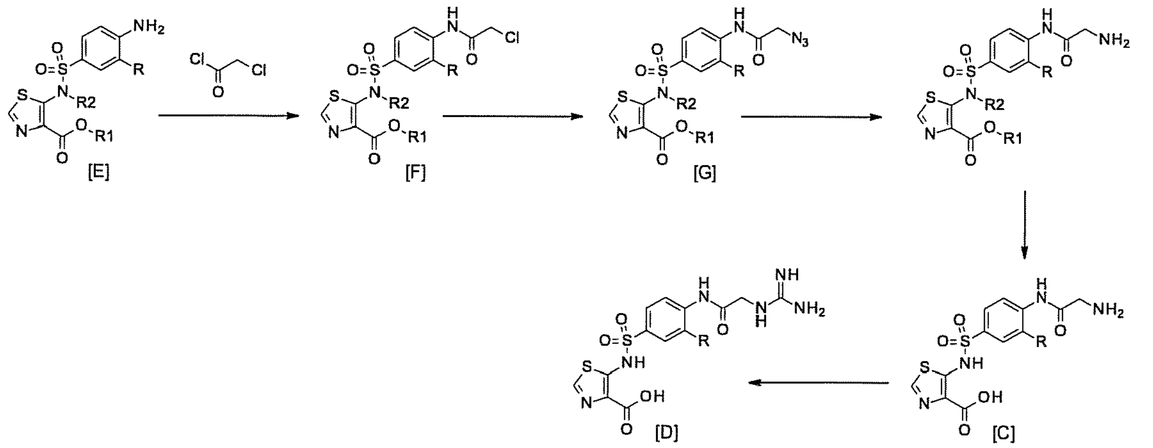

Scheme 3

In certain circumstances, eg where the substituents on the aryl ring are particularly electron-withdrawing, neither the Buchwald amidation nor the amide formation using protected glycine derivatives are successful. For these situations it is necessary to react the aniline with highly reactive chloroacetyl chloride to give intermediate [F]. Displacement with sodium azide then affords azidoacetamide [G] which can be reduced with standard reducing agents, accessing [C] and [D] in the usual manner, (Scheme 3).

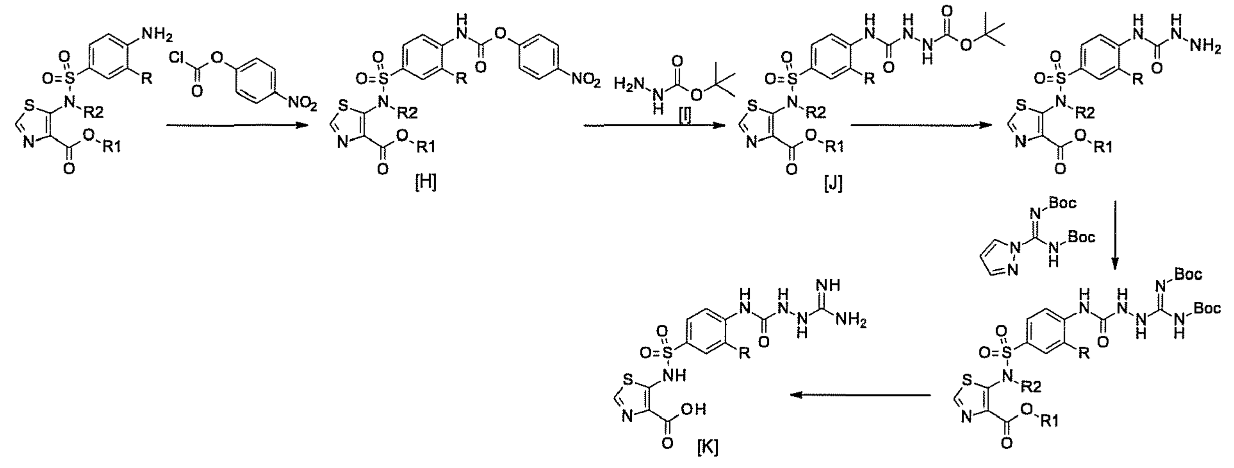


Scheme 4

Certain ureido derivatives require bespoke syntheses (Scheme 4). For example Buchwald reaction of the usual bromoarylsulphonamide with ammonia itself as the nitrogen-containing component gives the corresponding aniline. Activation of this aniline with 4-nitrophenyl chloroformate gives [H] which reacts with BOC-protected hydrazine [I] to give coupled product [J], possibly by the intermediacy of the isocyanate derived from [H]. Mild acid treatment removes the BOC group which can be guanidinylated to afford a protected guanidine functionality. Global deprotection of BOC, *p*-methoxybenzyl and *tert*-butyl ester groups then affords guanidine [K].

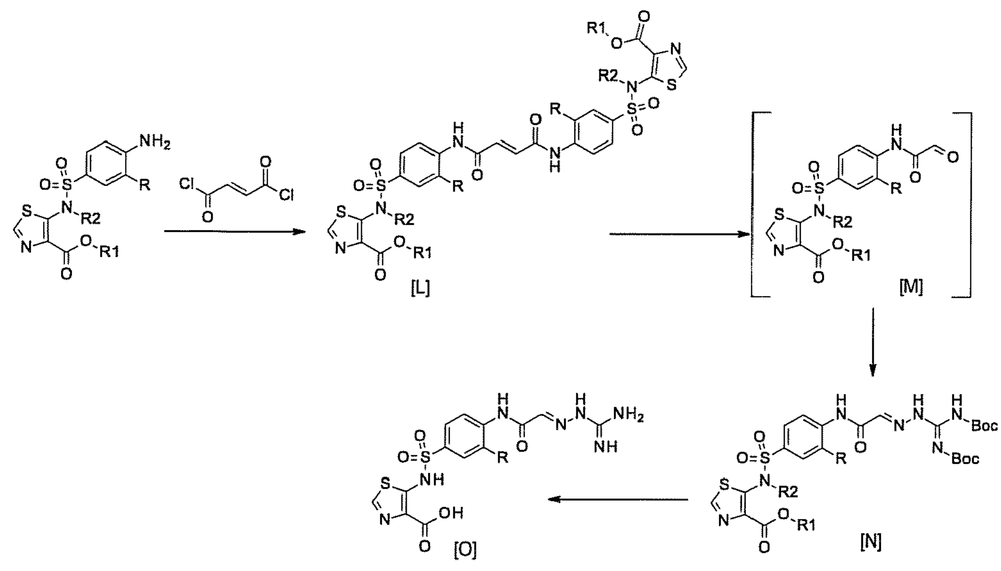


Scheme 5

Certain analogues require a critical glyoxamide intermediate [M], which is synthesised by reacting the usual aniline with 0.5 equivalents of fumaryl chloride to give symmetrical bis amide [L]. Ozonolysis proceeds to give the labile glyoxamide [M] which can be reacted with a variety of nucleophiles including the bis-BOC protected aminoguanidine affording [N]. Global deprotection in the usual way then affords the corresponding imine [O], (Scheme 5).

*Abbreviations*

| | |
|---|---|
| ACN | Acetonitrile |
| AcOH | Acetic acid |
| Ag(OTf) | Silver triflate |

| | |
|---|---|
| AIBN | Azobisisobutyronitrile |
| Boc | Tert-butoxy-carbonyle |
| Boc2O | Di-tert-butyl dicarbonate |
| $Cs_2CO_3$ | Cesium carbonate |
| CFU | Colony forming unit |
| CuI | Copper iodide |
| DCM | Dichloromethane |
| DIPEA | N,N-Diisopropylethylamine |
| DMAP | 4-Dimethylaminopyridine |
| DMF | Dimethylformamide |
| DMS | Dimethylsulfide |
| DMSO | Dimethyl sulfoxide |
| dppf | 1,1'-Bis(diphenylphosphino)ferrocene |
| EDC.HCl | N-(3-Dimethylaminopropyl)-N'-ethylcarbodiimide hydrochloride |
| EtOAc | Ethyl acetate |
| EtOH | Ethanol |
| Et3N | Triethylamine |
| HATU | 1-[Bis(dimethylamino)methylene]-1H-1,2,3-triazolo[4,5-b]pyridinium 3-oxid hexafluorophosphate |
| HCl | Hydrochloric acid |
| HOBt | Hydroxybenzotriazole |
| H2SO4 | Sulfuric Acid |
| IPA | *Iso*-propyl alcohol |
| Km | Michaelis constant |
| MeI | Methyl iodide |
| MeOH | Methanol |
| NBS | N-bromo succinimide |
| Na2CO3 | Sodium carbonate |
| Na2SO4 | Sodium sulfate |
| $Pd_2(dba)_3$ | Tris(dibenzylideneacetone)dipalladium(0) |
| $PdCl_2$(PPh3)2 | Bis(triphenylphosphine)palladium(II) dichloride |
| $PdCl_2$(dppf) | [1,1'-Bis(diphenylphosphino)ferrocene]dichloropalladium(II) |
| PMB | Paramethoxybenzyl |
| TEA | Triethylamine |
| TES | Triethylsilane |
| TMSOK | Potassium trimethylsilanolate |
| TFA | Trifluoroacetic acid |
| TMSOTf | Trimethylsilyl trifluoromethanesulfonate |
| TFA | Trifluoroacetic acid |
| THF | Tetrahydrofurane |
| T3P | Propylphosphinic anhydride |
| RT | Room temperature |

The structure of (RuPhos) Palladium (II) phenethylamine chloride (1:1 MTBE adduct) used for Buchwald coupling steps (RuPhos Pd G1 complex) is shown below.

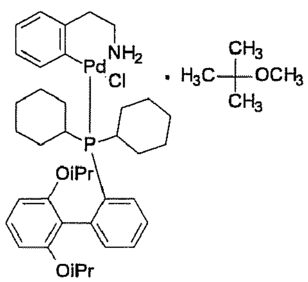

## Examples

*General Techniques*

1H NMR spectra are reported at 300 or 400 MHz in DMSO-d6 solutions (δ in ppm), using chloroform as the reference standard (7.25 ppm). When peak multiplicities are reported, the following abbreviations are used: s (singlet), d (doublet), t (triplet), m (multiplet), bs (broadened singlet), dd (doublet of doublets), dt (doublet of triplets), q (quartet). Coupling constants, when given, are reported in hertz (Hz).

The term "purified by prep hplc (MDAP)" refers compound purification using a mass-directed auto purification system on an Agilent 1260 infinity machine with an XSelect CHS Prep C18 column, eluting with 0.1% formic acid in water/acetonitrile and detection with a Quadruploe LC/MS.

### Example 1

**tert-butyl 5-[(4-methoxyphenyl)methylamino]thiazole-4-carboxylate** *(Key Intermediate-1)*

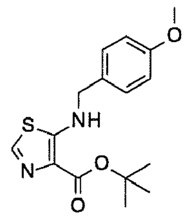

A suspension of potassium tert-butoxide (874 mg, 7.79 mmol) in dry tetrahydrofuran (10 mL) was stirred vigorously at room temperature. To this, a solution of tert-butyl isocyanoacetate (1.0 g, 7.08 mmol) in dry tetrahydrofuran (5 mL) was added drop wise and the mixture stirred at room temperature for 10 minutes. To this, a solution of 4-methoxybenzyl isothiocyanate (1.27 g, 7.08 mmol) in dry tetrahydrofuran (5 mL) was added drop wise at room temperature. After 2 hours the solution was poured into saturated NaHCO3 solution and extracted with ethyl acetate. The organic layer was dried with Na2SO4, filtered and concentrated in vacuo to dryness. The residue was purified by silica gel chromatography (eluting with 0-50% ethyl acetate/cyclohexane) affording the title product as a pale yellow solid (852 mg).

1H NMR ($CDCl_3$) δ: 7.81 (1H, m), 7.73 (1H, br s), 7.31-7.23 (2H, m), 6.92-6.85 (2H, m), 4.35 (2H, d), 3.80 (3H, s), 1.61 (9H, s).
M/z 321 $(M+H)^+$

### Example 2

**5-[[3,5-difluoro-4-[(2-guanidinoacetyl)amino]phenyl]sulfonylamino]thiazole-4-carboxylic acid**

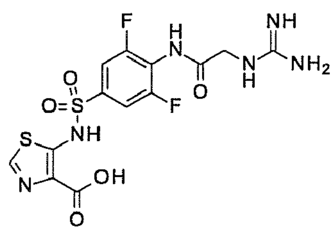

a. tert-butyl 5-[(4-bromo-3,5-difluoro-phenyl)sulfonyl-[(4-methoxyphenyl)methyl]amino]thiazole-4-carboxylate

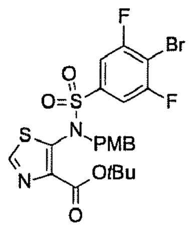

A solution of tert-butyl 5-[(4-methoxyphenyl)methylamino]thiazole-4-carboxylate (1 g, 3.12 mmol, 1 eq) in THF (15 mL) was added to NaH suspension in THF (10 mL) at 0 °C under argon atmosphere. After 30 minutes, a solution of 4-bromo-3,5-difluoro-benzenesulfonyl chloride (1.0 g, 3.43 mmol, 1.1 eq) in THF (15 mL) was added at 0 °C under argon atmosphere. The resulting reaction mixture was stirred at RT for 1 h, quenched with ice cold water (20 mL) and extracted with ethyl acetate (2 x 20 mL). The combined organic layer was dried over $Na_2SO_4$, filtered and concentrated. The crude material was purified by trituration with diethyl ether (2 x 5 mL) affording a pale yellow solid (800 mg, 44%).
M/z 577.0 $(M+H)^+$

b. tert-butyl 5-[[4-[[2-(tert-butoxycarbonylamino)acetyl]amino]-3,5-difluorophenyl]sulfonyl-[(4-methoxyphenyl)methyl]amino]thiazole-4-carboxylate

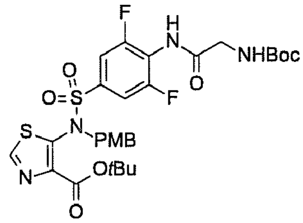

A solution of tert-butyl 5-[(4-bromo-3,5-difluoro-phenyl)sulfonyl-[(4-methoxyphenyl)methyl]amino]thiazole-4-carboxylate (100 mg, 0.173 mmol, 1 eq) in 1,4-dioxane (5 mL) was purged with argon for 15 minutes. Then tert-butyl N-(2-amino-2-oxoethyl)carbamate (45 mg, 0.26 mmol, 1.5 eq), $K_3PO_4$(110 mg, 0.521 mmol, 3 eq), Pd2(dba)3 (16 mg, 0.17 mmol, 0.1 eq) and Xantphos (30 mg, 0.052 mmol, 0.3 eq) were added under argon atmosphere. The resulting reaction mixture was heated to 85 °C for 16 h in a closed vial. The temperature was allowed to cool to RT, and the reaction mixture was filtered through celite pad and the pad was washed with EtOAc (2 x 5 mL). The organic layer was concentrated under reduced pressure. The resulting crude compound was dissolved in ethyl acetate (25 mL), washed with water (10 mL) and brine solution (10 mL). The organic layer was dried over $Na_2SO_4$, filtered and concentrated. The crude material was purified by flash chromatography (eluting with 55% ethyl acetate in petroleum ether) affording a pale yellow solid (60 mg, 51%).
M/z 669.5 $(M+H)^+$

c. 5-[[4-[(2-aminoacetyl)amino]-3,5-difluoro-phenyl]sulfonylamino]thiazole-4-carboxylic acid, trifluoroacetate

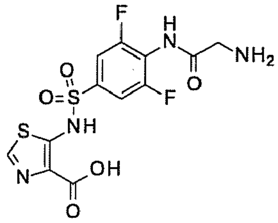

TFA (4 mL) was added to tert-butyl 5-[[4-[[2-(tert-butoxycarbonylamino)acetyl]amino]-3,5-difluoro-phenyl]sulfonyl-[(4-methoxyphenyl)methyl]amino]thiazole-4-carboxylate (300 mg, 0.448 mmol, 1 eq) at RT and stirred for 4 h. TFA was evaporated by reduced pressure. The resulting crude product was triturated with diethyl ether (2 x 5 mL) and dried under high vacuum to afford a pale yellow solid (150 mg, 85%).
M/z 393.3 $(M+H)^+$

d. 5-[[3,5-difluoro-4-[(2-guanidinoacetyl)amino]phenyl]sulfonylamino]thiazole-4-carboxylic acid

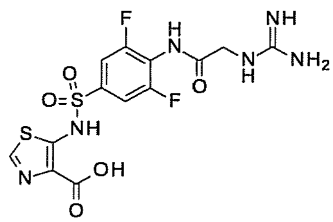

To a stirred solution of 5-[[4-[(2-aminoacetyl)amino]-3,5-difluorophenyl]sulfonylamino]thiazole-4-carboxylic acid, trifluoroacetate (150 mg, 0.382 mmol, 1 eq) in DMF (5 mL) was added pyrazole-1-carboxamidine;hydrochloride (84 mg, 0.573 mmol, 1.5 eq) and DIPEA (0.3 mL, 1.91 mmol, 5 eq) at RT. The resulting reaction mixture was stirred at RT for 16 h, and concentrated under reduced pressure. Water (5 mL) was added to the residue and the precipitate was filtered and washed with diethyl ether (2 x 5 mL). The crude product was purified by preparative HPLC to afford the title compound as a white solid (47 mg, 28%).

$^1$H NMR (400 MHz, DMSO-$d_6$) $\delta$ 13.20 (1H, s), 10.14 (1H, brs), 8.12 (1H, s), 7.55 (1H, brs), 7.43 (2H, d, $J$= 7.2 Hz), 7.35-7.10 (3H, brs), 4.12 (2H, s).
M/z 434.9 $(M+H)^+$

**LC-MS Condition:**

Column: Acquity BEH C18 (50mmx2.1mm, 1.7um);
Mobile Phase: A: 0.05% Formic Acid in water;
B: 0.05% Formic Acid in ACN;
Time (min) /%B: 0/3, 0.4/3, 2/98, 3.4/98, 3.5/3, 4/3;
Column Temp: 35°C, Flow Rate: 0.6mL/min

**Prep. HPLC Condition:**

Column: Symmetry C18 (300*19) mm, 7u;
Mobile phase: (A) 0.1% Formic Acid (B) Acetonitrile;
Flow: 19 mL/min;
Gradient (T/%B): 0/5,1/5,8/20,11/20,11.02/99,12/99,12.1/5,15/5;
Solubility: ACN +$H_2O$+THF+FA

**Example 3**

**5-[[5-fluoro-6-[(2-guanidinoacetyl)amino]-3-pyridyl]sulfonylamino]thiazole-4-carboxylic acid**

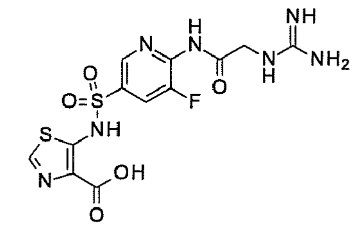

a. 5-fluoro-6-hydroxy-pyridine-3-sulfonyl chloride

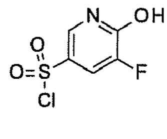

3-fluoropyridin-2-ol (2 g, 17.6 mmol) was added to chlorosulfonic acid (20 mL, 300.3 mmol) at 0 °C. The reaction mixture was stirred at 160 °C for 2 h, cooled to RT and slowly poured into ice cold water (50 mL). The aqueous layer was extracted with EtOAc (3 x 50 mL). The organic layer was dried over $Na_2SO_4$, filtered and concentrated under reduced pressure. The crude compound was triturated with n-pentane (2 x 50 mL) to afford an off-white solid (2.7 g, 72%).
M/z 212.11 $(M+H)^+$

b. 6-chloro-5-fluoro-pyridine-3-sulfonyl chloride

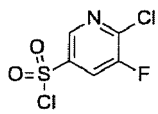

Thionyl chloride (5 mL, 68.9 mmol) was added to 5-fluoro-6-hydroxy-pyridine-3-sulfonyl chloride (1 g, 4.73 mmol) in toluene (25 mL) at 0 °C. DMF (0.2 mL) was then added slowly at 0 °C. The reaction mixture was refluxed for 3 h, cooled to RT and concentrated under reduced pressure. The resulting crude material was co-distilled with toluene (2 x 25 mL) to afford a pale yellow liquid which was used in the next step without further purification (0.9 g, crude).
$^1H$ NMR (400 MHz, DMSO-$d_6$) δ 8.83 (1H, m), 8.04 (1H, m). c. tert-butyl 5-[(6-chloro-5-fluoro-3-pyridyl)sulfonyl-[(4-methoxyphenyl)methyl]amino]thiazole-4-carboxylate

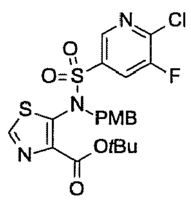

A solution of tert-butyl 5-[(4-methoxyphenyl)methylamino]thiazole-4-carboxylate (1.5 g, 4.68 mmol) in THF (25 mL) was added to NaH (1.12 g, 46.8 mmol) suspension in THF (10 mL) at 0 °C under argon atmosphere. After 15 minutes, a solution of 6-chloro-5-fluoro-pyridine-3-sulfonyl chloride (1.6 g, 7.0 mmol) in THF (15 mL) was added to the above reaction mixture at 0 °C under argon atmosphere. The resulting reaction mixture was stirred at RT for 0.5 h, quenched with ice cold water (20 mL) and extracted with ethyl acetate (3 x 20 mL). The combined organic layer was dried over $Na_2SO_4$, filtered and concentrated. The crude compound was purified by flash chromatography (eluting with 10-15% ethyl acetate in petroleum ether) to afford a yellow oil (1.3 g, 54%).
M/z 514.27 $(M+H)^+$

d. tert-butyl 5-[[6-[[2-(tert-butoxycarbonylamino)acetyl]amino]-5-fluoro-3-pyridyl]sulfonyl-[(4-methoxyphenyl)methyl]amino]thiazole-4-carboxylate

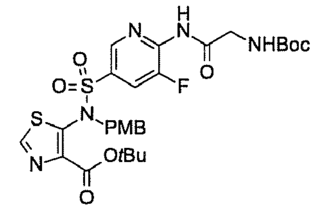

A solution of tert-butyl 5-[(6-chloro-5-fluoro-3-pyridyl)sulfonyl-[(4-methoxyphenyl)methyl]amino]thiazole-4-carboxylate (150 mg, 0.29 mmol) in 1,4-dioxane (5 mL) was purged with argon for 20 minutes. Then tert-butyl N-(2-amino-2-oxoethyl)carbamate (75 mg, 0.43 mmol), $Cs_2CO_3$ (282 mg, 0.87 mmol), Pd2(dba)3 (26 mg, 0.02 mmol) and Xantphos (50 mg, 0.08 mmol) were added under argon atmosphere. The resulting reaction mixture was heated to 70 °C for 0.5 h in a sealed tube, allowed to cool to RT, filtered through celite pad and the pad was washed with ethyl acetate (2 x 3 mL). The organic layer was concentrated under reduced pressure. The crude material was purified by flash chromatography (eluting with 50% ethyl acetate in petroleum ether) to afford a pale yellow solid (75 mg, 39%).
M/z 652.41 $(M+H)^+$

e. 5-[[6-[(2-aminoacetyl)amino]-5-fluoro-3-pyridyl]sulfonylamino]thiazole-4-carboxylic acid, trifluoacetate

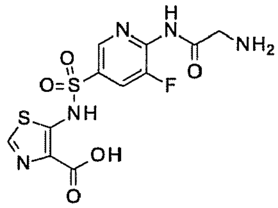

TFA (1.5 mL) was added to a solution of tert-butyl 5-[[6-[[2-(tert-butoxycarbonylamino)acetyl]amino]-5-fluoro-3-pyridyl]sulfonyl-[(4-methoxyphenyl)methyl]amino]thiazole-4-carboxylate (150 mg, 0.23 mmol) in DCM (2 mL) at 0 °C, allowed to stir at RT for 18 h and concentrated under reduced pressure. The residue was triturated with diethyl ether (2 x 2 mL), n-pentane (2 x 2 mL) and dried under high vacuum to afford an off white solid which was used in next step without further purification (60 mg, crude).
M/z 376.24 $(M+H)^+$

f. 5-[[5-fluoro-6-[(2-guanidinoacetyl)amino]-3-pyridyl]sulfonylamino]thiazole-4-carboxylic acid

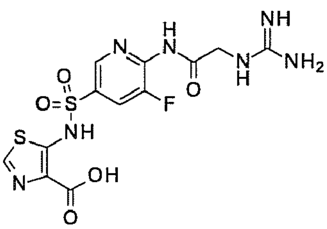

Pyrazole-1-carboxamidine;hydrochloride (70 mg, 0.48 mmol) and DIPEA (0.27 mL, 1.6 mmol) were added to a stirred solution of 5-[[6-[(2-aminoacetyl)amino]-5-fluoro-3-pyridyl]sulfonylamino]thiazole-4-carboxylic acid, trifluoroacetate (120 mg, 0.32 mmol) in DMF (2 mL) at RT. The resulting reaction mixture was stirred at RT for 4 h, concentrated under reduced pressure and ice cold IN HCl (2 mL) was added to the crude compound and stirred for 10 minutes. The

resulting precipitate was filtered, washed with diethyl ether (2 x 5 mL) and dried under high vacuum. The crude product was purified by preparative .HPLC affording the title product as an off white solid (25 mg, 18%).

$^1H$ NMR (400 MHz, DMSO-$d_6$) δ 13.20 (1H, brs), 10.8 (1H, brs), 8.51 (1H, d, *J* = 1.6 Hz), 8.13 (1H, s), 7.99 (1H, dd, *J* = 9.6 Hz, *J* = 1.6 Hz), 7.52 (1H, brs), 7.26 (3H, brs), 4.20 (2H, d, *J* = 4.4 Hz).
M/z 418.18 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column used: PHENYL HEXYL (150*30) mm 5 u;
Mobile phase: (A) 0.1% Formic Acid, (B) Acetonitrile;
Flow: 19 mL/min;
Gradient -(T/%B): 0/5, 1/5, 6/30, 8.9/30, 8.95/99, 11/99, 11.1/5, 14/5;
Solubility: ACN+THF.

**Example 4**

**5-[[4-[(3-amino-3-imino-propanoyl)amino]-3-fluoro-phenyl]sulfonylamino]thiazole-4-carboxylic acid**

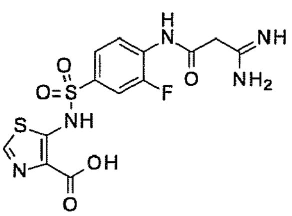

a. tert-butyl N-[3-amino-1-(tert-butoxycarbonylamino)-3-oxo-prop-1-enyl]carbamate

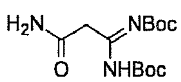

Saturated $NaHCO_3$ solution (10 mL) was added to a stirred solution of 3-amino-3-imino-propanamide (3 g, 29.6 mmol) in dioxane (20 mL) at RT. Then $(Boc)_2O$ (16.5 mL, 74.0 mmol) was added drop wise at 0 °C. The resulting reaction mixture was stirred at RT for 16 h, concentrated under reduced pressure and water (30 mL) was added to the residue. The crude compound was extracted with ethyl acetate (2 x 50 mL). The combined organic layer was dried over $Na_2SO_4$, filtered and concentrated. The crude material was purified by flash column chromatography (eluting with 2% methanol in DCM) to afford an off-white solid (3.1 g, 34%).
M/z 302.36 $(M+H)^+$

b. tert-butyl 5-[[4-[3,3-bis(tert-butoxycarbonylamino)prop-2-enoylamino]-3-fluorophenyl]sulfonyl-[(4-methoxyphenyl)methyl]amino]thiazole-4-carboxylate

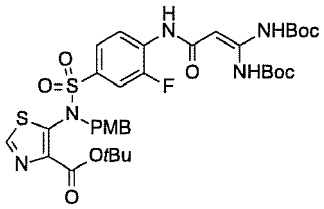

A solution of tert-butyl 5-[(4-bromo-3-fluoro-phenyl)sulfonyl-[(4-methoxyphenyl)methyl]amino]thiazole-4-carboxylate (1.1 g, 1.97 mmol) in 1,4-dioxane (15 mL) was purged with argon for 15 minutes. Then tert-butyl N-[3-amino-1-(tert-butoxycarbonylamino)-3-oxo-prop-1-enyl]carbamate (892 mg, 2.95 mmol), $K_3PO_4$ (837 mg, 3.94 mmol), $Pd_2(dba)_3$ (180 mg, 0.19 mmol) and Xantphos (342 mg, 0.59 mmol) were added under argon atmosphere. The resulting reaction mixture was heated to 65 °C for 3 h in a sealed tube, cooled to RT, filtered through a celite pad and the pad was washed with EtOAc (2 x 10 mL). The filtrate was concentrated under reduced pressure. The resulting crude compound was dissolved in ethyl acetate (50 mL), washed with water (30 mL) and brine solution (30 mL). The organic layer was dried over $Na_2SO_4$, filtered and concentrated under reduced pressure. The crude material was purified by flash chromatography (eluting with 55% ethyl acetate in petroleum ether) to afford a pale yellow solid (1.3 g, 85%).
M/z 778.52 $(M+H)^+$

c. 5-[[4-[(3-amino-3-imino-propanoyl)amino]-3-fluorophenyl]sulfonylamino]thiazole-4-carboxylic acid

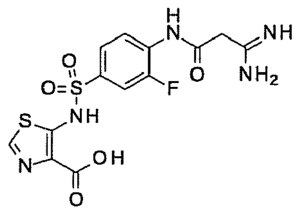

TFA (6 mL) was added to tert-butyl 5-[[4-[3,3-bis(tert-butoxycarbonylamino)prop-2-enoylamino]-3-fluoro-phenyl]sulfonyl-[(4-methoxyphenyl)methyl]amino]thiazole-4-carboxylate (600 mg, 0.77 mmol) at RT. The resulting mixture was stirred for 3 h and concentrated under reduced pressure. The resulting crude product was triturated with diethyl ether (2 x 10 mL) and dried under high vacuum. The crude product was purified by preparative HPLC affording the title product as an off-white solid (47 mg, 15%).

$^1H$ NMR (400 MHz, DMSO-$d_6$) $\delta$ 13.42 (1H, brs), 10.34 (1H, brs), 8.99 (2H, brs), 8.62 (2H, brs), 8.14-8.02 (2H, m), 7.58-7.50 (2H, m), 3.68 (2H, s).
M/z 402.3 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column used: PRONTOSIL (250*19) mm, 5u;
Mobile phase: (A) 0.1% Formic Acid, (B) Acetonitrile;
Flow: 19 mL/min;
Gradient -(T/%B): 0/5, 1/5, 7.3/59, 7.4/99, 11/99, 11.1/5, 14/5;
Solubility: ACN+THF+ H2O+formic acid.

**Examples 5 and 6**

**Example 5: 5-[[3-cyano-4-[(2-guanidinoacetyl)amino]phenyl]sulfonylamino] thiazole-4-carboxylic acid**

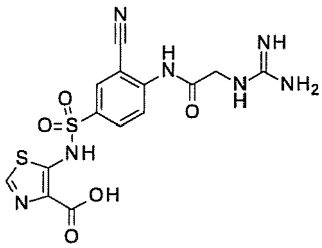

**Example 6: 5-[[3-carbamoyl-4-[(2-guanidinoacetyl)amino]phenyl]sulfonylamino] thiazole-4-carboxylic acid**

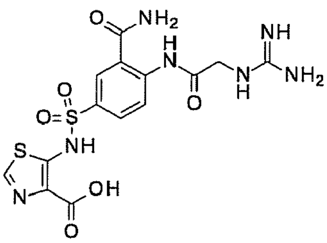

a. 4-bromo-3-cyano-benzenesulfonyl chloride

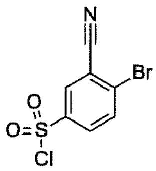

Solution-A: To a stirred solution of 5-amino-2-bromo-benzonitrile (2 g, 10.1 mmol) in AcOH (25 mL) was added conc. HCl (5 mL) at 0 °C and stirred for 10 minutes. Then $NaNO_2$ (770 mg, 11.1 mmol) in $H_2O$ (10 mL) was added at the same temperature and stirred for 20 minutes.
Solution-B: SO2 gas was purged in AcOH (25 mL) for 30 minutes. Then a solution of $CuCl_2$ (1.62 g, 12.2 mmol) in $H_2O$ (10 mL) was added at 0 °C and stirred for 20 minutes. After that, Solution-B was added drop wise to Solution-A. The reaction mixture was stirred at RT for 20 minutes and diluted with water (20 mL). The resulting precipitate was filtered, washed with n-pentane (2 x 20 mL) and dried under high vacuum to afford a yellow solid (1.7 g, 60%).

b. 5-[[3-cyano-4-[(2-hydroxyacetyl)amino]phenyl]sulfonyl-methyl-amino]thiazole-4-carboxylic acid

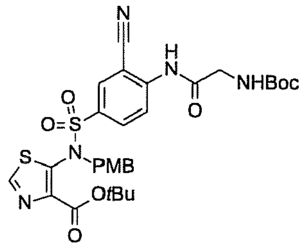

This compound was prepared following the procedure reported for Example 2 step b. M/z 658.8 $(M+H)^+$

c. 5-[[4-[(2-aminoacetyl)amino]-3,5-difluoro-phenyl]sulfonylamino]thiazole-4-carboxylic acid, trifluoroacetate

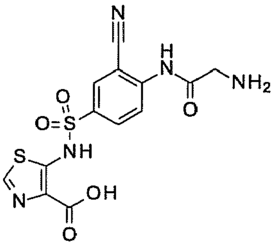

This compound was prepared following the procedure reported for Example 2 step c. M/z 382.4 $(M+H)^+$

d. 5-[[4-[(2-aminoacetyl)amino]-3-cyano-phenyl]sulfonylamino]thiazole-4-carboxylic acid and 5-[[4-[(2-aminoacetyl)amino]-3-carbamoylphenyl]sulfonylamino]thiazole-4-carboxylic acid

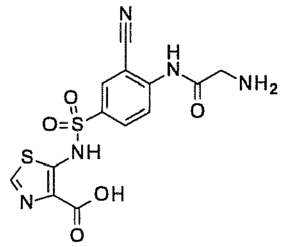

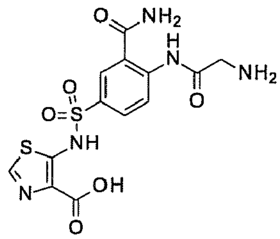

TFA: $H_2O$ (9:1, 5 mL) was added to tert-butyl 5-[[4-[[2-(tert-butoxycarbony)amino)acetyl]amino]-3-cyano-phenyl]sulfonyl-[(4-methoxyphenyl)methyl]amino]thiazole-4-carboxylate (400 mg, 0.60 mmol) at RT. The reaction mixture was stirred for 6 h and concentrated under reduced pressure. The resulting material was triturated with diethyl ether (2 x 10 mL) and dried under high vacuum to obtain a yellow solid which was used in the next step without further purification (300 mg, crude) (72% of 5-[[4-[(2-aminoacetyl)amino]-3-cyano-phenyl]sulfonylamino]thiazole-4-carboxylic acid and 8% of 5-[[4-[(2-aminoacetyl)amino]-3-carbamoylphenyl]sulfonylamino]thiazole-4-carboxylic acid).
M/z 382.05 $(M+H)^+$ and 400.01 $(M+H)^+$

e. 5-[[3-cyano-4-[(2-guanidinoacetyl)amino]phenyl]sulfonylamino]thiazole-4-carboxylic acid and 5-[[3-carbamoyl-4-[(2-guanidinoacetyl)amino]phenyl]sulfonylamino]thiazole-4-carboxylic acid

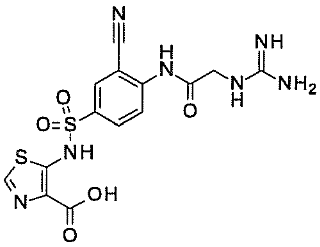

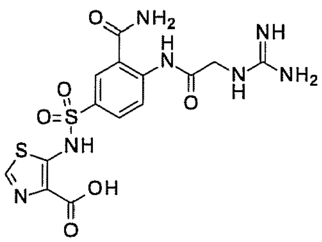

Pyrazole-1-carboxamidine (172 mg, 1.18 mmol) and DIPEA (0.3 mL, 1.57 mmol) were added to a stirred solution of 5-[[4-[(2-aminoacetyl)amino]-3-cyanophenyl]sulfonylamino]thiazole-4-carboxylic acid and 5-[[4-[(2-aminoacetyl)amino]-3-carbamoyl-phenyl]sulfonylamino]thiazole-4-carboxylic acid (300 mg, 0.78 mmol) in DMF (5 mL) at RT. The resulting reaction mixture was stirred at RT for 16 h and concentrated under reduced pressure. Water (5 mL) was added to the residue. The resulting precipitate was filtered and washed with diethyl ether (2 x 5 mL).The crude product was purified by preparative HPLC affording the title products:

*Example 5*

(72 mg, off-white solid):

$^1$H NMR (400 MHz, DMSO-$d_6$) $\delta$ 13.30 (1H, brs), 10.50 (1H, brs), 8.09 (1H, s), 8.02 (1H, d, *J* = 2.0 Hz), 7.99 (1H, dd, *J* = 8.8 Hz, *J* =2.0 Hz), 7.82 (1H, d, *J* = 8.8 Hz), 7.52 (2H, brs), 7.23 (3H, brs), 4.13 (2H, s).
M/z 424.34 $(M+H)^+$

*Example 6*

(5.2 mg, off-white solid):

$^1$H NMR (400 MHz, DMSO-$d_6$) $\delta$ 13.42 (1H, brs), 12.0 (1H, brs), 8.59 (1H, brs), 8.51 (1H, d), 8.20 (1H, d, *J* = 2.0 Hz), 8.03 (1H, s), 7.83 (1H, dd, *J* = 8.8 Hz, *J* =2.0 Hz), 7.80 (1H, brs), 7.44 (4H, brs), 4.07 (2H, s).
M/z 442.34 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: Symmetry C18 (150*25) mm, 10 u;

Mobile phase: (A) 0.05% Formic Acid (B) Acetonitrile;

Flow: 19 mL/min;

Gradient (T/%B): 0/5, 1/5, 5/20, 10.5/24, 10.52/99, 12/99, 12.02/5, 15/5;

Solubility: ACN +$H_2O$+THF+FA.

Compounds prepared using analogous methods to those described for Examples 2 to 6 and purified in a similar manner by preparative HPLC are shown in the Table below.

| Example | Structure | Name, NMR and mass |
|---|---|---|
| 7 | 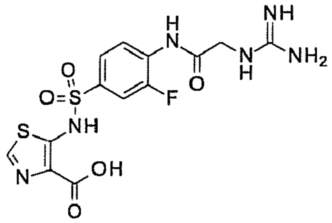 | 5-[[3-fluoro-4-[(2-guanidinoacetyl)amino]phenyl] sulfonylamino] thiazole-4-carboxylic acid<br><br>M/z 415.2 (M-Na)$^-$<br><br>$^1$H NMR (d6-DMSO) δ 13.1 (1H, s), 8.08 (1H, s), 7.93 (1H, brs), 7.48 (2H, d, *J* = 7 Hz), 3.88 (2H, s). |
| 8 | 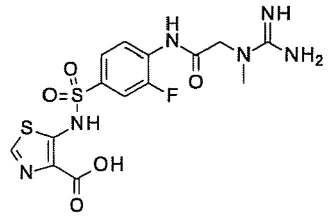 | 5-[[4-[[2-[carbamimidoyl(methyl) amino]acetyl]amino]-3-fluorophenyl]sulfonylamino]thiazole-4-carboxylic acid<br><br>M/z 431.2 (M+H)$^+$<br>$^1$H NMR (d6-DMSO) δ 13.30 (1H, s), 10.14 (1H, brs), 8.44 (1H, s), 8.13 (1H, m), 8.07 (1H, s), 7.57-7.32 (5H, brs), 4.27 (2H, s), 2.95 (3H, s). |
| 9 | 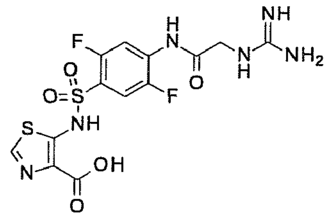 | 5-[[2,5-difluoro-4-[(2-guanidinoacetyl) amino]phenyl] sulfonylamino]thiazole-4-carboxylic acid<br><br>M/z 435.3 (M+H)$^+$<br><br>$^1$H NMR (d6-DMSO) δ 13.3 (1H, s), 10.3 (1H, brs), 8.1 (1H, s), 8.04 (1H, m), 7.57 (2H, m), 7.39-7.09 (4H, brs), 4.10 (2H, s). |
| 10 | 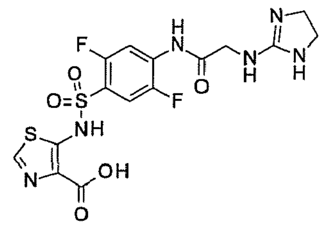 | 5-[[4-[[2-(4,5-dihydro-1H-imidazol-2-ylamino)acetyl]amino]-2,5-difluorophenyl]sulfonylamino]thiazole-4-carboxylic acid<br><br>M/z 461.3 (M+H)+<br><br>$^1$H NMR (d6-DMSO) δ 13.19 (1H, s), 10.1 (1H, brs), 8.39 (2H, brs), 8.10 (1H, s), 8.03 (1H, m), 7.55 (1H, m), 4.09 (2H, s), 3.60 (4H, s). |
| 11 | 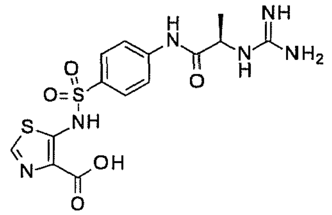 | 5-[[4-[[(2R)-2-guanidinopropanoyl] amino]phenyl]sulfonylamino] thiazole-4-carboxylic acid<br><br>M/z 413.3 (M+H)$^+$<br>$^1$H NMR (d6-DMSO) δ 13.6 (1H, s), 10.3 (1H, s), 8.03 (1H, s), 7.71 (5H, m), 7.25-6.96 (4H, brs), 4.25 (1H, t, *J* = 7.2 Hz), 1.40 (3H, d, *J* = 6.8 Hz). |
| 12 | 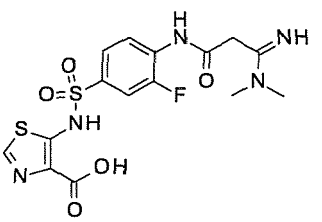 | 5-[[4-[[3-(dimethylamino)-3-imino-propanoyl]amino]-3-fluorophenyl]sulfonylamino]thiazole-4-carboxylic acid<br><br>M/z 430.3 (M+H)$^+$<br>$^1$H NMR (d6-DMSO) δ 13.5 (1H, brs), 9.51 (1H, brs), 8.45 (1H, t, *J* = 8 Hz), 8.19 (1H, s), 7.91 (2H, m), 4.22 (2H, s), 3.42 (3H, s), 3.35 (3H, s). |
| 13 | 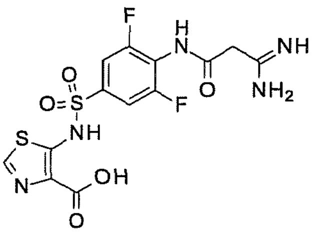 | 5-[[4-[(3-amino-3-imino-propanoyl)amino]-3,5-difluorophenyl] sulfonylamino]thiazole-4-carboxylic acid<br><br>M/z 420.3 (M+H)$^+$<br><br>$^1$H NMR (d6-DMSO) δ 13.21 (1H, s), 9.01 (2H, brs), 8.58 (2H, brs), 8.12 (1H, s), 7.43 (2H, d, *J* = 7.2 Hz), 3.62 (2H, s). |

(continued)

| Example | Structure | Name, NMR and mass |
|---|---|---|
| 14 | 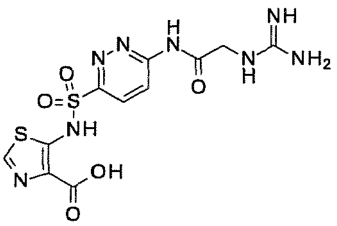 | 5-[[6-[(2-guanidinoacetyl)amino] pyridazin-3-yl]sulfonylamino] thiazole-4-carboxylic acid<br><br>M/z 401.4 $(M+H)^+$<br>$^1$H NMR (d6-DMSO) $\delta$ 13.1 (1H, brs), 11.5 (1H, brs), 8.45 (1H, s), 8.38 (1H, d, $J$ = 9.6 Hz), 8.21 (1H, brs), 8.10 (1H, s), 8.07 (1H, m), 7.89-7.68 (4H, brs), 4.13 (2H, s).<br><br>Key intermediate 6-chloro-3-pyridazinesulfonyl chloride was prepared as described in the literature, K. Ashton et al, WO2013/123444. |
| 15 | 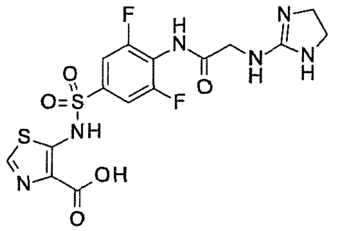 | 5-[[4-[[2-(4,5-dihydro-1H-imidazol-2-ylamino)acetyl]amino]-3,5-difluorophenyl]sulfonylamino]thiazole-4-carboxylic acid<br><br>M/z 461.3 (M+H)+<br><br>$^1$H NMR (d6-DMSO) $\delta$ 13.1 (1H, brs), 10.1 (1H, brs), 8.12 (1H, s), 7.42 (2H, d, $J$ = 9.6 Hz), 4.14 (2H, s), 3.59 (4H, s). |
| 16 | 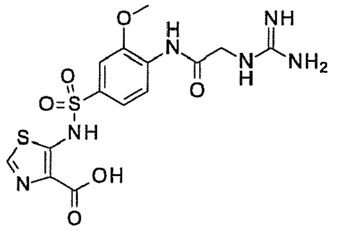 | 5-[[4-[(2-guanidinoacetyl)amino]-3-methoxy-phenyl] sulfonylamino]thiazole-4-carboxylic acid<br><br>M/z 429.3 $(M+H)^+$<br>$^1$H NMR (d6-DMSO) $\delta$ 13.59 (1H, brs), 9.41 (1H, brs), 8.44 (1H, s), 8.10 (1H, d, $J$ = 8.4 Hz), 8.04 (1H, s), 7.77 (1H, brs), 7.56-7.32 (4H, brs), 7.30 (2H, m), 4.09 (2H, s), 3.86 (3H, s).<br><br>The key intermediate 4-acetamido-3-methoxybenzenesulfonyl chloride was prepared by literature procedures, P. Patel et al, Bioorg Med Chem Lett, 2007, 17, 6610. |
| 17 | 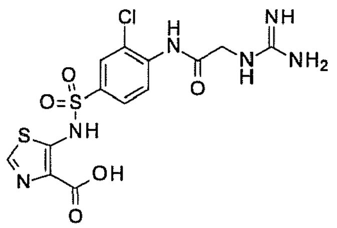 | 5-[[3-chloro-4-[(2-guanidinoacetyl) amino]phenyl]sulfonylamino] thiazole-4-carboxylic acid<br><br>M/z 433.3 $(M+H)^+$<br>$^1$H NMR (d6-DMSO) $\delta$ 13.4 (1H, brs), 9.81 (1H, brs), 8.43 (1H, s), 8.08 (1H, s), 7.95 (1H, d, $J$ = 8.4 Hz), 7.38 (1H, d, $J$ = 2 Hz), 7.67 (1H, m), 7.51-7.22 (4H, brs), 4.12 (2H, s).<br><br>The key intermediate 3-chloro-4-nitrobenzene-1-sulfonyl chloride is commercially available. |
| 16 | 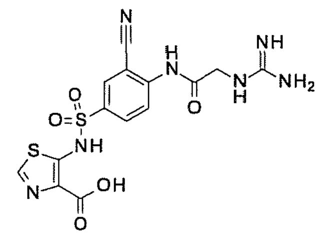 | 5-[[3-cyano-4-[(2-guanidinoacetyl)amino]phenyl]sulfonyla mino] thiazole-4-carboxylic acid<br><br>M/z 424.3 $(M+H)^+$<br>$^1$H NMR (d6-DMSO) $\delta$ 13.26 (1H, brs), 10.51 (1H, brs), 8.09 (1H, s), 8.03 (1H, m), 7.99 (1H, m), 7.83 (1H, d, $J$ = 8.8 Hz), 7.52 (1H, brs), 7.38-7.13 (4H, brs), 4.13 (2H, s). |

(continued)

| Example | Structure | Name, NMR and mass |
|---|---|---|
| 17 | 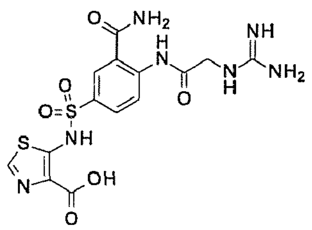 | 5-[[3-carbamoyl-4-[(2-guanidinoacetyl)amino]phenyl]sulfonylamino]thiazole-4-carboxylic acid<br><br>M/z 442.3 $(M+H)^+$<br>$^1H$ NMR (d6-DMSO) $\delta$ 13.41 (1H, brs), 12.01 (1H, brs), 8.59 (1H, brs), 8.51 (1H, d, $J$ = 8.8 Hz), 8.41 (1H, s), 8.20 (1H, d, $J$ = 2 Hz), 8.04 (1H, s), 7.84 (1H, m), 7.82 (1H, m), 7.54-7.30 (4H, brs), 4.07 (2H, s). |
| 18 | 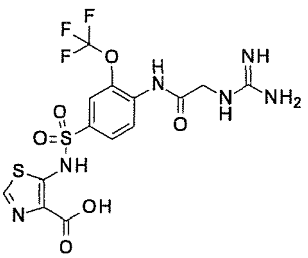 | 5-[[4-[(2-guanidinoacetyl)amino]-3-(trifluoromethoxy)phenyl] sulfonylamino] thiazole-4-carboxylic acid<br><br>M/z 483.0 $(M+H)^+$<br>$^1H$ NMR (d6-DMSO) $\delta$ 13.38 (1H, s), 10.1 (1H, brs), 8.17 (1H, d, $J$ = 9 Hz), 8.09 (1H, s), 7.72 (1H, m), 7.64 (1H, d, $J$ = 1.5 Hz), 7.54 (1H, brs), 7.39-7.25 (4H, brs), 4.12 (2H, s).<br><br>Key intermediate 4-bromo-3-(trifluoromethoxy) benzenesulfonyl chloride was prepared according to literature procedures, C-M. Park et al, J Med Chem, 2008, 51, 6902 |
| 19 | 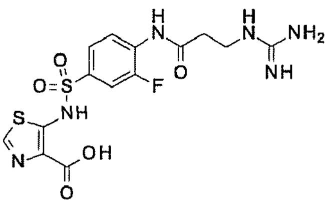 | 5-[[3-fluoro-4-(3-guanidinopropanoylamino)phenyl]sulfonylamino]thiazole-4-carboxylic acid<br><br>M/z 431.2 $(M+H)^+$<br>$^1H$ NMR (d6-DMSO) $\delta$ 10.19 (1H, s), 8.37 (1H, s), 8.18 (1H, t, $J$ = 8.1 Hz), 7.60 (2H, m), 7.46 (1H, m), 7.35-6.81 (4H, brs), 3.40 (2H, m), 2.70 (2H, t, $J$ = 6.3 Hz). |

**Example 20**

**5-[[4-[(2-guanidinoacetyl)amino]phenyl]sulfonylamino]thiazole-4-carboxylic acid**

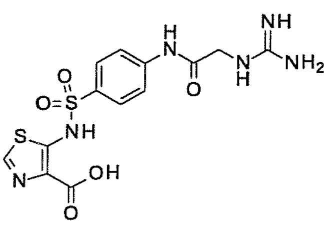

a. ethyl 5-[[4-[[2-(tert-butoxycarbonylamino)acetyl]amino]phenyl]sulfonylamino]thiazole-4-carboxylate

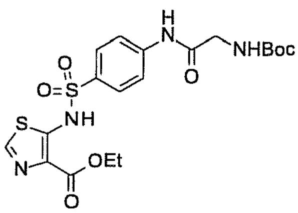

DIPEA (0.63 mL, 3.66 mmol) and HATU (696 mg, 1.83 mmol) were added to a stirred solution of 2-(tert-butoxycarbonylamino)acetic acid (321 mg, 1.83 mmol) in DMF (5 mL). The reaction mixture was stirred at RT for 15 minutes and then ethyl 5-[(4-aminophenyl)sulfonylamino]thiazole-4-carboxylate (400 mg, 1.22 mmol) was added at the same temperature under $N_2$ atmosphere. The resulting reaction mixture was stirred at RT for 16 h and concentrated under reduced pressure. The resulting crude compound was dissolved in 10% MeOH in DCM (20 mL), washed with sat $NH_4Cl$ (2 x 10 mL), water (10 mL) and brine solution (10 mL). The organic layer was dried over $Na_2SO_4$, filtered and concentrated under vacuum. The crude material was purified by column chromatography (eluting with 3% MeOH) affording an off-white solid (400 mg, 67%).
M/z 484.8 $(M+H)^+$ 507.06 $(M+Na)^+$

b. ethyl 5-[[4-[(2-aminoacetyl)amino]phenyl]sulfonylamino]thiazole-4-carboxylate

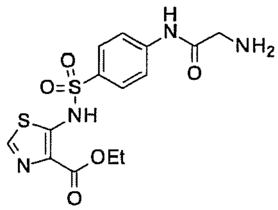

2N HCl in $Et_2O$ (4 mL) was added to ethyl 5-[[4-[[2-(tert-butoxycarbonylamino)acetyl]amino]phenyl]sulfonylamino]thiazole-4-carboxylate (400 mg, 0.82 mmol) in diethyl ether (5 mL) at 0 °C,. The reaction mixture was stirred for 5 h at RT and concentrated under reduced pressure. The crude product was purified by preparative HPLC (HCOOH/ $CH_3CN$/ $H_2O$) affording an off-white solid (300 mg, 94%).
M/z 385.13 $(M+H)^+$

c. ethyl 5-[[4-[[2-[[N,N'-bis(tert-butoxycarbonyl)carbamimidoyl]amino]acetyl]amino] phenyl]sulfonylamino]thiazole-4-carboxylate

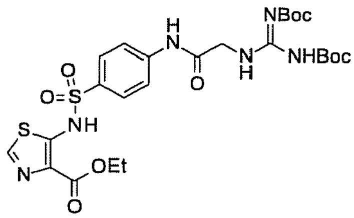

DIPEA (0.08 mL, 0.49 mmol) and tert-butyl N-[(tert-butoxycarbonylamino)-pyrazol-1-yl-methylene]carbamate (87 mg, 0.28 mmol) were added to a stirred solution of ethyl 5-[[4-[(2-aminoacetyl)amino]phenyl]sulfonylamino]thiazole-4-carboxylate (270 mg, 0.70 mmol) in DMF (5 mL) at RT. The resulting reaction mixture was stirred at RT for 16 h and concentrated under reduced pressure. The resulting crude compound was dissolved in 10% MeOH in DCM (20 mL), washed with water (10 mL) and brine solution (10 mL). The organic layer was dried over $Na_2SO_4$, filtered and concentrated under reduced pressure. The crude material was purified by column chromatography (eluting with 4% MeOH in DCM) affording an off white solid (250 mg, 56%).
M/z 626.97 $(M+H)^+$

d. 5-[[4-[[2-[[(Z)-N,N'-bis(tert-butoxycarbonyl)carbamimidoyl]amino]acetyl]amino]phenyl]sulfonylamino]thiazol e-4-carboxylic acid

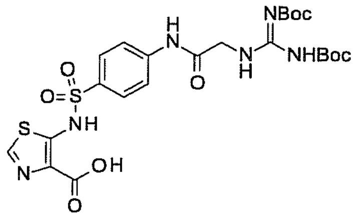

TMSOK (69 mg, 0.54 mmol) was added to a stirred solution of ethyl 5-[[4-[[2-[[N,N'-bis(tert-butoxycarbonyl)carbamimidoyl]amino]acetyl]amino]phenyl]sulfonylamino]thiazole-4-carboxylate (170 mg, 0.27 mmol) in THF (4 mL) at RT under $N_2$ atmosphere. The resulting reaction mixture was stirred at 40 °C for 5 h and concentrated under reduced pressure. The resulting crude product was triturated with diethyl ether (2 x 5 mL) and dried under high vacuum. The residue was dissolved in water (5 mL) and acidified with 1N HCl (adjusted pH~2). The resulting solid was filtered, washed with n-pentane and dried under high vacuum to afford an off-white solid which was used to next step without further purification (70 mg crude, 43%).
M/z 598.92 $(M+H)^+$

e. 5-[[4-[(2-guanidinoacetyl)amino]phenyl]sulfonylamino]thiazole-4-carboxylic acid

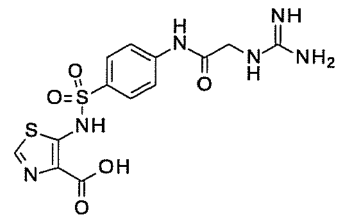

2N HCl in Ether (1 mL) was added to 5-[[4-[[2-[[(Z)-N,N'-bis(tert-butoxycarbonyl)carbamimidoyl]amino]acetyl]amino]phenyl]sulfonylamino]thiazole-4-carboxylic acid (70 mg, 0.11 mmol) in diethyl ether (2 mL) at 0 °C. The reaction mixture was stirred at RT for 5 h and concentrated under reduced pressure. The crude product was purified by preparative HPLC to afford the title product as an off white solid (11 mg, 23%).
$^1H$ NMR (500 MHz, DMSO-$d_6$) δ 13.59 (1H, s), 10.42 (1H, brs), 8.02 (1H, s), 7.68-7.63 (5H, m), 7.42 (4H, brs), 4.02 (2H, s).
M/z 398.78 $(M+H)^+$

**Example 21**

**5-[[4-[[2-(4,5-dihydro-1H-imidazol-2-yl)acetyl]amino]-3-fluoro-phenyl]sulfonylamino]thiazole-4-carboxylic acid**

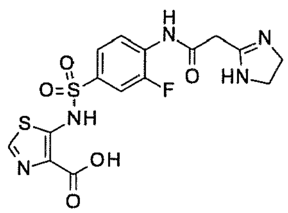

a. tert-butyl 5-[[4-[(2-cyanoacetyl)amino]-3-fluoro-phenyl]sulfonyl-[(4-methoxyphenyl)methyl]amino]thiazole-4-carboxylate

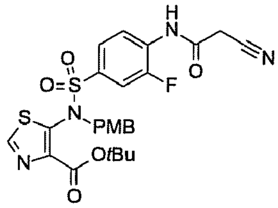

A solution of 2-cyanoacetic acid (86 mg, 1.01 mmol) and $PCl_5$ (210 mg, 1.01 mmol) in DCM (20 mL) was heated to reflux for 30 minutes. The reaction mixture temperature was cooled to RT and a solution of tert-butyl 5-[(4-amino-3-fluoro-phenyl)sulfonyl-[(4-methoxyphenyl)methyl]amino]thiazole-4-carboxylate (500 mg, 1.01 mmol) in DCM (30 mL) was added under nitrogen atmosphere. The resulting reaction mixture was heated to reflux for 2.5 h, cooled to RT, diluted with DCM (50 mL) and washed with aqueous $NaHCO_3$ solution (30 mL), water (30 mL) and brine (30 mL) solution. The organic layer was dried over $Na_2SO_4$, filtered and concentrated under reduced pressure. The crude material was purified by flash chromatography (eluting with 1-2% MeOH in DCM) to afford a pale yellow solid (180 mg, 31%).
M/z 561.43 $(M+H)^+$

b. tert-butyl 5-[[4-[[2-(4,5-dihydro-1H-imidazol-2-yl)acetyl]amino]-3-fluorophenyl]sulfonyl-[(4-methoxyphenyl)methyl]amino]thiazole-4-carboxylate

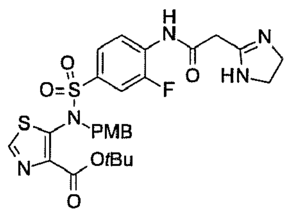

HCl gas was passed to a solution of tert-butyl 5-[[4-[(2-cyanoacetyl)amino]-3-fluorophenyl]sulfonyl-[(4-methoxyphenyl)methyl]amino]thiazole-4-carboxylate (300 mg, 0.53 mmol) in ethanol:$Et_2O$ (1:2, 30 mL) at 0 °C for 2 h. The resulting reaction mixture was kept in refrigerator for 16 h. Then the volatile components were evaporated under reduced pressure at 40 °C. The residue was dissolved in ethanol (10 mL) and ethylene diamine (48 mg, 0.80 mmol) was added at RT. The reaction mixture was stirred at RT for 16 h and concentrated under reduced pressure. The resulting crude product was triturated with diethyl ether (2 x 5mL) and dried under high vacuum to afford a pale brown solid which was used in the next step without further purification (330 mg, crude).
M/z 548.29 $(M\text{-}Boc+H)^+$

c. 5-[[4-[[2-(4,5-dihydro-1H-imidazol-2-yl)acetyl]amino]-3-fluorophenyl]sulfonylamino]thiazole-4-carboxylic acid

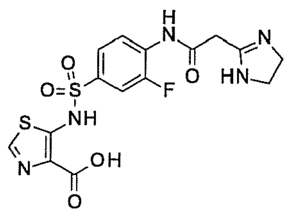

TFA (3 mL) was added to tert-butyl 5-[[4-[[2-(4,5-dihydro-1H-imidazol-2-yl)acetyl]amino]-3-fluoro-phenyl]sulfonyl-[(4-methoxyphenyl)methyl]amino]thiazole-4-carboxylate (300 mg, 0.54 mmol) at RT. The reaction mixture was stirred at RT for 4 h and concentrated under reduced pressure. The resulting crude product was triturated with diethyl ether (2 x 5 mL) and dried under high vacuum. The crude product was purified by preparative HPLC to afford the title product as an off-white solid (26 mg, 11%).
$^1H$ NMR (400 MHz, DMSO-$d_6$) $\delta$ 13.50 (1H, brs), 10.30 (1H, brs), 8.29 (2H, brs), 8.08-8.04 (2H, m), 7.54-7.48 (2H, m), 3.40 (2H, s), 3.36-3.28 (2H, obs), 2.88-2.85 (2H, m).

M/z 428.37 $(M+H)^+$

**Example 22**

**5-[[3-fluoro-4-[[3-imino-3-(methylamino)propanoyl]amino]phenyl]sulfonylamino] thiazole-4-carboxylic acid**

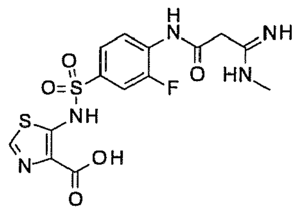

a. tert-butyl 5-[[3-fluoro-4-[[3-[hydroxy(methyl)amino]-3-imino-propanoyl]amino]phenyl]sulfonyl-[(4-methoxyphenyl)methyl]amino]thiazole-4-carboxylate

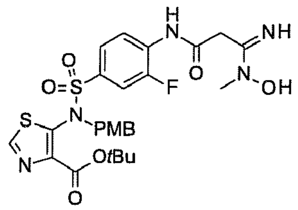

MeNHOH.HCl (298 mg, 3.56 mmol) and sodium carbonate (472 mg, 4.45 mmol) were added to a solution of tert-butyl 5-[[4-[(2-cyanoacetyl)amino]-3-fluoro-phenyl]sulfonyl-[(4-methoxyphenyl)methyl]amino]thiazole-4-carboxylate (1 g, 1.78 mmol) in ethanol (15 mL) at RT. The resulting reaction mixture was stirred at 60 ° C for 3 h, cooled to RT, filtered and washed with ethanol (2x10 mL). The combined organic layer was concentrated under reduced pressure. The obtained crude compound was triturated with $Et_2O$ (2x10 mL) and dried under high vacuum to afford a brown solid which was used in next step without further purification.
M/z 608.03 $(M+H)^+$

b. tert-butyl 5-[[3-fluoro-4-[[3-imino-3-(methylamino)propanoyl]amino]phenyl]sulfonyl-[(4-methoxyphenyl)methyl]amino]thiazole-4-carboxylate

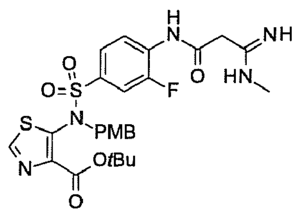

Bis(pinacolato)diboron (*Adv. Synth. catal. 2015, 357, 451-462*) (357 mg, 1.4 mmol) was added to a solution of tert-butyl 5-[[3-fluoro-4-[[3-[hydroxy(methyl)amino]-3-imino-propanoyl]amino]phenyl]sulfonyl-[(4-methoxyphenyl)methyl]amino]thiazole-4-carboxylate (570 mg, 0.93 mmol) in acetonitrile (10 mL) at RT. The resulting reaction mixture was stirred at RT for 1 h and concentrated under reduced pressure. The crude compound was purified by flash chromatography (eluting with 2% triethyl amine in 10% methanol and DCM) to afford a pale yellow solid (130 mg, 23%).
M/z 592.05 $(M+H)^+$

c. 5-[[3-fluoro-4-[[3-imino-3-(methylamino)propanoyl]amino]phenyl]sulfonylamino] thiazole-4-carboxylic acid

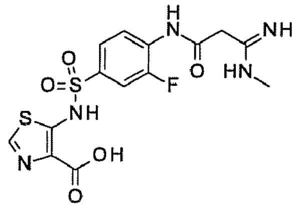

TFA (3 mL) was added to tert-butyl 5-[[3-fluoro-4-[[3-imino-3-(methylamino)propanoyl]amino]phenyl]sulfonyl-[(4-methoxyphenyl)methyl]amino]thiazole-4-carboxylate (130 mg, 0.21 mmol) at RT. The reaction mixture was stirred for 2 h at RT and concentrated under reduced pressure. The resulting crude product was triturated with diethyl ether (2 x 5 mL) and dried under high vacuum. The crude product was purified by preparative HPLC affording the title product as yellow solid (20 mg, 22%).
$^1$H NMR (400 MHz, $CF_3COOD$) δ 9.53 (1H, brs), 8.42 (1H, t, *J* = 8.0 Hz), 8.20 (1H, s), 7.92 (1H, d, *J* = 8.8 Hz), 7.88 (1H, d, *J* = 9.2 Hz), 4.08 (2H, s), 3.18 (3H, s).
M/z 416.34 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: Symmetry C18 (300*19) mm, 7 u;
Mobile phase: (A) 0.1% Formic Acid (B) Acetonitrile;
Flow: 19 mL/min;
Gradient (T/%B): 0/5, 1/5, 8.9/40, 8.92/99, 12/99, 12.1/5, 15/5;
Solubility: ACN +$H_2O$+THF.

**Example 23**

**5-[[2-[(2-guanidinoacetyl)amino]thiazol-5-yl]sulfonylamino]thiazole-4-carboxylic acid**

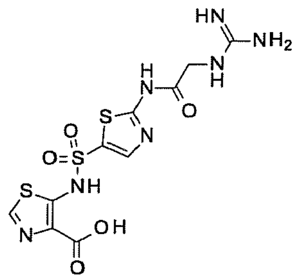

a. 2-acetamidothiazole-5-sulfonyl chloride

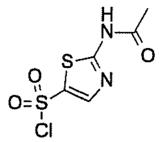

N-thiazol-2-ylacetamide (5 g, 35.2 mmol) was added portion wise to a solution of chlorosulfonic acid (11.7 mL, 176 mmol) at 0 °C. The reaction mixture was stirred at 100 °C for 4 h, cooled to RT and poured into ice cold water (100 mL). The resulting precipitate was filtered and washed with water (20 mL). The precipitate was triturated with *n*-pentane (2 x 20 mL) and azeotroped with toluene to afford an off-white solid which was used in the next step without further purification (2 g crude, 23%).
M/z 241.23 $(M+H)^+$

b. ethyl 5-[(2-acetamidothiazol-5-yl)sulfonylamino]thiazole-4-carboxylate

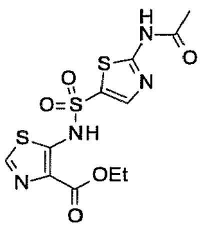

A solution of ethyl 5-aminothiazole-4-carboxylate (300 mg, 1.74 mmol) in THF (10 mL) was added to a stirred solution ofNaH (250 mg, 10.4 mmol) in THF (10 mL) at 0 °C and stirred for 5 minutes. Then a solution of 2-acetamidothiazole-5-sulfonyl chloride (502 mg, 2.0 mmol) in THF (10 mL) was added to the reaction mixture at 0 °C. The reaction mixture was stirred at the same temperature for 1 h. Ice cold water (30 mL) was added to the reaction mixture which was then washed with EtOAc (2 x 15 mL). The aqueous layer was acidified to pH 2.0 using 1N HCl and extracted with EtOAc (3 x 15 mL). The organic layer was dried over $Na_2SO_4$, filtered and concentrated under reduced pressure to afford a pale brown solid which was used to next step without further purification (175 mg crude, 26%).
M/z 377.32 $(M+H)^+$

c. ethyl 5-[(2-aminothiazol-5-yl)sulfonylamino]thiazole-4-carboxylate, hydrochloride

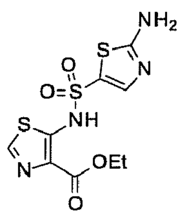

Concentrated HCl (7 mL) was added to a solution of ethyl 5-[(2-acetamidothiazol-5-yl)sulfonylamino]thiazole-4-carboxylate (700 mg, 1.86 mmol) in ethanol (70 mL) at RT. The reaction mixture was refluxed for 5 h and concentrated under reduced pressure. The resulting crude compound was washed with diethyl ether (20 mL), n-pentane (20 mL) and dried under high vacuum to afford a brown solid which was used in the next step without further purification (600 mg, crude).
M/z 335.04 $(M+H)^+$

d. ethyl 5-[[2-[[2-(tert-butoxycarbonylamino)acetyl]amino]thiazol-5-yl]sulfonylamino]thiazole-4-carboxylate

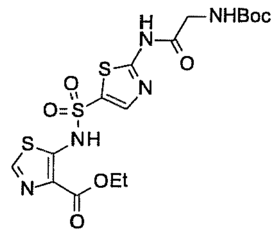

HATU (1.36 g, 3.58 mmol) and DIPEA (2.5 mL, 14.3 mmol) were added to a stirred solution of 2-(tert-butoxycarbonylamino)acetic acid (628 mg, 3.58 mmol) in DMF (6 mL) at RT. The reaction mixture was stirred at RT for 15 minutes and then ethyl 5-[(2-aminothiazol-5-yl)sulfonylamino]thiazole-4-carboxylate, hydrochloride (600 mg, 1.79 mmol) was added at the same temperature under $N_2$ atmosphere. The resulting reaction mixture was stirred at RT for 18 h. Ice cold water (30 mL) was added and extracted with DCM (3 x 20 mL). The organic layer was dried over $Na_2SO_4$, filtered and concentrated under reduced pressure. The crude compound was purified by flash chromatography (eluting with using 60-80% of EtOAc in petroleum ether) affording a brown solid (400 mg, 45%).
M/z 492.34 $(M+H)^+$

e. 5-[[2-[[2-(tert-butoxycarbonylamino)acetyl]amino]thiazol-5-yl]sulfonylamino]thiazole-4-carboxylic acid

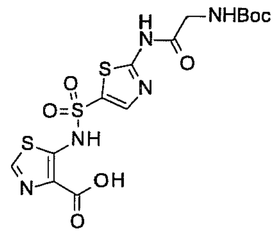

TMSOK (625 mg, 4.8 mmol) was added to a stirred solution of ethyl 5-[[2-[[2-(tert-butoxycarbonylamino)acetyl]amino]thiazol-5-yl]sulfonylamino]thiazole-4-carboxylate (400 mg, 0.8 mmol) in THF (40 mL) at RT. The reaction mixture was stirred at 40 °C for 1 h, concentrated under reduced pressure and water (2 mL) was added to the residue. The reaction mixture was acidified to pH 2 using 1N HCl. The resulting precipitate was filtered, washed with diethyl ether (2 x 10 mL), n-pentane (10 mL) and dried under high vacuum to afford a pale yellow solid (200 mg, 53%).
M/z 464.30 $(M+H)^+$

f. 5-[[2-[(2-aminoacetyl)amino]thiazol-5-yl]sulfonylamino]thiazole-4-carboxylic acid, hydrochloride

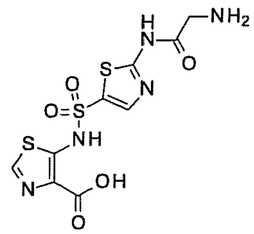

HCl in $Et_2O$ (2M, 10 mL) was added to 5-[[2-[[2-(tert-butoxycarbonylamino)acetyl]amino]thiazol-5-yl]sulfonylamino]thiazole-4-carboxylic acid (200 mg, 0.43 mmol) at RT. The reaction mixture was stirred at the same temperature for 3 h, concentrated under reduced pressure and the resulting residue was washed with diethyl ether (2 x 5 mL) and

*n*-pentane (5 mL) affording a pale yellow solid which was used in the next step without further purification (150 mg, crude). M/z 364.30 $(M+H)^+$

g. 5-[[2-[(2-guanidinoacetyl)amino]thiazol-5-yl]sulfonylamino]thiazole-4-carboxylic acid

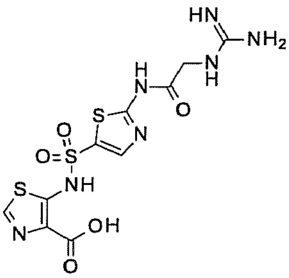

DIPEA (0.44 mL, 2.7 mmol) was added to a stirred solution of 5-[[2-[(2-aminoacetyl)amino]thiazol-5-yl]sulfonylamino]thiazole-4-carboxylic acid, hydrochloride (100 mg, 0.27 mmol) and pyrazole-1-carboxamidine, hydrochloride (80 mg, 0.55 mmol) in DMF (2 mL) at RT. The reaction mixture was stirred at the same temperature for 6 h. DMF was evaporated and then water (3 mL) was added to the resulting crude material, stirring for 5 minutes. The resulting precipitate was filtered and washed with water (2 x 2 mL) then dried under high vacuum. The crude compound was purified by preparative HPLC affording an off-white solid (16 mg, 14%).

$^1H$ NMR (400 MHz, DMSO-$d_6$) $\delta$ 13.28 (1H, s), 12.6 (1H, brs), 8.15 (1H, s), 7.71 (1H, s), 7.44 (1H, t, *J* = 6.4 Hz), 7.21 (4H, brs), 4.11 (2H, d, *J* = 6.4 Hz).
M/z 405.9 $(M+H)^+$

**LC-MS Condition:**

Column: Acquity BEH C18 (50 mm x 2.1 mm, 1.7um)
Mobile Phase: A: 0.05% Formic Acid in water; B: 0.05% Formic Acid in ACN
Gradient: Time (min) /%B: 0/3, 0.4/3, 3.2/98, 3.8/98, 4.2/3, 4.5/3
Column Temp: 35 °C,
Flow Rate: 0.6 mL/min

**Prep. HPLC Condition:**

Column used: Atlantis T3 (250*19) mm, 5u;
Mobile phase: (A) 0.1% Formic Acid (B) Acetonitrile
Flow: 19 mL/min
Gradient -(T/%B): 0/5, 1/5, 8.2/55, 8.21/99, 10/99, 10.1/5, 13/5
Diluent: ACN +$H_2O$+FA

**Example 24**

**5-[[4-[2-(2-carbamimidoylhydrazino)-2-oxo-ethyl]-3-fluoro-phenyl]sulfonylamino]thiazole-4-carboxylic acid**

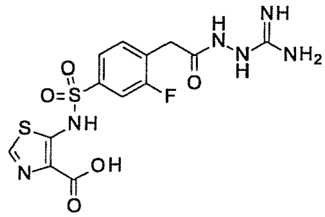

a. tert-butyl 5-[[4-(2-ethoxy-2-oxo-ethyl)-3-fluoro-phenyl]sulfonyl-[(4-methoxyphenyl)methyl]amino]thiazole-4-carboxylate

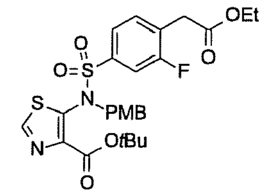

A mixture of tert-butyl 5-[(4-bromo-3-fluoro-phenyl)sulfonyl-[(4-methoxyphenyl)methyl]amino]thiazole-4-carboxylate (2 g, 3.58 mmol), potassium 3-ethoxy-3-oxo-propanoate (1.2 g, 7.16 mmol) and DMAP (43 mg, 0.35 mmol) in mesitylene (20 mL) was purged with argon gas for 30 minutes. BINAP (222 mg, 0.35 mmol) and Pd2(dba)3 (327 mg, 0.35 mmol) were then added at the same temperature. The reaction mixture was stirred at 120 °C for 18 h, cooled to RT and concentrated under reduced pressure. The crude product was purified by flash chromatography (eluting with 40% EtOAc in petroleum ether) to afford a yellow solid which was used in the next step without further purification (0.45 g, crude).
M/z 565.43 (M+H)+

b. tert-butyl 5-[[3-fluoro-4-(2-hydrazino-2-oxo-ethy1)phenyl]sulfonyl-[(4-methoxyphenyl)methyl]amino]thiazole-4-carboxylate

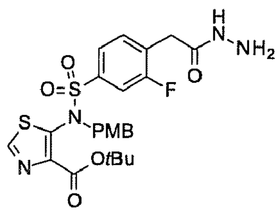

Hydrazine hydrate (709 mg, 14.1 mmol) was added to a stirred solution of tert-butyl 5-[[4-(2-ethoxy-2-oxo-ethyl)-3-fluoro-phenyl]sulfonyl-[(4-methoxyphenyl)methyl]amino]thiazole-4-carboxylate (400 mg, 0.7 mmol) in ethanol (20 mL) at RT. The reaction mixture was refluxed for 5 h and concentrated under reduced pressure to afford a brown solid which was used in the next step without further purification (350 mg, crude).
M/z 551.42 $(M+H)^+$

c. tert-butyl 5-[[4-[2-[2-N,N'-bis(tert-butoxycarbonyl)carbamimidoyl]hydrazino]-2-oxo-ethyl]-3-fluoro-phenyl]sulfonyl-[(4-methoxyphenyl)methyl]amino]thiazole-4-carboxylate

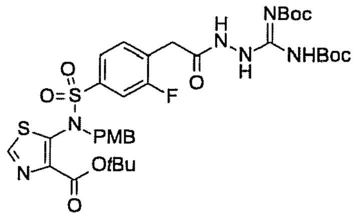

DIPEA (0.32 mL, 1.89 mmol) was added to a stirred solution of tert-butyl 5-[[3-fluoro-4-(2-hydrazino-2-oxo-ethyl)phenyl]sulfonyl-[(4-methoxyphenyl)methyl]amino]thiazole-4-carboxylate (350 mg, 0.63 mmol) and tert-butyl N-N-[(tert-butoxycarbonylamino)-pyrazol-1-yl-methylene]carbamate (394 mg, 1.27 mmol) in DMF (5 mL) at RT. The reaction mixture was stirred at the same temperature for 18 h. Ice cold water was added to the reaction mixture and stirred for 10 minutes. The resulting precipitate was filtered, washed with water (2x5 mL) and dried under high vacuum. The crude product was purified by flash chromatography (eluting with 60% EtOAc in petroleum ether) to afford a yellow solid (120 mg, 23%).
M/z 793.53 $(M+H)^+$

d. 5-[[4-[2-(2-carbamimidoylhydrazino)-2-oxo-ethyl]-3-fluorophenyl]sulfonylamino]thiazole-4-carboxylic acid

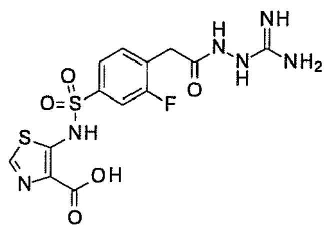

TFA (2 mL) was added to tert-butyl 5-[[4-[2-[2-[(Z)-N,N'-bis(tert-butoxycarbonyl)carbamimidoyl]hydrazino]-2-oxo-ethyl]-3-fluoro-phenyl]sulfonyl-[(4-methoxyphenyl)methyl]amino]thiazole-4-carboxylate (120 mg, 0.15 mmol) at RT. The reaction mixture was stirred for 3 h at the same temperature and concentrated under reduced pressure. The resulting crude material was triturated with diethyl ether (2x5 mL). The crude product was purified by preparative HPLC affording the title product as an off-white solid (23 mg).

$^1H$ NMR (400 MHz, DMSO-$d_6$) $\delta$ 13.40 (1H, brs), 8.06 (1H, s), 7.63 (3H, brs), 7.51-7.39 (3H, m), 3.56 (2H, s).
M/z 417.35 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column used: Symmetry C18 (300*19) mm, 7 u;
Mobile phase: (A) 0.05% Formic Acid (B) Acetonitrile;
Flow: 19 mL/min;
Gradient (T/%B): 0/2, 1/2, 8/20, 10.5/20, 10.51/99, 12/99, 12.1/2, 15/2;
Solubility: ACN+H2O+THF.

Compounds prepared by analogous methods to those described above for Examples 20 to 24 and purified in a similar manner by preparative HPLC are shown in the Table below:-

| Example | Structure | Name, NMR and Mass |
|---|---|---|
| 25 | 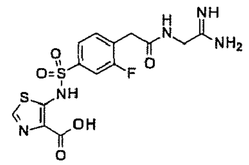 | 5-[[4-[2-[(2-amino-2-imino-ethyl)amino]-2-oxo-ethyl]-3-fluoro-phenyl] sulfonylamino] thiazole-4-carboxylic acid<br><br>M/z 416.1 (M+H)+<br>$^1$H NMR (d6-DMSO) δ 13.1 (1H, s), 8.67 (1H, brs), 8.49 (3H, brs), 8.07 (1H, brs), 7.48 (1H, m), 7.43 (2H, m), 3.89 (2H, s), 3.61 (2H, s), 2.07 (1H, s). |

**Example 26**

**5-[[3,5-difluoro-4-(guanidinocarbamoylamino)phenyl]sulfonylamino]thiazole-4-carboxylic acid**

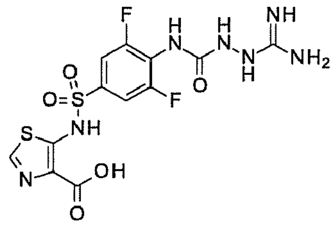

a. tert-butyl 5-[(4-amino-3,5-difluoro-phenyl)sulfonyl-[(4-methoxyphenyl)methyl]amino]thiazole-4-carboxylate

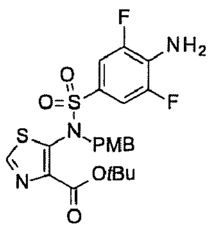

A saturated solution of $NH_3$ in dioxane (120 mL) was added to a mixture of tert-butyl 5-[(4-bromo-3,5-difluoro-phenyl)sulfonyl-[(4-methoxyphenyl)methyl]amino]thiazole-4-carboxylate (2 g, 3.47 mmol), Xantphos (0.6 g, 1.04 mmol), $Pd_2(dba)_3$ (0.317 g, 0.34 mmol) and $K_3PO_4$ (2.2 g, 10.4 mmol). The resulting mixture was stirred in sealed tube at 100 °C for 5 h, filtered through Celite pad and the pad was washed with ethyl acetate (2 x 25 mL). The filtrate was concentrated under reduced pressure. The crude material was purified by flash chromatography (eluting with 50% ethyl acetate in petroleum ether) affording a pale yellow solid (1.25 g, 70%).
M/z 512.4 (M+H)+ ; 534.56 (M+Na)+

b. tert-butyl 5-[[3,5-difluoro-4-[(4-nitrophenoxy)carbonylamino]phenyl]sulfonyl-[(4-methoxyphenyl)methyl]amino]thiazole-4-carboxylate

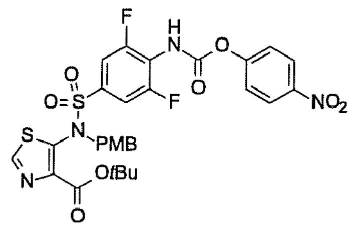

(4-nitrophenyl) carbonochloridate (1.57 g, 7.82 mmol) was added to a stirred solution tert-butyl 5-[(4-amino-3,5-difluoro-phenyl)sulfonyl-[(4-methoxyphenyl)methyl]amino]thiazole-4-carboxylate (2 g, 3.91 mmol) in toluene (120 mL) at room temperature and refluxed for 3 h. The reaction mixture was concentrated under reduced pressure. The crude product was used in the next step without further purification (3.5 g, crude).

c. tert-butyl 5-[[4-[(tert-butoxycarbonylamino)carbamoylamino]-3,5-difluorophenyl]sulfonyl-[(4-methoxyphenyl)methyl]amino]thiazole-4-carboxylate

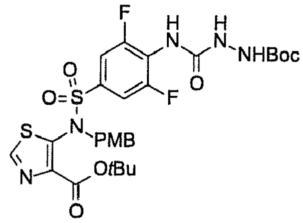

DIPEA (2.6 mL, 15.5 mmol) was added to a suspension of tert-butyl 5-[[3,5-difluoro-4-[(4-nitrophenoxy)carbonylamino]phenyl]sulfonyl-[(4-methoxyphenyl)methyl]amino]thiazole-4-carboxylate (3.5 g, 5.17 mmol) and tert-butyl N-aminocarbamate (1.36 g, 10.3 mmol) in THF (100 mL) at 0 °C. The reaction mixture was stirred at RT for 3 h and concentrated under reduced pressure. The crude material was purified by flash chromatography (eluting with 70% ethyl acetate in petroleum ether) affording a pale yellow solid (1.5 g, 43%).
M/z 670.4 $(M+H)^+$

d. 5-[[3,5-difluoro-4-(hydrazinecarbonylamino)phenyl]sulfonyl-[(4-methoxyphenyl)methyl]amino]thiazole-4-carboxylic acid, hydrochloride

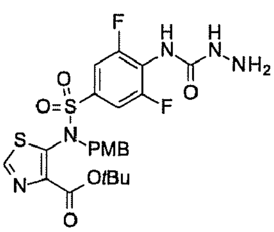

HCl in $Et_2O$ (2M, 200 mL) was added to tert-butyl 5-[[4-[(tert-butoxycarbonylamino)carbamoylamino]-3,5-difluoro-phenyl]sulfonyl-[(4-methoxyphenyl)methyl]amino]thiazole-4-carboxylate (1.5 g, 2.24 mmol) at RT. The reaction mixture was stirred for 24 h, cooled to 0 °C for 30 minutes and $Et_2O$ was decanted. The crude product was triturated with diethyl ether (2 x 40 mL) and dried under high vacuum affording an off-white solid (1 g, crude).
M/z 514.3 $(M+H)^+$

e. 5-[[4-[[[N,N'-bis(tert-butoxycarbonyl)carbamimidoyl]amino]carbamoylamino]-3,5-difluoro-phenyl]sulfonyl-[(4-methoxyphenyl)methyl]amino]thiazole-4-carboxylic acid

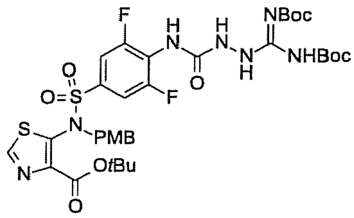

DIPEA (3.0 mL, 17.5 mmol) was added to a stirred solution of 5-[[3,5-difluoro-4-(hydrazinecarbonylamino)phenyl]sulfonyl-[(4-methoxyphenyl)methyl]amino]thiazole-4-carboxylic acid, hydrochloride (1 g, 1.75 mmol) and tert-butyl (NZ)-N-[(tert-butoxycarbonylamino)-pyrazol-1-yl-methylene]carbamate (0.54 g, 1.75 mmol) in DMF (6 mL) at RT. The reaction mixture was stirred for 5 h and DMF was removed . Then water was added to the crude product, stirring for 5 minutes. The resulting precipitate was filtered, washed with water (2 x 5 mL) and dried under high vacuum affording an off-white solid (1.25 g, crude).
M/z 756.1 $(M+H)^+$

f. 5-[[3,5-difluoro-4-(guanidinocarbamoylamino)phenyl]sulfonylamino]thiazole-4-carboxylic acid

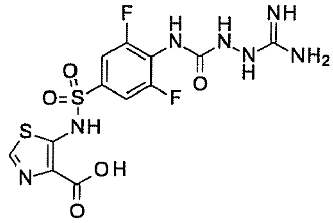

TFA (13 mL) was added to 5-[[4-[[[(Z)-N,N'-bis(tert-butoxycarbonyl)carbamimidoyl]amino]carbamoylamino]-3,5-difluoro-phenyl]sulfonyl-[(4-methoxyphenyl)methyl]amino]thiazole-4-carboxylic acid (1.25 g, 1.54 mmol) at RT. The reaction mixture was stirred for 3 h at RT and TFA was evaporated by flushing with $N_2$ gas. The resulting crude product was triturated with diethyl ether and purified by preparative HPLC to afford the title compound as a white solid (150 mg).
$^1$H NMR (300 MHz, DMSO-$d_6$) $\delta$ 13.28 (1H, brs), 8.70 (3H, br s), 8.12 (1H, s), 7.39 (2H, d, $J$= 6.9 Hz), 7.0 -7.37 (3H, br s).
M/z 436.0 $(M+H)^+$

**LC-MS Condition:**

Column: Aquity UPLC BEH C18 (50 mm x 2.1 mm, 1.7 um);
Mobile phase: A: 0.1% of Formic Acid in Water, B: 0.1% of Formic acid in Acetonitrile;
Gradient: Time (min)/ %B 0/2, 0.2/2, 1.5/98, 2.6/98, 2.61/2, 3.2/2;
Column Temp: 45 °C, Flow rate: 0.8 mL/min

**Prep. HPLC Condition:**

Column: X select C18 (150*30 mm), 5u;
Mobile Phase: 0.05% Formic acid in $H_2O$: Acetonitrile;
Flow: 25 mL/min;

Gradient (T/%B): 0/50, 8/50, 8/40, 9/40, 9.1/98, 11/98, 11.1/5, 14/40
Diluent: ACN+$H_2O$+MeOH+THF.

**Example 27**

**5-[[3-fluoro-4-(2-guanidinoethoxycarbonylamino)phenyl]sulfonylamino]thiazole-4-carboxylic acid**

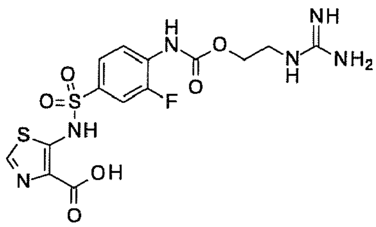

a. tert-butyl 5-[[3-fluoro-4-[(4-nitrophenoxy)carbonylamino]phenyl]sulfonyl-[(4-methoxyphenyl)methyl]amino]thiazole-4-carboxylate

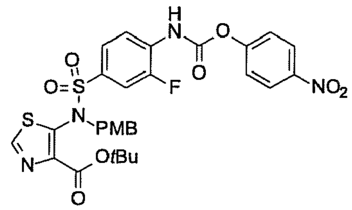

p-Nitrophenyl chloroformate (1.33 g, 6.0 mmol) was added to a stirred solution of tert-butyl 5-[(4-amino-3-fluoro-phenyl)sulfonyl-[(4-methoxyphenyl)methyl]amino]thiazole-4-carboxylate (1 g, 2.02 mmol) in toluene (30 mL) at RT. The resulting reaction mixture was stirred at 120 °C for 1 h and concentrated under reduced pressure. The resulting crude product was triturated with n-pentane (2 x 10 mL) and dried under high vacuum affording an off-white solid which was used in the next step without further purification (1.5 g, crude).
M/z 659.43 $(M+H)^+$

b. tert-butyl 5-[[4-[2-(tert-butoxycarbonylamino)ethoxycarbonylamino]-3-fluorophenyl]sulfonyl-[(4-methoxyphenyl)methyl]amino]thiazole-4-carboxylate

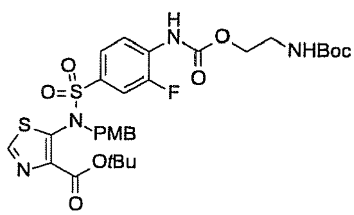

Tert-butyl N-(2-hydroxyethyl)carbamate (440 mg, 2.73 mmol) and DIPEA (0.97 mL, 5.46 mmol) were added to a stirred solution of tert-butyl 5-[[3-fluoro-4-[(4-nitrophenoxy)carbonylamino]phenyl]sulfonyl-[(4-methoxyphenyl)methyl]amino]thiazole-4-carboxylate (1.2 g, 1.82 mmol) in THF (20 mL) at RT. The resulting reaction mixture was stirred at RT for 2 h. Ice cold water was added followed by extraction with ethyl acetate (2 x 50 mL). The combined organic layer was dried over anhydrous $Na_2SO_4$, filtered and concentrated under reduced pressure. The crude material was purified by flash chromatography (eluting with 40% ethyl acetate in petroleum ether) affording an off-white solid (500 mg, 40%).

M/z 681.50 (M+H)+

c. 5-[[4-(2-aminoethoxycarbonylamino)-3-fluoro-phenyl]sulfonylamino]thiazole-4-carboxylic acid, trifluoroacetate

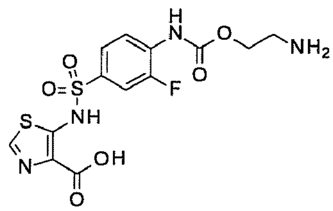

TFA (5 mL) was added to tert-butyl 5-[[4-[2-(tert-butoxycarbonylamino)ethoxycarbonylamino]-3-fluoro-phenyl]sulfonyl-[(4-methoxyphenyl)methyl]amino]thiazole-4-carboxylate (450 mg, 0.66 mmol) at RT. The reaction mixture was stirred for 24 h at the same temperature and concentrated under reduced pressure. The resulting crude product was triturated with diethyl ether (2 x 10 mL) and dried under high vacuum to afford an off-white solid which was used in the next step without further purification (350 mg, crude).
M/z 405.36 (M+H)+

d. 5-[[3-fluoro-4-(2-guanidinoethoxycarbonylamino)phenyl]sulfonylamino]thiazole-4-carboxylic acid

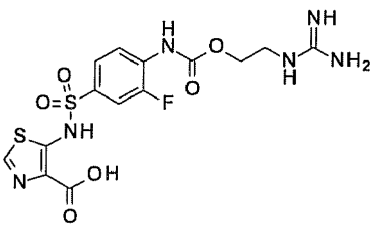

Pyrazole-1-carboxamidine, hydrochloride (136 mg, 0.92 mmol) and DIPEA (0.55 mL, 3.0 mmol) were added to a stirred solution of 5-[[4-(2-aminoethoxycarbonylamino)-3-fluorophenyl]sulfonylamino]thiazole-4-carboxylic acid, trifluoroacetate (250 mg, 0.61 mmol) in DMF (6 mL) at RT. The resulting reaction mixture was stirred at RT for 4 h concentrated under reduced pressure and water (5 mL) was added to the residue. The resulting precipitate was filtered, washed with diethyl ether (2 x 5 mL) and dried under high vacuum. The crude product was purified by preparative HPLC affording the title product as an off-white solid (45 mg, 16%).

$^1$H NMR (300 MHz, DMSO-$d_6$) δ 13.42 (1H, brs), 9.60 (1H, s), 8.07 (1H, s), 7.79 (1H, t, *J* = 8.0 Hz), 7.62-7.56 (1H, m), 7.51 (1H, d, *J* = 8.0 Hz, *J* = 2.0 Hz), 7.46 (1H, dd, *J* = 10.4 Hz, 2.0 Hz), 7.12 (4H, brs), 4.18 (2H, t, *J* = 5.2 Hz), 3.48-3.40 (2H, m).
M/z 447.27 (M+H)+

Compounds prepared using analogous methods to those described for Examples 26 and 27 and purified in a similar manner by preparative HPLC are shown in the Table below:

| Example | Structure | Name, NMR and Mass |
|---|---|---|
| 28 | 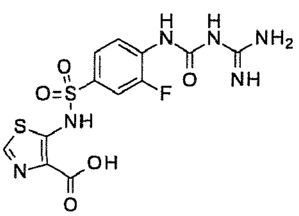 | 5-[[4-(carbamimidoylcarbamoyl-amino)-3-fluoro-phenyl] sulfonylamino] thiazole-4-carboxylic acid<br><br>M/z 402.9 (M+H)+<br><br>$^1$H NMR (d6-DMSO) δ 13.5 (1H, brs), 8.18 (1H, brs), 8.13 (1H, m), 8.04 (1H, s), 7.72 (1H, brs), 7.40 (2H, m), 6.96-6.61 (4H, m). |

(continued)

| Example | Structure | Name, NMR and Mass |
|---|---|---|
| 29 | 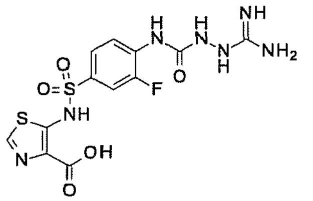 | 5-[[3-fluoro-4-(guanidinocarbamoylamino) phenyl]sulfonylamino] thiazole-4-carboxylic acid<br><br>M/z 416.3 (M+H)$^+$<br>$^1$H NMR (d6-DMSO) δ 8.49 (1H, s), 8.04 (1H, brs), 7.88 (1H, brs), 7.50 (4H, m), 3.94 (2H, brs). |
| 30 | 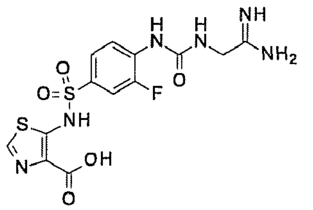 | 5-[[4-[(2-amino-2-imino-ethyl) carbamoylamino]-3-fluoro-phenyl] sulfonylamino]thiazole-4-carboxylic acid<br><br>M/z 417.3 (M+H)$^+$<br>$^1$H NMR (d6-DMSO) δ 13.5 (1H, brs), 9.01 (1H, s), 9.21-8.71 (4H, m), 8.43 (1H, s), 8.19 (1H, t, *J* = 8.4 Hz), 8.06 (1H, s), 7.46 (2H, t, *J* = 8.8 Hz), 7.29 (1H, m), 4.04 (2H, d, *J* = 5.2 Hz). |
| 31 | 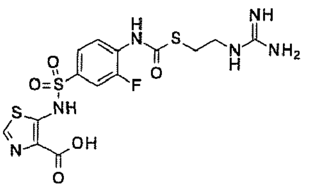 | 5-[[3-fluoro-4-(2-guanidinoethylsulfanyl-carbonylamino)phenyl] sulfonylamino]thiazole-4-carboxylic acid<br><br>M/z 463.1 (M+H)$^+$<br>$^1$H NMR (d6-DMSO) δ 13.42 (1H, s), 10.51 (1H, s), 8.16 (1H, s), 8.08 (1H, s), 7.79 (1H, t, *J* = 7.5 Hz), 7.61 (1H, s), 7.51 (2H, m), 7.31-6.79 (4H, m), 3.36 (2H, t, *J* = 6.5 Hz), 3.36 (2H, t, *J* = 6.5 Hz). |

**Example 32**

**5-[[3,5-difluoro-4-(guanidinocarbamoylamino)phenyl]sulfonylamino]thiazole-4-carboxylic acid**

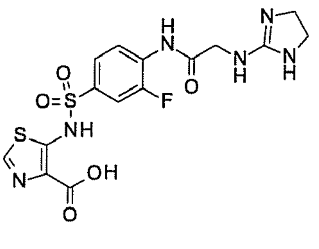

a. tert-butyl 5-[[4-[(2-chloroacetyl)amino]-3-fluoro-phenyl]sulfonyl-[(4-methoxyphenyl)methyl]amino]thiazole-4-carboxylate

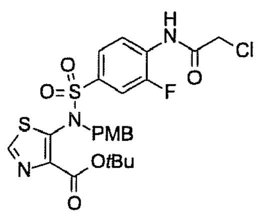

$Et_3N$ (276 mg, 2.73 mmol) and chloroacetyl chloride (185 mg, 1.64 mmol) were added to a stirred solution of tert-butyl 5-[(4-amino-3-fluoro-phenyl)sulfonyl-[(4-methoxyphenyl)methyl]amino]thiazole-4-carboxylate (450 mg, 0.91 mmol) in DCM (5 mL) at 0 °C. The resulting reaction mixture was stirred at RT for 2 h, quenched with ice cold water (5

mL) and extracted with DCM (2 x 10 mL). The combined organic layer was dried over $Na_2SO_4$, filtered and concentrated under reduced pressure. The crude material was purified by trituration with diethyl ether (2 x 5 mL) to afford a green solid which was used in the next step without further purification (400 mg, crude).
M/z 570.69 $(M+H)^+$

b. tert-butyl 5-[[4-[(2-azidoacetyl)amino]-3-fluoro-phenyl]sulfonyl-[(4-methoxyphenyl)methyl]amino]thiazole-4-carboxylate

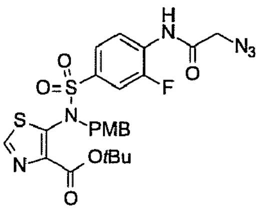

NaN3 (92 mg, 1.40 mmol) was added to a stirred solution of tert-butyl 5-[[4-[(2-chloroacetyl)amino]-3-fluorophenyl]sulfonyl-[(4-methoxyphenyl)methyl]amino]amino]thiazole-4-carboxylate (400 mg, 0.70 mmol) in DMF (5 mL) at RT. The resulting reaction mixture was stirred at RT for 16 h, quenched with ice cold water (10 mL) and extracted with ethyl acetate (2 x 10 mL). The combined organic extracts were dried over $Na_2SO_4$, filtered and concentrated under reduced pressure. The crude material was purified by trituration with diethyl ether (2 x 5 mL) to afford a light brown solid (370 mg, 91%).
M/z 577.23 $(M+H)^+$

c. tert-butyl 5-[[4-[(2-aminoacetyl)amino]-3-fluoro-phenyl]sulfonyl-[(4-methoxyphenyl)methyl]amino]thiazole-4-carboxylate

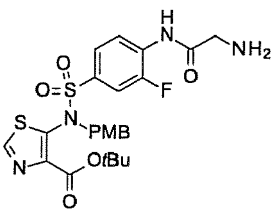

10% Pd/C (300 mg) was added to solution of tert-butyl 5-[[4-[(2-azidoacetyl)amino]-3-fluoro-phenyl]sulfonyl-[(4-methoxyphenyl)methyl]amino]thiazole-4-carboxylate (370 mg, 0.64 mmol) in EtOAc (10 mL) at RT under nitrogen atmosphere. The resulting reaction mixture was stirred at RT under a hydrogen atmosphere (balloon pressure) for 16 h, filtered through a pad of celite and washing with EtOAc (20 mL). The filtrate was concentrated under reduced pressure. The crude material was purified by trituration with diethyl ether (2 x 5 mL) to afford a brown solid (340 mg, 96%).
M/z 551.35 $(M+H)^+$

d. tert-butyl 5-[[4-[[2-(4,5-dihydro-1H-imidazol-2-ylamino)acetyl]amino]-3-fluorophenyl]sulfonyl-[(4-methoxyphenyl)methyl]amino]thiazole-4-carboxylate

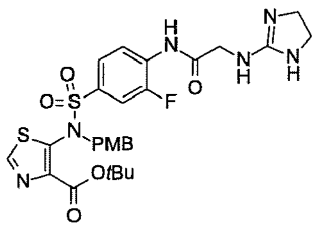

2-Methylsulfanyl-4,5-dihydro-1H-imidazole (124 mg, 0.50 mmol) was added to a stirred solution of tert-butyl 5-[[4-[(2-aminoacetyl)amino]-3-fluoro-phenyl]sulfonyl-[(4-methoxyphenyl)methyl]amino]thiazole-4-carboxylate (400 mg, 0.72 mmol) in THF (5 mL) at RT. The resulting reaction mixture was heated to 70 °C for 48 h in a closed vial and concentrated under reduced pressure to afford a yellow solid which was used in the next step without further purification (500 mg, crude).
M/z 619.36 $(M+H)^+$

e. 5-[[3,5-difluoro-4-(guanidinocarbamoylamino)phenyl]sulfonylamino]thiazole-4-carboxylic acid

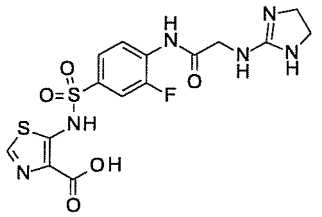

TFA (5 mL) was added to tert-butyl 5-[[4-[[2-(4,5-dihydro-1H-imidazol-2-ylamino)acetyl]amino]-3-fluoro-phenyl]sulfonyl-[(4-methoxyphenyl)methyl]amino]thiazole-4-carboxylate (500 mg, 0.50 mmol) at 0 °C and stirred at RT for 6 h. TFA was evaporated by reduced pressure and the resulting crude product was triturated with diethyl ether (2 x 5 mL) and dried under vacuum. The crude product was purified by preparative HPLC affording the title product as a white solid (37 mg, 18%).
$^1$H NMR (400 MHz, DMSO-$d_6$) δ 13.43 (1H, brs), 10.20 (1H, brs), 8.4 (3H, brs), 8.11-8.08 (2H, m), 7.55-7.48 (2H, m), 4.08 (2H, s), 3.60 (4H, s).
M/z 443.24 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 2/98, 3.8/98, 4.2/3, 4.5/3;
Column Temp: 35 °C,
Flow Rate: 0.6 mL/min.

**Prep. HPLC Condition:**

Column: Atlantis T3 (250*19) mm, 5u;
Mobile phase: (A) 0.1% Formic Acid (B) Acetonitrile;
Flow: 19 mL/min;
Gradient (T/%B): 0/5, 1/5, 9/30, 10.31/99, 12/99, 12.1/5, 15/5;
Solubility: ACN +$H_2O$+THF.

**Example 33**

**5-[[3-fluoro-4-[[2-(morpholine-4-carboximidoylamino)acetyl]amino]phenyl]sulfonylamino]thiazole-4-carboxylic acid**

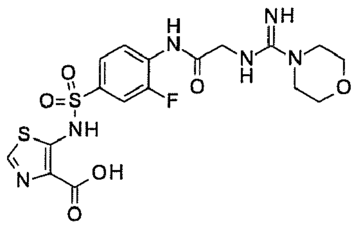

a. tert-butyl 5-[[3-fluoro-4-[[2-(morpholine-4-carbothioylamino)acetyl]amino]phenyl]sulfonyl-[(4-methoxyphenyl)methyl]amino]thiazole-4-carboxylate

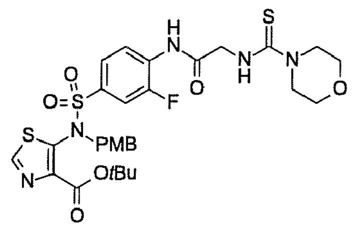

A solution of tert-butyl 5-[[4-[(2-aminoacetyl)amino]-3-fluoro-phenyl]sulfonyl-[(4-methoxyphenyl)methyl]amino]thiazole-4-carboxylate (500 mg, 0.9 mmol) and di(imidazol-1-yl)methanethione (242 mg, 1.36 mmol) in $CH_2Cl_2$ (15 mL) was stirred at RT for 30 minutes. Then morpholine (118 mg, 1.36 mmol) was added and the resulting reaction mixture was stirred at 40 °C for 1 h. The reaction mixture was concentrated under vacuum.
The crude compound was purified by flash chromatography eluting with 3% MeOH in $CH_2Cl_2$ to afford a pale pink gummy material (100 mg, 83%).
M/z 680.42 $(M+H)^+$

b. tert-butyl 5-[[3-fluoro-4-[[2-(morpholine-4-carboximidoylamino)acetyl]amino]phenyl]sulfonyl-[(4-methoxyphenyl)methyl]amino]thiazole-4-carboxylate

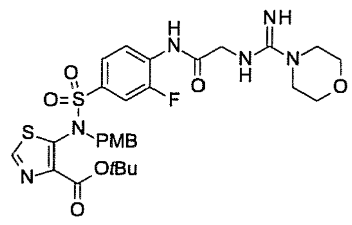

Ag(OTf) (255 mg, 0.99 mmol) was added to a solution of tert-butyl 5-[[3-fluoro-4-[[2-(morpholine-4-carbothioylamino)acetyl]amino]phenyl]sulfonyl-[(4-methoxyphenyl)methyl]amino]thiazole-4-carboxylate (450 mg, 0.66 mmol) in $CH_2Cl_2$:THF (1:1, 20 mL). The reaction mixture was cooled to - 30 °C and NH3 gas purged for 15 minutes. The reaction mixture was stirred at RT for 4 h, quenched with MeOH (1 mL) and concentrated under reduced pressure. Water (25 mL) was added and extracted with EtOAc (2x50 mL). The combined organic layer was dried over $Na_2SO_4$, filtered and concentrated. The crude material was purified by flash chromatography eluting with 3% MeOH in $CH_2Cl_2$ to afford a black gummy material (250 mg, 57%).

M/z 663.53 $(M+H)^+$

c. 5-[[3-fluoro-4-[[2-(morpholine-4-carboximidoylamino)acetyl]amino]phenyl]sulfonylamino]thiazole-4-carboxylic acid

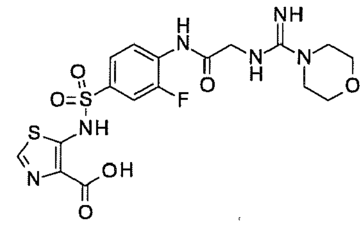

A solution of tert-butyl 5-[[3-fluoro-4-[[2-(morpholine-4-carboximidoylamino)acetyl]amino]phenyl]sulfonyl-[(4-methoxyphenyl)methyl]amino]thiazole-4-carboxylate in TFA: $H_2O$ (95:5, 2 mL) was stirred at RT for 3 h. The reaction mixture was concentrated and the crude product was neutralised with methanolic ammonia. Then the reaction mixture was concentrated under reduced pressure. The crude material was purified by preparative HPLC affording the title product as an off-white solid (12.4 mg, 8%).

$^1H$ NMR (400 MHz, DMSO-$d_6$) $\delta$ 13.32 (1H, brs), 9.67 (1H, s), 8.13 (1H, s), 8.05-7.85 (3H, m), 7.63-7.61 (2H, m), 7.52-7.49 (1H, m), 4.18 (2H, brs), 3.63-3.55 (4H, m), 3.50-3.30 (4H, obs).

M/z 487.34 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C,
Flow Rate: 0.6 mL/ min.

**Prep. HPLC Condition:**

Column used: Symmetry C18 (300*19) mm, 7 u;
Mobile phase: (A) 0.1% Formic Acid (B) Acetonitrile;
Flow: 19 mL/ min;
Gradient -(T/%B): 0/5, 1/5, 7.1/56, 7.15/99, 10/99, 10.1/5, 13/5;
Solubility: $CH_3CN$ +$H_2O$.

**Example 34**

**5-[[4-[[2-[(N-cyanocarbamimidoyl)amino]acetyl]amino]-3-fluoro-phenyl]sulfonylamino]thiazole-4-carboxylic acid**

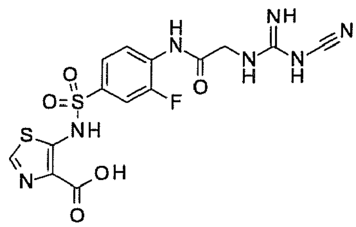

DIPEA (0.1 mL, 0.58 mmol) and $NaN(CN)_2$ (142 mg, 1.6 mmol) were added to a stirred solution of 5-[[4-[(2-aminoacetyl)amino]-3-fluoro-phenyl]sulfonylamino]thiazole-4-carboxylic acid (200 mg, 0.53 mmol) in DMF (5 mL) at RT under nitrogen atmosphere. The reaction mixture was stirred at 50 °C for 48 h and concentrated under reduced pressure. The crude compound was diluted with water (5 mL) and acidified to pH~2-3 with 1N HCl. The resulting precipitate was filtered and dried under high vacuum. The crude product was purified by preparative HPLC affording the title product as an off-white solid (20.8 mg, 8%).
$^1$H NMR (400 MHz, DMSO-$d_6$) $\delta$ 12.8 (1H, brs), 10.0 (1H, s), 8.17 (1H, s), 8.13-8.09 (1H, m), 7.55-7.52 (2H, m), 6.96 (1H, brs), 6.87 (2H, s), 4.00 (2H, d, *J* = 6.0 Hz).
M/z 442.18 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 2/98, 3.4/98, 3.5/3, 4/3;
Column Temp: 35 °C,
Flow Rate: 0.6 mL/ min.

**Prep. HPLC Condition:**

Column used: XBRIDGE C18 (150*19) mm, 5 u;
Mobile phase: (A) 0.1% Formic Acid (B) Acetonitrile;
Flow: 19 mL/min,
Gradient -(T/%B): 0/0, 2/0, 8/20, 10.9/20, 10.95/99, 13/99, 13.10/0, 16/0;
Solubility: ACN +$H_2O$+THF.

**Example 35**

**5-[[4-[(4-amino-4-imino-butanoyl)amino]-3-fluoro-phenyl]sulfonylamino]thiazole-4-carboxylic acid**

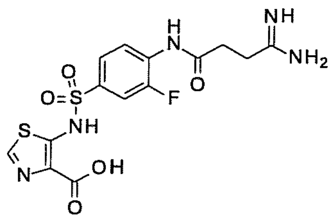

a. 5-[[4-(3-chloropropanoylamino)-3-fluoro-phenyl]sulfonyl-[(4-methoxyphenyl)methyl]amino]thiazole-4-carboxylic acid

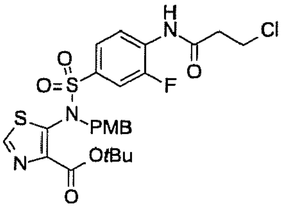

Acetic anhydride (5 mL) in DCM (5 mL) was added to a stirred solution of 3-chloropropanoyl chloride (1.5 g, 3.04 mmol) in DCM (5 mL) at 0 °C. After 10 minutes, tert-butyl 5-[(4-amino-3-fluoro-phenyl)sulfonyl-[(4-methoxyphenyl)methyl]amino]thiazole-4-carboxylate (1.5 g) in DCM (10 mL) was added at 0 °C. The resulting reaction mixture was stirred at RT for 2 h, quenched with ice cooled water (20 mL) and extracted with DCM (2x10 mL). The organic layer was dried over $Na_2SO_4$, filtered and concentrated. The crude material was purified by flash chromatography (eluting with 60% ethyl acetate in petroleum ether) to afford an off-white solid (600 mg, 33%).
M/z 584.40 $(M+H)^+$

b. tert-butyl 5-[[4-(3-cyanopropanoylamino)-3-fluoro-phenyl]sulfonyl-[(4-methoxyphenyl)methyl]amino]thiazole-4-carboxylate

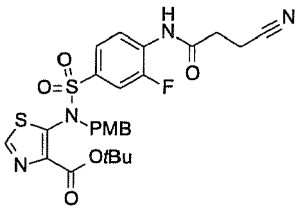

Sodium cyanide (76 mg, 1.54 mmol) was added to a stirred solution of 5-[[4-(3-chloropropanoylamino)-3-fluoro-phenyl]sulfonyl-[(4-methoxyphenyl)methyl]amino]thiazole-4-carboxylic acid (600 mg, 1.02 mmol) in DMF (5 mL) at RT. The resulting reaction mixture was stirred at RT for 6 h and quenched with ice cold water (10 mL). The resulting precipitate was filtered, washed with $Et_2O$ (2x10 mL) and dried under high vacuum to afford a brown solid (500 mg, 84%).
M/z 597.24 $(M+Na)^+$

c. tert-butyl 5-[[4-[(4-amino-4-imino-butanoyl)amino]-3-fluoro-phenyl]sulfonyl-[(4-methoxyphenyl)methyl]amino]thiazole-4-carboxylate

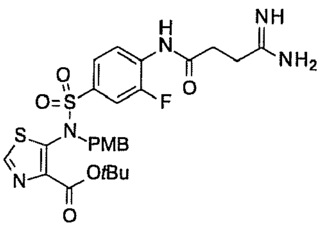

HCl gas was passed to a stirred solution of tert-butyl 5-[[4-(3-cyanopropanoylamino)-3-fluoro-phenyl]sulfonyl-[(4-methoxyphenyl)methyl]amino]thiazole-4-carboxylate (400 mg, 0.69 mmol) in ethanol: $Et_2O$ (1:4, 10 mL) at 0 °C for 2 h. The resulting reaction mixture was kept at 4 °C for 16 h. Then the volatile components were evaporated under reduced pressure. The residue was dissolved in ethanol (5 mL) and NH3 gas was passed for 20 minutes. The volatile components were evaporated under reduced pressure. The resulting crude product was triturated with diethyl ether (2 x 5mL) and dried under high vacuum to afford a brown solid which was used in the next step without further purification (350 mg, crude).
M/z 592.43 $(M+H)^+$

d. 5-[[4-[(4-amino-4-imino-butanoyl)amino]-3-fluoro-phenyl]sulfonylamino]thiazole-4-carboxylic acid

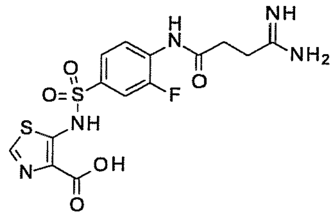

TFA:$H_2O$ (95:5, 3 mL) was added to tert-butyl 5-[[4-[(4-amino-4-imino-butanoyl)amino]-3-fluoro-phenyl]sulfonyl-[(4-methoxyphenyl)methyl]amino]thiazole-4-carboxylate (350 mg, 0.592 mmol) at 0 °C. The resulting reaction mixture was stirred at RT for 6 h and concentrated under reduced pressure. The resulting crude product was triturated with diethyl ether (2 x 5 mL) and dried under high vacuum. The crude product was purified by preparative HPLC affording the title product as an off-white solid (31 mg, 12%).
$^1$H NMR (400 MHz, DMSO-$d_6$) δ 13.40 (1H, s), 10.08 (1H, s), 8.99-8.71 (4H, m), 8.07 (1H, s), 8.05-8.01 (1H, m), 7.54-7.46 (2H, m), 2.85 (2H, t, *J*= 7.2 Hz), 2.62 (2H, t, *J*= 7.2 Hz).
M/z 415.93 $(M+H)^+$

<u>**LC-MS Condition:**</u>

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 2/98, 3.4/98, 3.5/3, 4/3;
Column Temp: 35 °C;
Flow Rate: 0.6 mL/min.

<u>**Prep. HPLC Condition:**</u>

Column: Symmetry C18 (300*19) mm, 7 u;
Mobile phase: (A) 0.1% Formic Acid (B) Acetonitrile;
Flow: 19 mL/min;
Gradient (T/%B): 0/5,1/5, 7/20, 10.1/20, 10.1/99, 13/99, 13.1/5, 16/5;
Solubility: ACN +$H_2O$+THF+DMSO+conc FA.

**Example 36**

**5-[[4-[3-(4,5-dihydro-1H-imidazol-2-yl)propanoylamino1-3-fluoro-phenyl] sulfonylamino]thiazole-4-carboxylic acid**

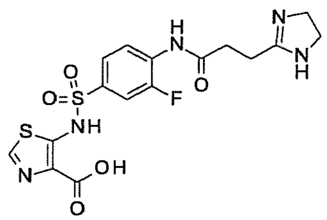

a. 5-[[4-[3-(4,5-dihydro-1H-imidazol-2-yl)propanoylamino]-3-fluorophenyl]sulfonyl-[(4-methoxyphenyl)methyl]amino]thiazole-4-carboxylic acid

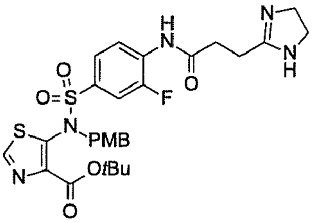

HCl gas was passed to a stirred solution of tert-butyl 5-[[4-(3-cyanopropanoylamino)-3-fluoro-phenyl]sulfonyl-[(4-methoxyphenyl)methyl]amino]thiazole-4-carboxylate (310 mg, 0.53 mmol) in ethanol: $Et_2O$ (1:4, 15 mL) at 0 °C for 2 h. The resulting reaction mixture was kept in refrigerator for 16 h. Then the volatile components were evaporated under reduced pressure. The resulting residue was dissolved in ethanol (5 mL). Then ethylene diamine (32 mg, 0.53 mmol) was added at RT. The resulting reaction mixture was stirred at RT for 8 h and concentrated under reduced pressure. The resulting crude product was triturated with n-pentane (2 x 5 mL) and dried under high vacuum to afford a brown solid which was used in the next step without further purification (400 mg, crude).
M/z 618.46 $(M+H)^+$

b. 5-[[4-[3-(4,5-dihydro-1H-imidazol-2-yl)propanoylamino]-3-fluoro-phenyl]sulfonylamino]thiazole-4-carboxylic acid

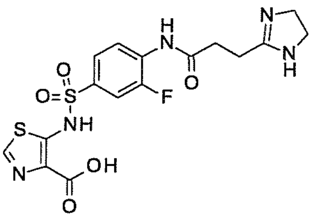

TFA:$H_2O$ (95:5, 3 mL) was added to 5-[[4-[3-(4,5-dihydro-1H-imidazol-2-yl)propanoylaminino]-3-fluoro-phenyl]sulfonyl-[(4-methoxyphenyl)methyl]amino]thiazole-4-carboxylic acid (280 mg, 0.45 mmol) at 0 °C. The resulting reaction mixture was stirred at RT for 4 h and concentrated under reduced pressure. The resulting crude product was triturated with diethyl ether (2 x 5 mL) and dried under high vacuum. The crude product was purified by preparative HPLC to afford the title product as an off-white solid (21 mg, 10%).
$^1H$ NMR (300 MHz, DMSO-$d_6$) $\delta$ 13.45 (1H, brs), 9.80 (1H, brs), 8.12-8.04 (2H, m), 7.54-7.44 (2H, m), 3.65 (4H, s), 2.82-2.77 (2H, m), 2.62-2.58 (2H, m).
M/z 441.98 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: Symmetry C18 (300*19) mm, 7 u;
Mobile phase: (A) 0.1% Formic Acid (B) Acetonitrile;
Flow: 19 mL/min;
Gradient (T/%B): 0/5, 1/5, 7/30, 8.7/30, 8.75/99, 11/99, 11.1/5, 13/5;
Solubility: ACN +$H_2O$+THF.

**Example 37**

**5-[[4-[(3-amino-3-imino-2-methyl-propanoyl)amino]-3-fluoro-phenyl]sulfonylamino]thiazole-4-carboxylic acid**

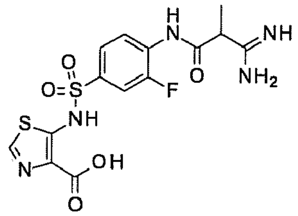

a. tert-butyl 5-[[4-(2-chloropropanoylamino)-3-fluoro-phenyl]sulfonyl-[(4-methoxyphenyl)methyl]amino]thiazole-4-carboxylate

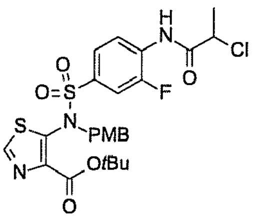

2-chloropropanoyl chloride (1.48 mL, 15.1 mmol) was added to a stirred solution of tert-butyl 5-[(4-amino-3-fluoro-phenyl)sulfonyl-[(4-methoxyphenyl)methyl]amino]thiazole-4-carboxylate (3 g, 6.0 mmol) in DCM (50 mL) at 0 °C. The resulting reaction mixture was stirred at RT for 2 h and concentrated under reduced pressure. The residue was triturated with diethyl ether (2 x 50 mL), pentane (2 x 50 mL) and dried under reduced pressure to afford an off-white solid (3.4 g, 95%).
M/z 606.28 $(M+Na)^+$; 582.75 $(M-H)^-$

b. tert-butyl 5-[[4-(2-cyanopropanoylamino)-3-fluoro-phenyl]sulfonyl-[(4-methoxyphenyl)methyl]amino]thiazole-4-carboxylate

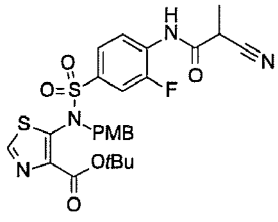

NaCN (570 mg, 11.6 mmol) was added to a solution of tert-butyl 5-[[4-(2-chloropropanoylamino)-3-fluoro-phenyl]sulfonyl-[(4-methoxyphenyl)methyl]amino]thiazole-4-carboxylate (3.4 g, 5.8 mmol) in DMF (35 mL) at RT. The resulting reaction mixture was stirred at RT for 16 h and quenched with ice cooled water. The resulting precipitate was filtered and dried under high vacuum. The crude product was purified by silica gel chromatography (eluting with 40% EtOAc in petroleum ether) to afford an off-white solid (1.5 g, 44%).

M/z 597.29 $(M+Na)^+$; 573.62 $(M-H)^-$

c. tert-butyl 5-[[3-fluoro-4-[[3-(hydroxyamino)-3-imino-2-methylpropanoyl]amino]phenyl]sulfonyl-[(4-methoxyphenyl)methyl]amino]thiazole-4-carboxylate

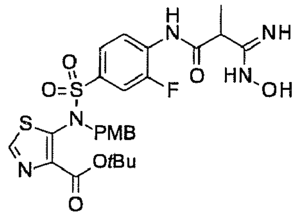

$NH_2OH$.HCl (362 mg, 5.22 mmol) and $Na_2CO_3$ (828 mg, 7.8 mmol) were added to a stirred solution of tert-butyl 5-[[4-(2-cyanopropanoylamino)-3-fluoro-phenyl]sulfonyl-[(4-methoxyphenyl)methyl]amino]thiazole-4-carboxylate (1.5 g, 2.61 mmol) in EtOH (30 mL) at RT. The resulting reaction mixture was stirred at 65 °C for 1 h, cooled to RT and filtered. The filtrate was concentrated under reduced pressure to afford a pale yellow gummy material which was used in the next step without further purification (1.5 g, crude).
M/z 608.48 $(M+H)^+$

d. tert-butyl 5-[[4-[(3-amino-3-imino-2-methyl-propanoyl)amino]-3-fluorophenyl]sulfonyl-[(4-methoxyphenyl)methyl]amino]thiazole-4-carboxylate

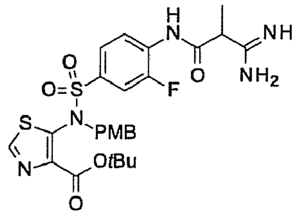

Iron powder (193 mg, 3.4 mmol) was added to tert-butyl 5-[[3-fluoro-4-[[3-(hydroxyamino)-3-imino-2-methyl-propanoyl]amino]phenyl]sulfonyl-[(4-methoxyphenyl)methyl]amino]thiazole-4-carboxy]ate (350 mg, 0.57 mmol) in ethanol:water (1:1, 3 mL) and heated to reflux for 30 minutes. Then 1N HCl (0.3 mL) in ethanol:water (1:1, 3 mL) was added to the reaction mixture over a period of 30 minutes. The reaction mixture was stirred for an additional 1 h at 70 °C, cooled to RT and filtered through celite. The celite pad was washed with ethanol (2 x 10 mL). The filtrate was concentrated under reduced pressure to afford a pale yellow liquid which was used in the next step without further purification (340 mg, crude).
M/z 592.28 $(M+H)^+$

e. 5-[[4-[(3-amino-3-imino-2-methyl-propanoyl)amino]-3-fluorophenyl]sulfonylamino]thiazole-4-carboxylic acid

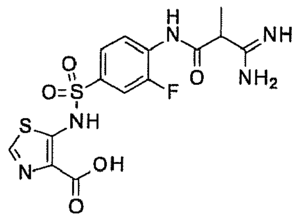

Tert-butyl 5-[[4-[(3-amino-3-imino-2-methyl-propanoyl)amino]-3-fluoro-phenyl]sulfonyl-[(4-methoxyphenyl)methyl]amino]thiazole-4-carboxylate (340 mg, 0.57 mmol) was added to a solution of TFA:$H_2O$ (9:1, 3 mL) at RT. The

reaction mixture was stirred at RT for 3 h and concentrated under reduced pressure (below 30 °C of bath temperature). The residue was triturated with diethyl ether (2 x 5 mL) and dried under high vacuum. The crude product was purified by preparative HPLC to afford the title product as an off-white solid (48.2 mg).

$^1H$ NMR (300 MHz, DMSO-$d_6$) δ 13.40 (1H, brs), 10.25 (1H, s), 8.89 (2H, s), 8.65 (2H, s), 8.11 (1H, s), 8.0 (1H, t, *J* = 8.1 Hz), 7.60-7.50 (2H, m), 3.87 (1H, q, *J*= 7.2 Hz), 1.49 (3H, d, *J*= 7.2 Hz).

M/z 416.34 $(M+H)^+$

**LC-MS Condition:**

Column: Acquity BEH C18 (50 mm x 2.1 mm, 1.7um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column used: Symmetry C18 (300*19) mm, 7 u;
Mobile phase: (A) 0.1% Formic Acid (B) Acetonitrile;
Flow: 19 mL/min;
Gradient-(T/%B): 0/5, 1/5, 8/50, 8.1/99, 11/99, 11.1/5, 14/5;
Solubility: ACN +$H_2O$+ Concentrated FA.

**Example 38**

**5-[[3-fluoro-4-[[2-(2-iminoimidazolidin-1-yl)acetyl]amino]phenyl]sulfonylamino] thiazole-4-carboxylic acid**

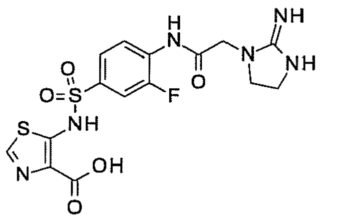

a. tert-butyl 5-[[3-fluoro-4-[[2-(2-thioxoimidazolidin-1-yl)acetyl]amino]phenyl]sulfonyl-[(4-methoxyphenyl)methyl]amino]thiazole-4-carboxylate

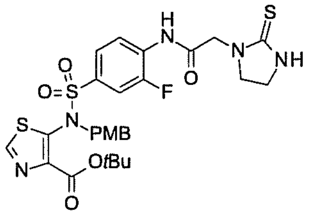

A solution of tert-butyl 5-[[4-[(2-chloroacetyl)amino]-3-fluoro-phenyl]sulfonyl-[(4-methoxyphenyl)methyl]amino]thiazole-4-carboxylate (500 mg, 0.87 mmol) in acetonitrile (50 mL) was added to a stirred solution of ethylenediamine (0.29 mL, 4.38 mmol) in acetonitrile (50 mL) over a period of 30 minutes at 75 °C. The resulting reaction mixture was stirred at 75°C for 2.5 h. Di(imidazol-1-yl)methanethione (1.56 g, 8.77 mmol) was then added at 75 °C and the reaction

was stirred for 1 h at the same temperature then concentrated under reduced pressure. The resulting crude compound was diluted with EtOAc (50 mL), washed with water (10 mL) and brine solution (10 mL). The organic layer was dried over $Na_2SO_4$, filtered and concentrated under reduced pressure. The crude material was purified by flash chromatography (eluting with 60% EtOAc in petroleum ether) to afford a light brown solid (200 mg, 36%).
M/z 636.20 $(M+H)^+$

b. tert-butyl 5-[[3-fluoro-4-[[2-(2-iminoimidazolidin-1-yl)acetyl]amino]phenyl]sulfonyl-[(4-methoxyphenyl)methyl]amino]thiazole-4-carboxylate

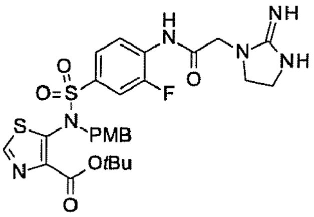

Ag(OTf) (121 mg, 0.47 mmol) and saturated $NH_3$ in THF (5 mL) were added to a stirred solution of tert-butyl 5-[[3-fluoro-4-[[2-(2-thioxoimidazolidin-1-yl)acetyl]amino]phenyl]sulfonyl-[(4-methoxyphenyl)methyl]amino]thiazole-4-carboxylate (200 mg, 0.31 mmol) in $CH_2Cl_2$ (10 mL) at -30 °C under nitrogen atmosphere. The resulting reaction mixture was stirred at RT for 16 h, quenched with MeOH (2 mL) and stirred at RT for 10 minutes. The reaction mixture was filtered through celite pad and the pad was washed with $CH_2Cl_2$ (2 x 10 mL). The filtrate was concentrated under reduced pressure to afford a dark brown liquid which was used in the next step without further purification (300 mg, crude).
M/z 619.48 $(M+H)^+$

c. 5-[[3-fluoro-4-[[2-(2-iminoimidazolidin-1-yl)acetyl]amino]phenyl]sulfonylamino]thiazole-4-carboxylic acid

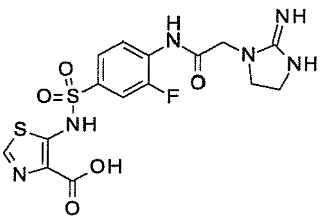

TFA: $H_2O$ (9:1, 5 mL) was added to tert-butyl 5-[[3-fluoro-4-[[2-(2-iminoimidazolidin-1-yl)acetyl]amino]phenyl]sulfonyl-[(4-methoxyphenyl)methyl]amino]thiazole-4-carboxylate (270 mg, 0.43 mmol) at 0 °C. The resulting reaction mixture was stirred at RT for 3 h and concentrated under reduced pressure. The resulting crude product was triturated with diethyl ether (2 x 5 mL) and dried under high vacuum. The crude product was purified by preparative HPLC affording the title product as an off-white solid (10.6 mg).

$^1H$ NMR (400 MHz, DMSO-$d_6$) δ 13.32 (1H, brs), 10.24 (1H, s), 8.70 (1H, brs), 8.12 (1H, s), 7.68-7.60 (3H, m), 7.47 (1H, dd, *J*= 8.0 Hz, *J*= 7.6 Hz), 7.38 (1H, s), 4.11 (2H, s), 3.74-3.67 (2H, m), 3.61-3.55 (2H, m).
M/z 443.24 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C,
Flow Rate: 0.6 mL/min

**Prep. HPLC Condition:**

Column used: Atlantis T3 (250*19) mm, 5 u;
Mobile phase: (A) 0.1% Formic Acid (B) Acetonitrile;
Flow: 19 mL/min;
Gradient -(T/%B): 0/5, 1/5, 7/30, 8.25/30, 8.3/99, 11/99, 11.1/5, 14/5;
Solubility: ACN +$H_2O$+THF

**Example 39**

**5-[[4-[[2-[[N-(2-aminoethyl)carbamimidoyl]amino]acetyl]amino]-3-fluoro-phenyl]sulfonylaminol]thiazole-4-carboxylic acid**

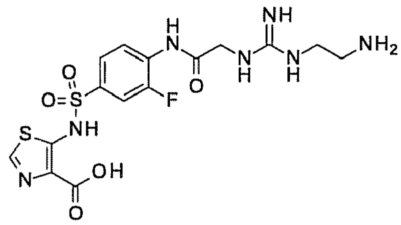

a. tert-butyl 5-[[4-[[2-[2-(tert-butoxycarbonylamino)ethylcarbamothioylamino]acetyl]amino]-3-fluorophenyl]sulfonyl-[(4-methoxyphenyl)methyl]amino]thiazole-4-carboxylate

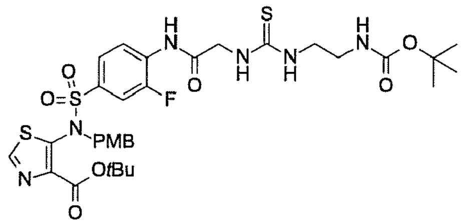

Di(imidazol-1-yl)methanethione (97 mg, 0.54 mmol) was added to a stirred solution of tert-butyl 5-[[4-[(2-aminoacetyl)amino]-3-fluoro-phenyl]sulfonyl-[(4-methoxyphenyl)methyl]amino]thiazole-4-carboxylate (200 mg, 0.36 mmol) in DCM (10 mL) at RT. The reaction mixture was stirred at RT for 4 h and $NH_2CH_2CH_2NHBoc$ (174 mg, 1.08 mmol) was added. The reaction mixture was stirred at 40 °C for 6 h and concentrated under reduced pressure. The crude product was purified by column chromatography (eluting with 60% EtOAc in petroleum ether) to afford a brown solid (80 mg, 29%). M/z 753.43 $(M+H)^+$

b. tert-butyl 5-[[4-[[2-[[N-[2-(tert-butoxycarbonylamino)ethyl]carbamimidoyl]amino]acetyl]amino]-3-fluorophenyl]sulfonyl-[(4-methoxyphenyl)methyl]amino]thiazole-4-carboxylate

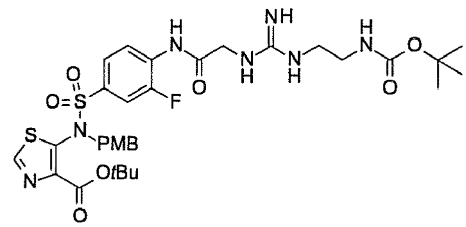

Ag(OTf) (107 mg, 0.47 mmol) was added to a stirred solution of tert-butyl 5-[[4-[[2-[2-(tert-butoxycarbonylamino)ethylcarbamothioylamino]acetyl]amino]-3-fluorophenyl]sulfonyl-[(4-methoxyphenyl)methyl]amino]thiazole-4-carboxylate (220 mg, 0.27 mmol) in $CH_2Cl_2$ (10 mL) at RT. The reaction mixture was stirred at RT for 15 minutes and saturated NH3 in THF (5 mL) was added at -30 °C under nitrogen atmosphere. The resulting reaction mixture was stirred at RT for 6 h, filtered through celite pad and the pad was washed with $CH_2Cl_2$ (10 mL). The filtrate was concentrated and the obtained crude material was triturated with n-pentane (10 mL) to afford a brown solid which was used in the next step without further purification.
M/z 736.40 $(M+H)^+$

c. 5-[[4-[[2-[[N-(2-aminoethyl)carbamimidoyl]amino]acetyl]amino]-3-fluorophenyl]sulfonylamino]thiazole-4-carboxylic acid

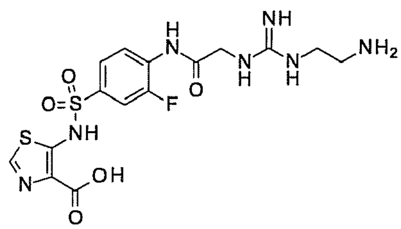

TFA: $H_2O$ (9:1, 2 mL) was added to tert-butyl 5-[[4-[[2-[[N-[2-(tert-butoxycarbonylamino)ethyl]carbamimidoyl]amino]acetyl]amino]-3-fluorophenyl]sulfonyl-[(4-methoxyphenyl)methyl]amino]thiazole-4-carboxylate (210 mg, 0.28 mmol) at 0 °C. The reaction mixture was stirred at RT for 4 h and concentrated under reduced pressure. The resulting crude product was triturated with diethyl ether (2 x 5 mL) and dried under high vacuum. The crude product was purified by preparative HPLC affording the title product as brown solid (25 mg).
$^1$H NMR (300 MHz, DMSO-$d_6$) $\delta$ 8.13 (1H, brs), 8.09 (1H, s), 7.42-7.30 (2H, m), 6.96 (1H, dd, *J*= 8.7 Hz, *J*= 8.4 Hz), 6.20-6.00 (1H, m), 5.70-5.40 (1H, m), 3.82 (2H, s), 3.27 (2H, brs), 2.80-2.75 (2H, m).
M/z 460.30 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: Atlantis T3 (250* 19) mm, 5 u;
Mobile phase: (A) 0.1% Formic Acid (B) Acetonitrile;
Flow: 19 mL/min;

Gradient (T/%B): 0/5, 1/5, 7/25, 12/30, 12.1/99, 15/99, 15.1/5, 18/5;
Solubility: ACN +$H_2O$+THF+FA.

Compounds prepared using methods analogous to those described above for Example 39 by using methylamine in step-a and purified in a similar manner by preparative HPLC are shown in the Table below:-

| Example | Structure | Name, NMR and Mass |
|---|---|---|
| 40 | 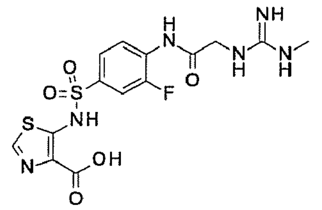 | 5-[[3-fluoro-4-[[2-[(N-methylcarbamimidoyl)amino]acetyl]amino] phenyl]sulfonylamino]thiazole-4-carboxylic acid<br><br>M/z 431.2 $(M+H)^+$<br>$^1$H NMR (d6-DMSO) δ 13.39 (1H, s), 9.65 (1H, s), 8.11 (1H, m), 8.05 (1H, m), 7.94 (3H, m), 7.61 (2H, m), 7.49 (1H, t, *J* = 8.4 Hz), 3.88 (2H, d, *J* = 5.2 Hz), 2.65 (3H, d, *J* = 4.4 Hz). |

**Example 41**

**5-[[4-[[2-(2-carbamimidoylhydrazino)acetyl]amino]-3-fluoro-phenyl]sulfonylamino]thiazole-4-carboxylic acid**

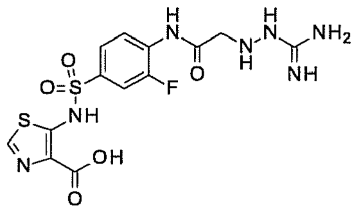

a. tert-butyl 5-[[4-[[2-(2-tert-butoxycarbonylhydrazino)acetyl]amino]-3-fluorophenyl]sulfonyl-[(4-methoxyphenyl)methyl]amino]thiazole-4-carboxylate

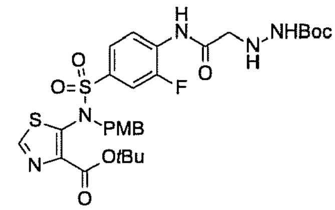

KI (1.17 g, 7.01 mmol) was added to a solution of tert-butyl 5-[[4-[(2-chloroacetyl)amino]-3-fluoro-phenyl]sulfonyl-[(4-methoxyphenyl)methyl]amino]thiazole-4-carboxylate (2 g, 3.50 mmol) in DMF (20 mL) at RT. After 10 minutes, tert-butyl N-aminocarbamate (695 mg, 5.26 mmol) was added to the reaction mixture at the same temperature. The resulting reaction mixture was stirred at RT for 16 h and concentrated under reduced pressure. Water (25 mL) was added to the crude compound and stirred for 20 minutes. The resulting precipitate was filtered, washed with diethyl ether and dried under high vacuum to afford a pale yellow solid which was used in the next step without further purification (1.2 g, 52%).
M/z 666.48 $(M+H)^+$

b. 5-[[3-fluoro-4-[(2-hydrazinoacetyl)amino]phenyl]sulfonylamino]thiazole-4-carboxylic acid

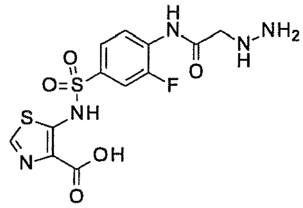

TFA (4 mL) was added to tert-butyl 5-[[4-[[2-(2-tert-butoxycarbonylhydrazino)acetyl]amino]-3-fluoro-phenyl]sulfonyl-[(4-methoxyphenyl)methyl]amino]thiazole-4-carboxylate (1.2 g, 1.80 mmol) at RT. The reaction mixture was stirred at RT for 16 h and concentrated under reducing pressure. The resulting crude product was triturated with diethyl ether (3x10 mL) to afford a pale yellow solid which was used in the next step without further purification (1g, crude).
M/z 390.32 $(M+H)^+$

c. 5-[[4-[[2-(2-carbamimidoylhydrazino)acetyl]amino]-3-fluorophenyl]sulfonylamino]thiazole-4-carboxylic acid

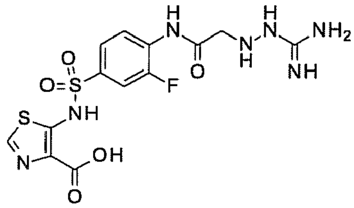

DIPEA (1.1 mL, 6.42 mmol) and pyrazole-1-carboxamidine;hydrochloride (212 mg, 1.92 mmol) were added to a stirred solution of 5-[[3-fluoro-4-[(2-hydrazinoacetyl)amino]phenyl]sulfonylamino]thiazole-4-carboxylic acid (500 mg, 1.28 mmol) in DMF (5 mL) at RT. The resulting reaction mixture was stirred at RT for 16 h, concentrated under reduced pressure and water (5 mL) was added to the residue. The resulting precipitate was filtered and washed with $Et_2O$ (2x10 mL) and dried under high vacuum. The crude product was purified by preparative HPLC affording the title product as an off-white solid (15 mg).
$^1H$ NMR (400 MHz, DMSO-$d_6$) δ 13.40 (1H, brs), 10.0 (1H, brs), 9.00 (1H, brs), 8.48 (1H, s), 8.05 (1H, m), 7.55-7.49 (2H, m), 7.45-7.22 (3H, brs), 5.67 (1H, brs), 3.62 (2H, d, *J* = 4.4 Hz).
M/z 432.37 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: X BRIDGE C18 (150*19) mm, 5 u;
Mobile phase (A) 0.1% Formic Acid (B) Acetonitrile;
Flow: 19 mL/min;
Gradient (T/%B): (T/%B): 0/0, 3/0, 8.8/33, 9/33, 9.10/99, 12/99, 12.10/0, 15/0;
Solubility: ACN+$H_2O$+THF+DMSO+FA.

**Example 42**

**5-[[3-fluoro-4-[(2-guanidinooxyacetyl)amino]phenyl]sulfonylamino] thiazole-4-carboxylic acid**

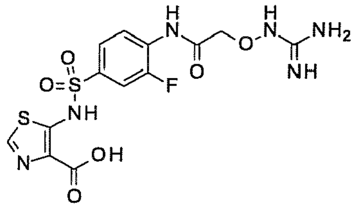

a. tert-butyl 5-[[4-[[2-(tert-butoxycarbonylamino)oxyacetyl]amino]-3-fluorophenyl]sulfonyl-[(4-methoxyphenyl)methyl]amino]thiazole-4-carboxylate

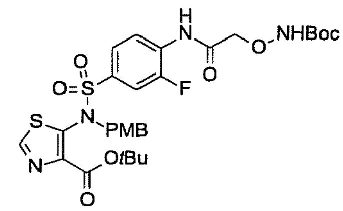

A solution of tert-butyl N-hydroxycarbamate (175 mg, 1.31 mmol) in THF (10 mL) was added to NaH (195 mg, 4.38 mmol) suspension in THF (10 mL) at 0 °C under argon atmosphere and stirred at RT for 30 minutes. Then tert-butyl 5-[[4-[(2-chloroacetyl)amino]-3-fluoro-phenyl]sulfonyl-[(4-methoxyphenyl)methyl]amino]thiazole-4-carboxylate (500 mg, 0.87 mmol) in THF (10 mL) was added to above reaction mixture at 0 °C under argon atmosphere. The resulting reaction mixture was stirred at RT for 1.5 h, quenched with ice cold water (20 mL) and extracted with ethyl acetate (2x20 mL). The combined organic layer was dried over $Na_2SO_4$, filtered and concentrated under reduced pressure. The crude material was purified by column chromatography (eluting with 30% ethyl acetate in petroleum ether) to afford a yellow solid (350 mg, 59%).
M/z 667.10 $(M+H)^+$

b. 5-[[4-[(2-aminooxyacetyl)amino]-3-fluoro-phenyl]sulfonylamino]thiazole-4-carboxylic acid

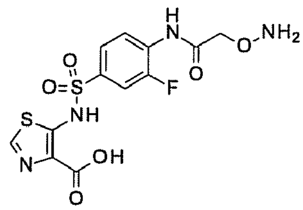

TFA: $H_2O$ (9:1, 3 mL) was added to tert-butyl 5-[[4-[[2-(tert-butoxycarbonylamino)oxyacetyl]amino]-3-fluorophenyl]sulfonyl-[(4-methoxyphenyl)methyl]amino]thiazole-4-carboxylate (300 mg, 0.44 mmol) at 0 °C. The reaction mixture was stirred at RT for 4 h and concentrated under reduced pressure. The resulting crude product was triturated with diethyl ether (3x10 mL) to afford an off-white solid which was used in next step without further purification (200 mg, crude).
M/z 390.95 $(M+H)^+$

c. 5-[[3-fluoro-4-[(2-guanidinooxyacetyl)amino]phenyl]sulfonylamino]thiazole-4-carboxylic acid

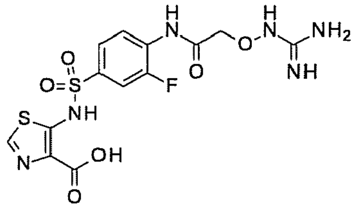

DIPEA (0.26 mL, 1.53 mmol) and pyrazole-1-carboxamidine;hydrochloride (149 mg, 0.92 mmol) were added to a stirred solution of 5-[[4-[(2-aminooxyacetyl)amino]-3-fluorophenyl]sulfonylamino]thiazole-4-carboxylic acid (200 mg, 0.51 mmol) in DMF (6 mL) at 0 °C. The resulting reaction mixture was stirred at RT for 6 h, concentrated under reduced pressure and water (5 mL) was added to the residue. The resulting precipitate was filtered, washed with $Et_2O$ (2x10 mL) and dried under high vacuum. The crude product was purified by preparative HPLC affording the title product as an off-white solid (70 mg, 31%).

$^1$H NMR (300 MHz, DMSO-$d_6$) δ 13.50 (1H, brs), δ 9.60 (1H, s), 8.37 (2H, brs), 8.17-8.12 (1H, m), 8.07 (1H, s), 7.58-7.51 (2H, m), 5.45 (2H, brs), 4.62 (2H, brs), 4.22 (2H, s).

M/z 433.30 $(M+H)^+$

**LC-MS Condition:**

Column: Acquity BEH C18 (50 mm x 2.1 mm, 1.7 um);
Mobile Phase: A: 0.05% Formic Acid in Water B: 0.05% Formic Acid in Acetonitrile;
Time (min) /%B: 0/3, 0.4/3,3.2/98, 3.8/98, 4.2/3, 4.5/3;
Column Temp: 35 °C;
Flow Rate: 0.6 mL/min.

**Prep. HPLC Condition:**

Column: X BRIDGE C18 (150*19) mm, 5u;
Mobile phase (A) 0.1% Formic Acid (B) Acetonitrile;
Flow: 19 mL/min;
Gradient (T/%B): -(T/%B):0/0, 3/0, 8.8/33, 9/33, 9.10/99, 12/99, 12.10/0, 15/0;
Solubility: ACN+$H_2O$+THF+DMSO+FA.

**Example 43**

**5-[[4-[[(2E)-2-(carbamimidoylhydrazono)acetyl]amino]-3-fluoro-phenyl]sulfonylamino]thiazole-4-carboxylic acid**

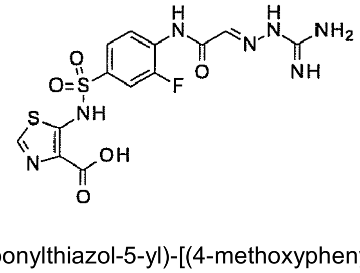

a. tert-butyl 5-[[4-[[4-[4-[(4-tert-butoxycarbonylthiazol-5-yl)-[(4-methoxyphenyl)methyl]sulfamoyl]-2-fluoro-anilino]-4-oxo-but-2-enoyl]amino]-3-fluoro-phenyl]sulfonyl-[(4-methoxyphenyl)methyl]amino]thiazole-4-carboxylate

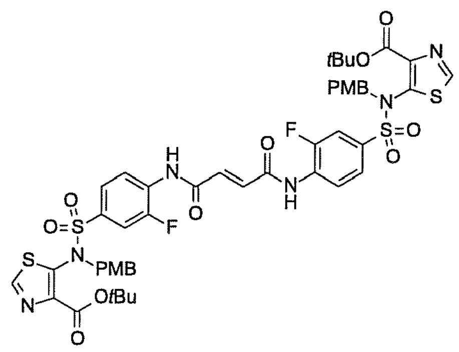

But-2-enedioyl dichloride (0.18 g, 1.2 mmol) in DCM (20 mL) was added to a solution of tert-butyl 5-[(4-amino-3-fluoro-phenyl)sulfonyl-[(4-methoxyphenyl)methyl]amino]thiazole-4-carboxylate (1 g, 2.0 mmol) in DCM (10 mL) at 0 °C. The resulting reaction mixture was stirred at RT for 6 h, diluted with DCM and washed with water (2 x 10 mL) and brine (2 x 10 mL) solution. The combined organic layer was dried over $Na_2SO_4$, filtered and concentrated under reduced pressure. The crude material was purified by flash chromatography (eluting with 50% ethyl acetate in petroleum ether) to afford a pale yellow solid (500 mg, 23%).
M/z 1067.05 $(M+H)^+$

b. tert-butyl 5-[[3-fluoro-4-(oxaldehydoylamino)phenyl]sulfonyl-[(4-methoxyphenyl)methyl]amino]thiazole-4-carboxylate

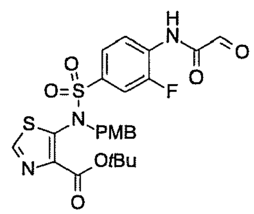

A solution of tert-butyl 5-[[4-[[4-[4-[(4-tert-butoxycarbonylthiazol-5-yl)-[(4-methoxyphenyl)methyl]sulfamoyl]-2-fluoro-anilino]-4-oxo-but-2-enoyl]amino]-3-fluorophenyl]sulfonyl-[(4-methoxyphenyl)methyl]amino]thiazole-4-carboxylate (500 mg, 0.46 mmol) in DCM/MeOH (3:1, 40 mL) was purged with ozone gas for 1 h at -78 °C. Then DMS (2 mL) was added to the reaction mixture at the same temperature and the resulting mixture was stirred at RT for 2 h. The crude reaction mixture was used in next step without further purification.

c. tert-butyl 5-[[4-[[(2E)-2-(carbamimidoylhydrazono)acetyl]amino]-3-fluorophenyl]sulfonyl-[(4-methoxyphenyl)methyl]amino]thiazole-4-carboxylate

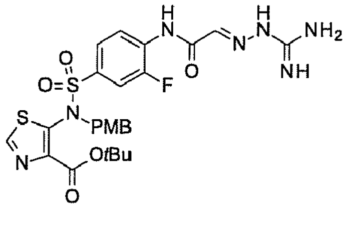

1-aminoguanidine;hydrochloride (30 mg, 0.27 mmol) was added to a stirred solution of tert-butyl 5-[[3-fluoro-4-(oxaldehydoylamino)phenyl]sulfonyl-[(4-methoxyphenyl)methyl]amino]thiazole-4-carboxylate (100 mg, 0.18 mmol) in DCM/MeOH (1:1, 10 mL) at RT. The resulting reaction mixture was stirred at RT for 16 h and concentrated under reduced pressure. The crude product was used in next step without further purification (100 mg, crude).
M/z 522.1 $(M+H)^+$

d. 5-[[4-[[(2E)-2-(carbamimidoylhydrazono)acetyl]amino]-3-fluorophenyl]sulfonylamino]thiazole-4-carboxylic acid

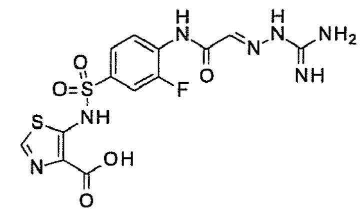

TFA/$H_2O$ (9:1, 1.0 mL) was added to tert-butyl 5-[[4-[[(2E)-2-(carbamimidoylhydrazono)acetyl]amino]-3-fluorophenyl]sulfonyl-[(4-methoxyphenyl)methyl]amino]thiazole-4-carboxylate (80 mg, 0.13 mmol) at 0 °C, stirred at RT for 4 h and concentrated under reduced pressure. The resulting crude product was triturated with diethyl ether (2 x 5 mL) and dried under high vacuum. The crude product was purified by preparative HPLC affording the title product as an off-white solid (11 mg). $^1$H NMR (400 MHz, DMSO-$d_6$) δ 9.68 (1H, brs), 8.05 (1H, s), 7.80 (1H, dd, *J*= 8.4 Hz, *J*= 8.0 Hz), 7.53-7.47 (2H, m), 7.19 (1H, s), 6.80-6.00 (4H, brs).
M/z 430.31 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C;
Flow Rate: 0.6 mL/min.

**Prep. HPLC Condition:**

Column: Atlantis T3 (250*19) mm, 5 u;
Mobile phase: (A) 0.1% Formic Acid (B) Acetonitrile;
Flow: 19 mL/min;
Gradient (T/%B): 0/5, 1/5,7/25, 12/30, 12.1/99, 15/99, 15.1/5, 18/5;
Solubility: ACN +$H_2O$+THF+FA

**Example 44**

**5-[(4-guanidinophenyl)sulfonylaminolthiazole-4-carboxylic acid**

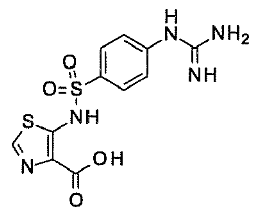

a. tert-butyl 5-[[4-[[N,N'-bis(tert-butoxycarbonyl)carbamimidoyl]amino]phenyl]sulfonyl-(4-methoxyphenyl)methyl]amino]thiazole-4-carboxylate

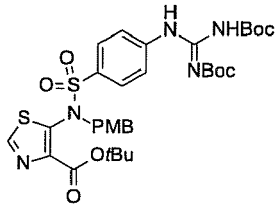

A solution of tert-butyl 5-[(4-aminophenyl)sulfonyl-[(4-methoxyphenyl)methyl]amino]thiazole-4-carboxylate (500 mg, 1.05 mmol) in THF (20 mL) was added to NaH (250 mg, 10.5 mmol) suspension in THF (20 mL) at 0 °C under argon atmosphere. After 30 minutes, a solution of tert-butyl N-[(tert-butoxycarbonylamino)-pyrazol-1-yl-methylene]carbamate (1.0 g, 3.43 mmol) in THF (10 mL) was added at 0 °C under argon atmosphere. The resulting reaction mixture was stirred at RT for 16 h, quenched with ice cold water (20 mL) and extracted with ethyl acetate (2 x 20 mL). The combined organic layer was dried over $Na_2SO_4$, filtered and concentrated under reduced pressure. The crude material was purified by trituration with diethyl ether (2 x 5 mL) to afford a pale yellow solid which was used in the next step without further purification (150 mg, crude).
M/z 662.03 $(M+H-Boc)^+$

b. 5-[(4-guanidinophenyl)sulfonylamino]thiazole-4-carboxylic acid

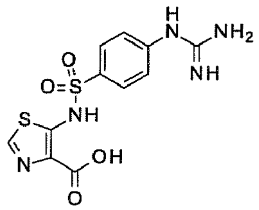

TFA:$H_2O$ (9:1, 2 mL) was added to tert-butyl 5-[[4-[[N,N'-bis(tert-butoxycarbonyl)carbamimidoyl]amino]phenyl]sulfonyl-[(4-methoxyphenyl)methyl]amino]thiazole-4-carboxylate (150 mg, 0.22 mmol) at RT. The resulting mixture was stirred for 4 h and concentrated under reduced pressure. The resulting crude product was triturated with diethyl ether (2 x 5 mL) and dried under high vacuum. The crude product was purified by preparative HPLC affording the title product as an off-white solid (22 mg, 28%).
$^1$H NMR (400 MHz, DMSO-$d_6$) $\delta$ 13.60 (1H, brs), 8.05 (1H, s), 7.74 (2H, d, $J$ = 8.8 Hz), 7.47 (3H, brs), 7.25 (2H, d, $J$= 8.8 Hz).
M/z 342.29 $(M+H)^+$

**LC-MS Condition:**

Column: Acquity BEH C18 (50 mm x 2.1 mm, 1.7 um);
Mobile Phase: A: 0.05% Formic Acid in water; B: 0.05% Formic Acid in ACN;
Time (min) /%B: 0/3, 0.4/3, 3.2/98, 3.8/98, 4.2/3, 4.5/3;
Column Temp: 35 °C,
Flow Rate: 0.6 mL/min.

**Prep. HPLC Condition:**

Column: Symmetry C18 (300*19) mm, 7 u;
Mobile phase: (A) 0.1% Formic Acid (B) Acetonitrile;
Flow: 19 mL/min;
Gradient (T/%B): 0/2, 1/2, 8/30, 9.10/99, 12/99, 12.10/2, 15/2;
Solubility: ACN +$H_2O$+DMSO.

**Example 45**

**5-[[3-fluoro-4-[[(2-guanidinoacetyl)amino]methyl]phenyl]sulfonylamino]thiazole-4-carboxylic acid**

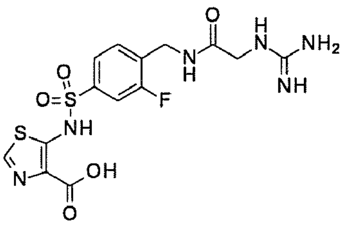

a. tert-butyl 5-[(3-fluoro-4-vinyl-phenyl)sulfonyl-[(4-methoxyphenyl)methyl]amino]thiazole-4-carboxylate

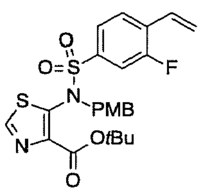

A solution of tert-butyl 5-[(4-bromo-3-fluoro-phenyl)sulfonyl-[(4-methoxyphenyl)methyl]amino]thiazole-4-carboxylate (3 g, 5.38 mmol) in 1,4-dioxane (40 mL) was purged with argon for 15 minutes. Then 4,4,5,5-tetramethyl-2-vinyl-1,3,2-dioxaborolane (0.99 g, 6.45 mmol), $K_2CO_3$ (1.11 g, 8.07 mmol), $PdCl_2(PPh_3)_2$ (0.37 g, 0.53 mmol) were added under argon atmosphere. The resulting reaction mixture was heated to 85 °C for 24 h in a closed vial. The reaction mixture temperature was allowed to cool to RT, filtered through a celite pad (washed with EtOAc (2 x 50 mL)). The organic layer was concentrated under reduced pressure. The resulting crude compound was dissolved in ethyl acetate (50 mL), washed with water (50 mL) and brine solution (50 mL). The organic layer was dried over $Na_2SO_4$, filtered and concentrated under vacuum. The crude compound was purified by flash chromatography (eluting with 20% ethyl acetate in petroleum ether) affording an off-white solid (1.5 g, 55%).
M/z 505.1 $(M+H)^+$

b. tert-butyl 5-[(3-fluoro-4-formyl-phenyl)sulfonyl-[(4-methoxyphenyl)methyl]amino]thiazole-4-carboxylate

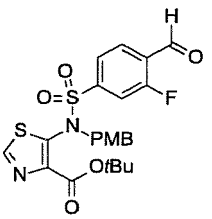

$NaIO_4$ (8.51 g, 39.8 mmol) and $OsO_4$ (1.68 g, 6.63 mmol) were added to a solution of tert-butyl 5-[(3-fluoro-4-vinyl-phenyl)sulfonyl-[(4-methoxyphenyl)methyl]amino]thiazole-4-carboxylate (6.7 g, 13.2 mmol) in acetonitrile:$H_2O$:$CCl_4$ (1:1:1, 60 mL). The resulting reaction mixture was stirred at RT for 4 h. Water (30 mL) was added and the mixture was extracted with ethyl acetate (2 x 100 mL). The combined organic extracts were dried over $Na_2SO_4$, filtered and concentrated. The crude material was purified by flash chromatography (eluting with 22% ethyl acetate in petroleum ether) affording an off-white solid (4.5 g, 66%).
M/z 507.4 $(M+H)^+$

c. tert-butyl 5-[[3-fluoro-4-[hydroxyiminomethyl]phenyl]sulfonyl-[(4-methoxyphenyl)methyl]amino]thiazole-4-carboxylate

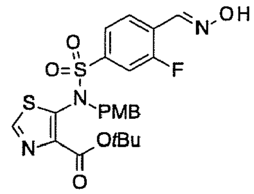

Tert-butyl 5-[(3-fluoro-4-formyl-phenyl)sulfonyl-[(4-methoxyphenyl)methyl]amino]thiazole-4-carboxylate (3.6 g, 7.10 mmol) solution in EtOH (20 mL) was added to a stirred solution of hydroxylamine hydrochloride (593.8 mg, 8.52 mmol) and ammonium chloride (454.9 mg, 8.52 mmol) in $H_2O$:EtOH (4:1, 30 mL). The resulting reaction mixture was stirred at RT for 4 h. Water (20 mL) was added and the mixture extracted with ethyl acetate (2 x 50 mL). The combined organic extracts were dried over $Na_2SO_4$, filtered and concentrated. The crude material was purified by flash chromatography (eluting with 40% ethyl acetate in petroleum ether) affording an off-white solid (2.8 g, 75%).
M/z 522.1 $(M+H)^+$

d. tert-butyl 5-[[4-(aminomethyl)-3-fluoro-phenyl]sulfonyl-[(4-methoxyphenyl)methyl]amino]thiazole-4-carboxylate

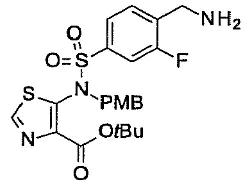

Zn dust (0.52 g, 8.04 mmol) was added to a stirred solution of tert-butyl 5-[[3-fluoro-4-[hydroxyiminomethyl]phenyl]sulfonyl-[(4-methoxyphenyl)methyl]amino]thiazole-4-carboxylate (2.8 g, 5.36 mmol) in AcOH (20 mL) at RT. The resulting reaction mixture was stirred at RT for 16 h. Ice cold water was added and the mixture was extracted with ethyl acetate (2 x 50 mL). The combined organic extracts were dried over $Na_2SO_4$, filtered and concentrated. The crude material was purified by trituration with diethyl ether (2 x 10 mL) affording a yellow solid (2 g, 73%).
M/z 508.1 $(M+H)^+$

e. tert-butyl 5-[[4-[[[2-(tert-butoxycarbonylamino)acetyl]amino]methyl]-3-fluorophenyl]sulfonyl-[(4-methoxyphenyl)methyl]amino]thiazole-4-carboxylate

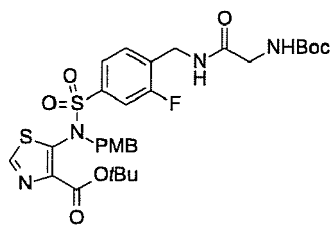

DIPEA (0.61 mL, 3.54 mmol) and HATU (0.67 g, 1.77 mmol) were added to a solution of tert-butyl 5-[[4-(aminomethyl)-3-fluoro-phenyl]sulfonyl-[(4-methoxyphenyl)methyl]amino]thiazole-4-carboxylate (0.6 g, 1.18 mmol) and 2-(tert-butoxycarbonylamino)acetic acid (0.31 g, 1.77 mmol) in DMF (20 mL) under argon atmosphere. The resulting reaction mixture was stirred at RT for 4 h. Ice cold water (10 mL) was added and the mixture was extracted with ethyl acetate (2 x 20 mL). The combined organic extracts were dried over $Na_2SO_4$, filtered and concentrated. The crude material was purified by flash chromatography (eluting with 50% ethyl acetate in petroleum ether) affording an off-white

solid (500 mg, 63%).
M/z 508.1 $(M+H)^+$

f. 5-[[4-[[(2-aminoacetyl)amino]methyl]-3-fluoro-phenyl]sulfonylamino]thiazole-4-carboxylic acid, trifluoroacetate

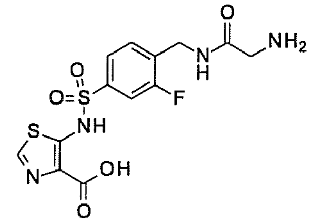

TFA (5 mL) was added to tert-butyl 5-[[4-[[[2-(tert-butoxycarbonylamino)acetyl]amino]methyl]-3-fluoro-phenyl]sulfonyl-[(4-methoxyphenyl)methyl]amino]thiazole-4-carboxylate (500 mg, 0.75 mmol) at RT and stirred for 4 h. TFA was evaporated by reduced pressure. The resulting crude product was triturated with diethyl ether (2 x 10 mL) and dried under high vacuum affording an off-white solid (250 mg, 85%).
M/z 389.1 $(M+H)^+$

g. 5-[[3-fluoro-4-[[(2-guanidinoacetyl)amino]methyl]phenyl]sulfonylamino]thiazole-4-carboxylic acid

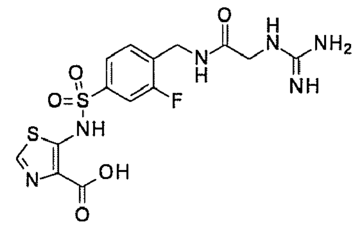

Pyrazole-1-carboxamidine, hydrochloride (141.2 mg, 0.96 mmol) and DIPEA (0.55 mL, 3.21 mmol) were added to a stirred solution of 5-[[4-[[(2-aminoacetyl)amino]methyl]-3-fluoro-phenyl]sulfonylamino]thiazole-4-carboxylic acid, trifluoroacetate (250 mg, 0.64 mmol) in DMF (6 mL) at RT. The resulting reaction mixture was stirred at RT for 18 h and concentrated under reduced pressure. Water (5 mL) was added to the residue. The resulting precipitate was filtered and washed with diethyl ether (2 x 5 mL). The crude product was purified by preparative HPLC to afford the title compound as a white solid (70 mg, 25%).
$^1$H NMR (500 MHz, DMSO-$d_6$) $\delta$ 13.43 (1H, brs), 8.60 (1H, brs), 8.08 (1H, s), 7.51-7.42 (3H, m), 7.50 -7.10 (4H, brs), 4.33 (2H, s), 3.85 (2H, s).
M/z 431.0 $(M+H)^+$

**<u>LC-MS Condition:</u>**

Column: Acquity UPLC BEH C18 (50mmx2.1 mm, 1.7um);
Mobile Phase: A: 0.1% Formic Acid in Water; B: 0.1% Formic Acid in Acetonitrile;
Flow Rate: 0.8 mL/min
Time (min) /%B: 0/2, 0.4/2, 2.2/98, 2.6/98, 2.61/2, 3.0/2.
Column Temp: 60 °C.

**<u>Prep. HPLC Condition:</u>**

Column: KROMASIL- C18 (150*25MM), 10u;
Mobile phase: 0.05% Formic acid in $H_2O$: ACETONITRILE;
Flow: 25 mL/min
Gradient (T/%B): 0/5, 1/5, 7/40, 7.1/98, 9/98, 9.1/5, 11/5;
Solubility: ACN +$H_2O$+THF+DMSO

Compounds prepared using methods analogous to those described above for Example 45 and purified in a similar manner by preparative HPLC are shown in the Table below:-

| Example | Structure | Name, NMR and Mass |
|---|---|---|
| 46 | 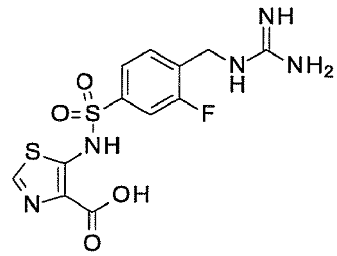 | 5-[[3-fluoro-4-(guanidinomethyl) phenyl]sulfonylamino]thiazole-4-carboxylic acid<br><br>M/z 374.0 $(M+H)^+$<br><br>$^1$HNMR(d6-DMSO) $\delta$ 13.40 (1H, s), 8.09 (1H, s), 7.59 (1H, d, $J$ = 7.5 Hz), 7.47 (1H, d, $J$ = 10 Hz), 7.59 (1H, t, $J$ = 7.5 Hz), 7.50-7.25 (4H, brs), 4.41 (2H, s). |

**Example 47: Activity of compounds of the invention**

Experiments were conducted to determine:

(1) The inhibitory activity of the compounds of the invention against MBL enzymes;
(2) The plasma protein binding for compounds of the invention; and
(3) The plasma stability of compounds of the invention.

Details of the protocols used for each of the sets of experiments are set out below:

**1. Enzymatic inhibition**

*In vitro enzyme inhibition assays*

Enzyme inhibition assays were performed using purified MBL enzymes (NDM-1; VIM-1; VIM-2; IMP-1) in 10mM HEPES buffer pH 7.5 in 96-well microtiter plates. Imipenem (300$\mu$M) was used as substrate and its hydrolysis was followed at UV 299 nm during 10mn every 30 seconds using a Perkin Elmer Envision UV fluorescence plate reader. Hydrolysis rate data in presence of a range of inhibitors was analysed using Dotmatics database software and calculated $IC_{50}$ values were converted to Ki values using the Cheng-Prusoff equation:

$$Ki = IC_{50} / (1+([S]/K_m)$$

where the $K_m$ values for NDM-1, VIM-2 and IMP-1 are 70 $\mu$M, 1.5 $\mu$M, 9 $\mu$M and 25 $\mu$M respectively. Compound dilution was performed in DMSO.

Mean Ki values from multiple experiments are presented below. Experimental results are shown given using the following bands:

- For NDM-1 the Ki values of < 0.05 $\mu$M are designated (A); Ki values of 0.05 - 0.2 $\mu$M are designated (B); Ki values of > 0.2 $\mu$M (0.2 - 2 $\mu$M) are designated (C).

- For VIM-1 the Ki values of < 0.2 $\mu$M are designated (A); Ki values of 0.2 - 0.5 $\mu$M are designated (B); Ki values of > 0.5 $\mu$M (0.5 - 1 $\mu$M) are designated (C).

- For VIM-2 the Ki values of < 0.02 $\mu$M are designated (A); Ki values of 0.02 - 0.05 $\mu$M are designated (B); Ki values of> 0.05 $\mu$M (0.05 - 0.15 $\mu$M) are designated (C).

- For IMP-1 the Ki values of < 0.5 μM are designated (A); Ki values of 0.5 - 1 μM are designated (B); Ki values of > 1 μM (1 - 10 μM) are designated (C).

**Ki values for compounds of the invention.**

| Example | Ki / μM | | | |
|---|---|---|---|---|
| | NDM-1 | VIM-1 | VIM-2 | IMP-1 |
| 2 | (A) | (B) | (B) | (C) |
| 3 | (B) | (B) | (B) | (C) |
| 4 | (A) | (B) | (B) | (C) |
| 5 | (B) | (A) | (B) | (A) |
| 6 | (C) | (B) | | (C) |
| 7 | (B) | (B) | (C) | (C) |
| 8 | (B) | (B) | | (C) |
| 9 | (B) | (B) | (A) | (C) |
| 10 | (A) | (A) | | (B) |
| 11 | (C) | (B) | | (C) |
| 12 | (B) | (B) | | (B) |
| 13 | (A) | (A) | (C) | (B) |
| 14 | (C) | (B) | | (C) |
| 15 | (B) | (C) | | (C) |
| 16 | (B) | (A) | (A) | (B) |
| 17 | (B) | (A) | (A) | (A) |
| 18 | (B) | (A) | (A) | (A) |
| 19 | (B) | (B) | (B) | (C) |
| 20 | (C) | (B) | | |
| 21 | (A) | (B) | | (B) |
| 22 | (A) | (B) | | (C) |
| 23 | (A) | (A) | (B) | (C) |
| 24 | (B) | (C) | | (C) |
| 25 | (B) | (B) | | (B) |
| 26 | (A) | (C) | (C) | (C) |
| 27 | (A) | (A) | | (B) |
| 28 | (B) | (A) | (A) | (A) |
| 29 | (A) | (B) | (B) | (B) |
| 30 | (A) | (B) | | (C) |
| 31 | (A) | (A) | | (A) |
| 32 | (B) | (B) | (B) | (C) |
| 33 | (C) | (B) | | (C) |
| 34 | (A) | (C) | | (A) |
| 35 | (A) | (A) | (B) | (B) |

(continued)

| Example | Ki / μM | | | |
|---|---|---|---|---|
| | NDM-1 | VIM-1 | VIM-2 | IMP-1 |
| 36 | (A) | (B) | | (C) |
| 37 | (C) | (A) | | (C) |
| 38 | (C) | (B) | | (C) |
| 39 | (C) | (A) | (B) | (C) |
| 40 | (C) | (B) | | (C) |
| 41 | (A) | (B) | (B) | (B) |
| 42 | (C) | (C) | | (C) |
| 43 | (B) | (B) | | (A) |
| 44 | (C) | (B) | | (C) |
| 45 | (C) | (C) | (C) | (C) |
| 46 | (B) | (B) | (C) | (C) |

**2. Antimicrobial susceptibility testing**

*Antibiotic activity of β-lactam antibiotics on MBL expressing bacteria in the presence of the compounds of the invention*

The experiments were carried out using the 'broth micro-dilution method' according to the protocols M07-A8 established by the Clinical Laboratory Standards Institute (CLSI). Serial dilutions of the β-lactam antibiotic (Meropenem) were prepared in 96-well plates in cation-adjusted Mueller-Hinton broth (CAMHB); the concentration range was defined from 0.03 mg/L to 512 mg/L. The compounds were added at a constant concentration of 8 μg/mL. A bacterial inoculum of each strain (clinical isolates) was adjusted to a 0.5 McFarland turbidity standard in physiologic serum (0.9 % NaCl), then diluted 1:100 in CAMHB and added to each well to give a final bacterial cell number of $5x10^5$ CFU/well. After incubation for 18-20 hours in a heating chamber at 37°C, the growth inhibition was evaluated by the absence of any bacterial development.

Minimal inhibitory concentrations (MIC) are taken as the lowest concentration of antibiotic at which the test organism did not show visible growth; results were confirmed by measuring the optical density (OD) at 600 nm in a spectrophotometer.

Compounds of the invention were tested at a constant concentration of 8 μg/mL. The clinical strains used in these potentiation experiments were NTBC020 (*E. coli* strain expressing NDM-1, TEM-1 and CTX-M-15); NTBC035-2 (*K. pneumoniae* strain expressing NDM-1, CMY-4 and SHV-11); NTBC104-1 (*K. pneumoniae* strain expressing NDM-1 and SHV-11); NTBC123 *(K. pneumoniae* strain expressing NDM-1); NTBC018 (*C.freundii* strain expressing VIM-2); NTBC024 (*K. pneumoniae* strain expressing VIM-19, TEM-1 and CTX-M-3); NTBC042 (*E. coli* strain expressing VIM-1, TEM-1, CTX-M-15, SHV-12); NTBC055 (*E. Coli* strain expressing VIM-1); NTBC062 *(K. pneumoniae* strain expressing IMP-1 and TEM-1) and NTBC039 (*K. oxytoca* strain expressing IMP-28).

Results are shown below. Data are banded as follows: MIC values of < 1 μg/mL are designated (A); MIC values of 1 - 2 μg/mL are designated (B); MIC values of >2 μg/mL (2 - 200 μg/mL) are designated (C).

| Example | Strain | | | | | | | | | |
|---|---|---|---|---|---|---|---|---|---|---|
| | NTBC020 | NTBC035-2 | NTBC104-1 | NTBC123 | NTBC018 | NTBC024 | NTBC042 | NTBC055 | NTBC062 | NTBC039 |
| 2 | (B) | (B) | (C) | (C) | (A) | (B) | (A) | (B) | (B) | (B) |
| 3 | (C) | (B) | (B) | (C) | (A) | (B) | (A) | (B) | (B) | (C) |
| 4 | (B) | (B) | (C) | (C) | (A) | (B) | (A) | (B) | (B) | (B) |
| 5 | (B) | (B) | (C) | (C) | (A) | (A) | (A) | (B) | (B) | (A) |
| 7 | (B) | (B) | (C) | (C) | (A) | (B) | (A) | (B) | (B) | (B) |
| 10 | (C) | (B) | (C) | (C) | (A) | (A) | (A) | (B) | (B) | (B) |
| 13 | (B) | (B) | (C) | (C) | (A) | (B) | (A) | (B) | (B) | (B) |
| 15 | (C) | (C) | (C) | (C) | (B) | (C) | (C) | (C) | (C) | (C) |
| 16 | (C) | (C) | (C) | (C) | (A) | (A) | (A) | (A) | (B) | (B) |
| 17 | (C) | (C) | (C) | (C) | (A) | (A) | (A) | (B) | (B) | (A) |
| 19 | (B) | (B) | (C) | (C) | (A) | (B) | (A) | (B) | (B) | (B) |
| 20 | (C) | (C) | (C) | (C) | (A) | (C) | (A) | (B) | (B) | (B) |
| 22 | (B) | (B) | (C) | (C) | (A) | (C) | (B) | (C) | (B) | (B) |
| 23 | (C) | (B) | (A) | (C) | (A) | (B) | (B) | (B) | (B) | (C) |
| 24 | (C) | (C) | (C) | (C) | (A) | (B) | (A) | (B) | (C) | (C) |
| 25 | (C) | (C) | (C) | (C) | (A) | (C) | (B) | (B) | (B) | (B) |
| 26 | (A) | (A) | (B) | (B) | (A) | (A) | (A) | (B) | (C) | (C) |
| 27 | (C) | (C) | (C) | (C) | (B) | (B) | (A) | (B) | (B) | (B) |
| 29 | (C) | (B) | (C) | (C) | (A) | (B) | (A) | (C) | (B) | (B) |
| 30 | (C) | (C) | (B) | (C) | (A) | (A) | (A) | (B) | (B) | (B) |
| 32 | (B) | (B) | (C) | (C) | (A) | (B) | (A) | (B) | (B) | (B) |
| 34 | (C) | (C) | (C) | (C) | (A) | (B) | (A) | (B) | (B) | (B) |
| 35 | (C) | (B) | (B) | (C) | (A) | (B) | (A) | (B) | (B) | (B) |
| 36 | (C) | (C) | (C) | (C) | (A) | (C) | (A) | (B) | (B) | (B) |
| 38 | (C) | (C) | (C) | (C) | (B) | (C) | (B) | (C) | (B) | (C) |

(continued)

| Example | Strain | | | | | | | | | |
|---|---|---|---|---|---|---|---|---|---|---|
| | NTBC020 | NTBC035-2 | NTBC104-1 | NTBC123 | NTBC018 | NTBC024 | NTBC042 | NTBC055 | NTBC062 | NTBC039 |
| 41 | (B) | (B) | (C) | (C) | (A) | (B) | (A) | (B) | (B) | (B) |
| 43 | (C) | (C) | (C) | (C) | (A) | (B) | (B) | (B) | (B) | (C) |
| 44 | (C) | (C) | (C) | (C) | (A) | (B) | (A) | (A) | (B) | (C) |
| 45 | (C) | (C) | (C) | (C) | (B) | (C) | (B) | (B) | (B) | (B) |
| 46 | (C) | (B) | (C) | (C) | (B) | (B) | (A) | (A) | (B) | (C) |

**Example 48: Comparative Study**

**3. Plasma protein binding**

*Protocol Summary*

| | |
|---|---|
| **Method** | Rapid equilibrium dialysis |
| **Species** | Mice and Human Plasma |
| **Plasma** | 100% plasma |
| **Test compound concentration** | 10 μM |
| **Buffer** | Phosphate buffer saline pH 7.4 |
| **Incubation Time** | 5 h |
| **No of replicates** | 2 |
| **QC Compounds** | Warfarin |
| **Final DMSO Concentration** | <0.1 % |
| **Analytical Method** | LC-MS/MS |

*Assay Procedure*

Test compound was spiked in plasma to a final concentration of 10 μM. An aliquot of 300 μL of plasma was placed in red chamber of the insert and 500 μL of PBS was placed into white chamber of the insert. The plate was incubated at 37 °C in thermomixer at 400 rpm for 5 hours. After incubation, the samples were matrix equilibrated with opposite matrix (10 μL of plasma/100 μL of buffer sample was matched with 100 μL of blank buffer/10μL of plasma). Matrix matched samples were precipitated with 200 μL of acetonitrile containing internal standard. Samples were vortexed at 1000 rpm for 5 min and centrifuged at 4000 rpm for 10 min. Supernatant was separated, diluted 2 fold with water and analyzed on LC-MS/MS. Blank control samples were processed immediately after the preparation of plasma working stock solutions. These samples served as a measure for calculating the % recovery of test compounds.

*Data Analysis and Calculation*

The percent plasma bound fraction was calculated by the following equations:

$$\% \text{ Unbound} = 100 * F_C / T_C$$

$$\% \text{ Recovery} = 100 * (F_C + T_C) / T_0$$

where

Tc = Total compound concentration as determined by the calculated concentration on the plasma side of the membrane
Fc = Free compound concentration as determined by the calculated concentration on the buffer side of the membrane
To = Total compound concentration as determined before dialysis

For each set of duplicates / compound, the percentage bound, percentage unbound and percentage recovery was determined. Results are as shown below.

**4. Plasma stability of test compounds**

*Protocol Summary*

| | |
|---|---|
| **Test compound concentration** | 1 μM |
| **Matrix** | Mice and Human Plasma |
| **Incubation Time** | 0, 1, 3 and 5h |
| **No of replicates** | 2 per each time point |
| **QC Compounds** | Propantheline |
| **Analytical Method** | LC-MS/MS |
| **End point** | % Remaining of the test compound |

*Assay Procedure*

Test compound and QC compound were incubated at a final concentration of 1 μM in plasma at 37 °C in shaker water bath with gentle shaking. At predetermined time points, reaction was terminated with 200 μL of acetonitrile containing internal standard and centrifuged at 4000x RCF, 4°C for 20 minutes. Supernatant was separated and analyzed by LC-MS/MS.

*Data Analysis and Calculation*

The following equation was used to determine the percentage remaining of test/QC compound following the procedure above:

$$\%\ \text{remaining of the test substance} = \frac{\text{Peak Area ratio at time (min)}}{\text{Peak Area Ratio at 0 min}} \times 100$$

Results are as shown below.

Studies were undertaken to compare the compounds of the invention with structurally similar compounds ("Comp. x"). Experiments were conducted as described above. Data were banded as set out below.

- For VIM-1 the Ki values of < 0.15 μM are designated (++++); Ki values of 0.15 - 0.3 μM are designated (+++); Ki values of 0.3 - 0.5 μM are designated (++); Ki values of > 0.5 μM (0.5 - 1 μM) are designated (+).
- For IMP-1 the Ki values of < 0.15 μM are designated (++++); Ki values of 0.15 - 0.6 μM are designated (+++); Ki values of 0.6 - 5 μM are designated (++); Ki values of > 5 μM (5 - 10 μM) are designated (+).
- For VIM-2 the Ki values of < 0.02 μM are designated (++++); Ki values of 0.02 - 0.05 μM are designated (+++); Ki values of 0.05 - 0.1 μM are designated (++); Ki values of > 0.1 μM (0.1 - 0.15 μM) are designated (+).
- For NDM-1 the Ki values of < 0.03 μM are designated (++++); Ki values of 0.03 - 0.1 μM are designated (+++); Ki values of 0.1 - 0.3 are designated (++); Ki values of > 0.5 μM (0.3 - 2 μM) are designated (C).

| Example | | Stability in human plasma | Plasma protein binding |
|---|---|---|---|
| Comp. A | 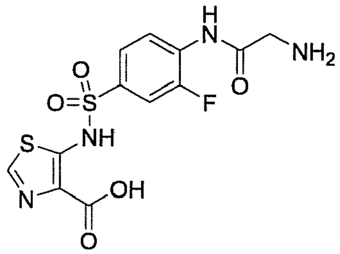 | 29% remaining after 2 hours | 90.3% |
| Example 7 | 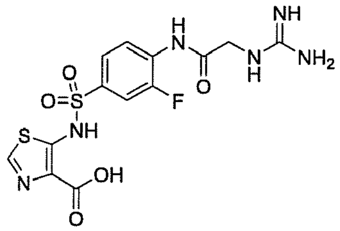 | 100% remaining after 5 hours | 60.8% |

MBL-inhibitory efficacy was observed for the compound of Example 7.

| Example | | Ki / μM | | | |
|---|---|---|---|---|---|
| | | VIM-1 | IMP-1 | VIM-2 | NDM-1 |
| Comp. B | 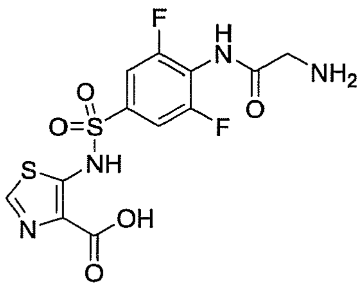 | ++ | | | |
| Example 2 | 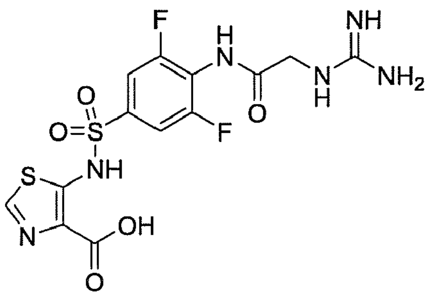 | +++ | | | |
| | | | | | |
| Comp. C | 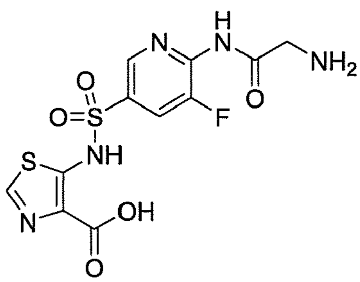 | + | + | + | + |

(continued)

| Example | | Ki / µM | | | |
|---|---|---|---|---|---|
| | | VIM-1 | IMP-1 | VIM-2 | NDM-1 |
| Example 3 | 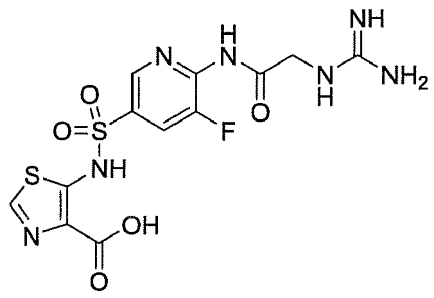 | ++ | ++ | +++ | ++ |
| | | | | | |
| Comp. D | 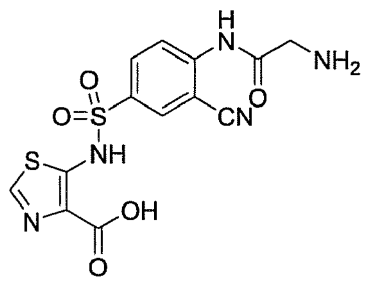 | + | | | |
| Example 5 | 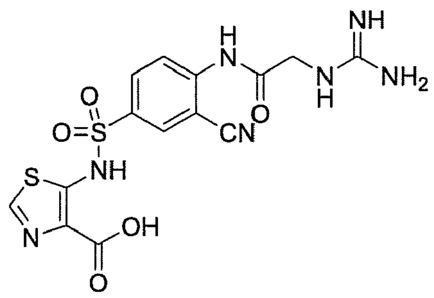 | +++ | | | |
| Comp. E | 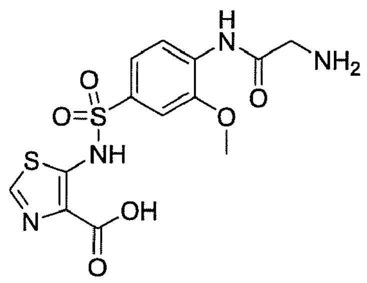 | ++ | ++ | | ++ |
| Example 16 | 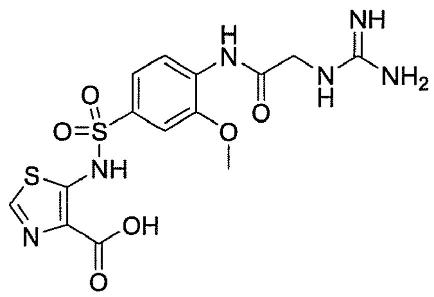 | ++++ | +++ | | +++ |
| | | | | | |

(continued)

| Example | | Ki / μM | | | |
|---|---|---|---|---|---|
| | | VIM-1 | IMP-1 | VIM-2 | NDM-1 |
| Comp. F | 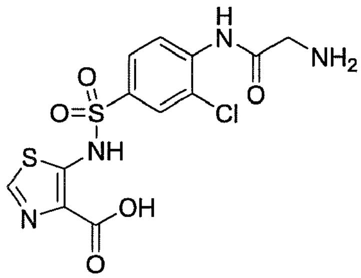 | +++ | | | |
| Example 17 | 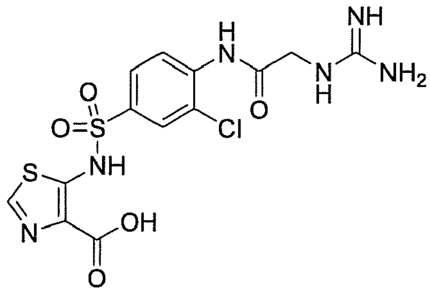 | ++++ | | | |
| Comp. G | 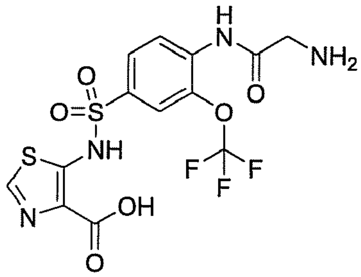 | ++ | +++ | +++ | |
| Example 18 | 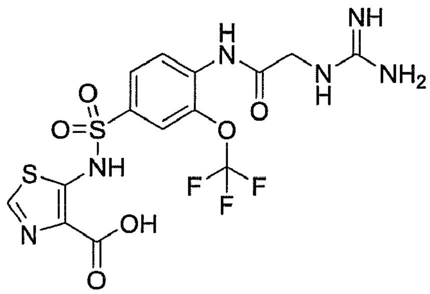 | ++++ | ++++ | ++++ | |
| | | | | | |
| Comp. H | 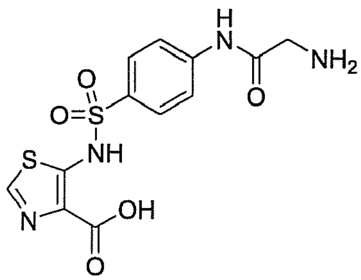 | ++ | | | |

(continued)

| Example | | Ki / μM | | | |
|---|---|---|---|---|---|
| | | VIM-1 | IMP-1 | VIM-2 | NDM-1 |
| Example 20 | 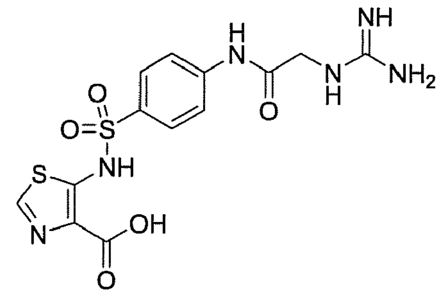 | +++ | | | |
| Comp. I | 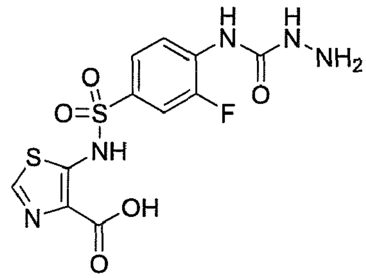 | + | | | ++ |
| Example 29 | 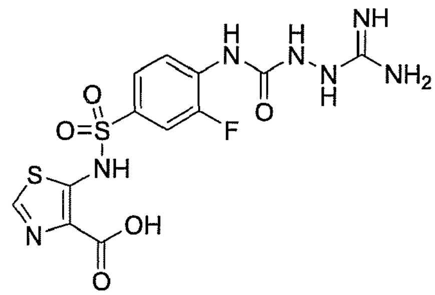 | +++ | | | ++++ |

The compounds of the invention were also found to exhibit better potentiation (lower MIC values) against the above mentioned MBL enzymes (VIM/IMP/NDM) as compared to the structurally related analogues lacking a -C(NR)-NR2 motif.

As can be readily seen, the compounds of the invention are associated with improved properties compared with structurally analogous compounds. This finding is surprising, not least because the -C(NR)-$NR_2$ motif common to the compounds of the invention can be associated with rapid hydrolysis, so may have been expected to render the compounds unsuitable for the type of use described herein. The fact that this motif can be introduced not only without prejudicing the efficacy of the compounds, but also with an enhancement of plasma stability and efficacy, is thus unexpected.

## Claims

**1.** A compound which is a thiazole derivative of Formula (I), or a pharmaceutically acceptable salt thereof,

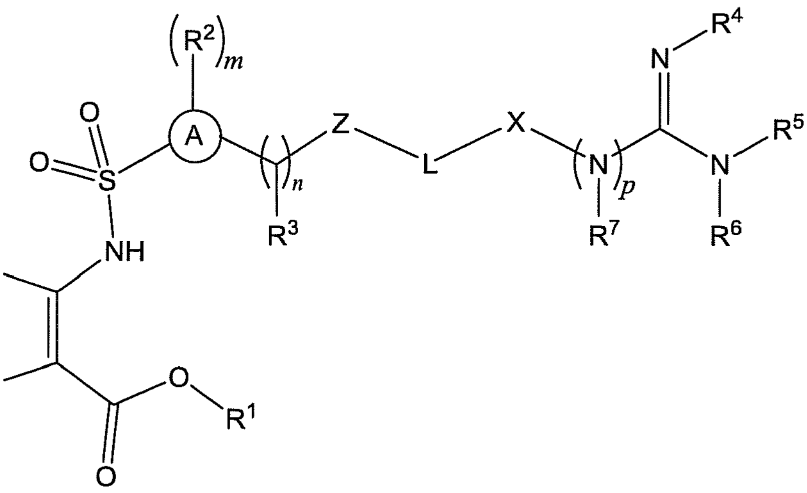

[FORMULA (I)]

wherein

- $R^1$ is selected from H, $R^{1a}$ and $-CH_2OC(O)R^{1a}$, wherein $R^{1a}$ is selected from an unsubstituted $C_1$ to $C_4$ alkyl group and phenyl;
- Ⓐ is a cyclic group selected from $C_6$ to $C_{10}$ aryl, 5- to 10-membered heteroaryl, and 4- to 10- membered carbocyclic and heterocyclic groups;
- each $R^2$ is independently selected from:

  (iv) halo or $R^8$;
  (v) $C_{1-3}$ alkyl, $O(C_{1-3}$ alkyl), $S(C_{1-3}$ alkyl), $SO(C_{1-3}$ alkyl) or $SO_2(C_{1-3}$ alkyl), any of which may optionally be substituted with 1, 2 or 3 halo substituents and/or one $R^8$ substituent; and
  (vi) $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;
  and

  - each $R^8$ is independently selected from CN, OH, $-C(O)NR^fR^g$, $-NR^fR^g$, $-NR^{10}C(NR^{11})R^{12}$, $-C(NR^{10})NR^{11}R^{12}$, and $-NR^{10}C(NR^{11})NR^{12}R^{13}$; wherein each of $R^f$ and $R^g$ is independently H or unsubstituted $C_{1-2}$ alkyl;

- *m* is 0, 1, 2 or 3
- $R^3$ is selected from hydrogen and a $C_1$ to $C_3$ alkyl group which is unsubstituted or is substituted with 1, 2 or 3 substituents selected from halogen, $-OR^{10}$, and $-NR^{10}R^{11}$;
- *n* is 0 or 1
- Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$, $-NR^{10}C(O)S-$, $-SC(O)NR^{10}$, $-NR^{10}C(NR^{11})-$, $-C(NR^{10})NR^{11}-$, $-NR^{10}C(NR^{11})NR^{12}-$, $-NR^{10}C(N^+R^{11}R^{12})-$, $-C(N^+R^{10}R^{11})NR^{12}-$, $-NR^{10}C(N^+R^{11}R^{12})NR^{13}-$, $-NR^{10}C(NR^{11})O-$, $-OC(NR^{10})NR^{11}$, $-NR^{10}C(N^+R^{11}R^{12})O-$, $-OC(N^+R^{10}R^{11})NR^{12}-$, $-NR^{10}C(NR^{11})S-$, $-SC(NR^{10})NR^{11}$, $-NR^{10}C(N^+R^{11}R^{12})S-$, and $-SC(N^+R^{10}R^{11})NR^{12}-$;
- L is a bond or is selected from $C_{1-4}$ alkylene, $C_{2-4}$ alkenylene, $C_{2-4}$ alkynylene, $C_{1-3}$ alkylene-($C_{3-6}$cycloalkylene)-$C_{1-3}$ alkylene, $C_{1-4}$ alkylene-($C_{3-6}$cycloalkylene) and ($C_{3-6}$cycloalkylene)-$C_{1-4}$ alkylene, wherein L is unsubstituted or is substituted with 1 or 2 substituents selected from halogen, $-OR^{10}$, and $-NR^{10}R^{11}$; or L is $-C(R^{10})=N-$;
- X is a bond or, when L is other than a bond or $-C(R^{10})=N-$, X is a bond or is selected from $-NR^{10}-$, -O-, $-NR^{10}C(NR^{11})-$, and $-C(NR^{10})-$;
- *p* is 0 or 1;
- $R^4$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or $R^4$ is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$,

$-NR^{10}R^{11}$, and -CN;

◦ $R^5$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or $R^5$ is joined together with $R^4$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

or $R^5$ is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ $R^6$ is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or $R^6$ is joined together with $R^5$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

or $R^6$ is joined together with $R^7$ if present to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ $R^7$ if present is selected from H, -CN and $C_1$ to $C_3$ alkyl which is unsubstituted or is substituted with 1, 2 or 3 substituents selected from halogen, $-OR^{10}$, $-NR^{10}R^{11}$, and -CN;

or $R^7$ is joined together with $R^6$ to form, together with the atoms to which they are attached, a 5- to 6- membered heterocyclic group comprising at least one saturated carbon atom in the ring, said heterocyclic group being unsubstituted or substituted with 1 or 2 substituents selected from unsubstituted $C_1$ to $C_2$ alkyl, halogen, $-OR^{10}$, $-NR^{10}R^{11}$, and -CN;

◦ each $R^{10}$, $R^{11}$, $R^{12}$, $R^{13}$ and $R^{14}$ is independently H or methyl.

**2.** A compound of Formula (I) wherein $R^1$ is H.

**3.** A compound according to claim 1 or 2, wherein Ⓐ is a cyclic group selected from phenyl, 5- to 6-membered heteroaryl, and 5- to 6- membered carbocyclic and heterocyclic groups.

**4.** A compound according to any one of claims 1 to 3 wherein Ⓐ is selected from phenyl, cyclohexane, piperidine, pyridazine, pyridine and thiazole.

**5.** A compound according to any one of the preceding claims wherein each $R^2$ is independently selected from:

(vii) halo, CN, OH, $-C(O)NR^fR^g$, $-NR^fR^g$; wherein each of $R^f$ and $R^g$ is independently H or methyl; and

(viii) $C_{1-2}$ alkyl, $O(C_{1-2}$ alkyl), $S(C_{1-2}$ alkyl), $SO(C_{1-2}$ alkyl) any of which may optionally be substituted with 1, 2 or 3 halo substituents and/or one substituent selected from CN and OH.

**6.** A compound according to any one of the preceding claims wherein $R^3$ is H.

**7.** A compound according to any one of the preceding claims wherein *n* is 0.

**8.** A compound according to any one of the preceding claims wherein Z is a bond or is selected from $-NR^{10}C(O)-$, $-C(O)NR^{10}-$, $-NR^{10}C(O)NR^{11}-$, $-NR^{10}C(O)O-$, $-OC(O)NR^{10}$, $-NR^{10}C(O)S-$, $-SC(O)NR^{10}$, $-NR^{10}C(NR^{11})-$, $-C(NR^{10})NR^{11}-$, and $-NR^{10}C(NR^{11})NR^{12}-$.

**9.** A compound according to any one of the preceding claims wherein Z is selected from $-NR^{10}C(O)-$, $-C(O)NR^{10}-$, and $-NR^{10}C(O)NR^{11}-$.

**10.** A compound according to any one of the preceding claims wherein L is a bond or is selected from $C_{1-4}$ alkylene, $C_{2-4}$ alkenylene, and $C_{2-4}$ alkynylene; or L is - $C(R^{10})=N-$.

**11.** A compound according to any one of the preceding claims wherein L is selected from $C_{1-3}$ alkylene and $C_{2-3}$

alkenylene.

**12.** A compound according to any one of the preceding claims wherein X is a bond.

**13.** A compound according to any one of the preceding claims wherein *p* is 1 and $R^7$ is H or methyl or is joined together with $R^6$ to form, together with the atoms to which they are attached, an unsubstituted 5- to 6- membered heterocyclic group comprising at least one saturated carbon atom in the ring.

**14.** A compound according to any one of the preceding claims wherein $R^4$ is H or is joined together with $R^5$ to form, together with the atoms to which they are attached, an unsubstituted 5- to 6- membered heterocyclic group comprising at least one saturated carbon atom in the ring.

**15.** A compound according to any one of the preceding claims for use in the treatment or prevention of bacterial infection.

**16.** A compound according to any one of claims 1 to 14 wherein $R^5$ is selected from H, -CN and $C_1$ to $C_2$ alkyl which is unsubstituted or is substituted with 1, 2 or 3 halo substituents and/or one -$NR^{10}R^{11}$ substituent and $R^6$ is H or methyl.

**17.** A compound according to any one of claims 1 to 14 or 16 wherein:

- $R^1$ is H;
- Ⓐ is a cyclic group selected from phenyl, 5- to 6-membered heteroaryl, and 5- to 6-membered carbocyclic and heterocyclic groups;
- *m* is 0, 1 or 2;
- each $R^2$ is independently selected from:
    - halo or $R^8$;
    - $C_{1-2}$ alkyl, O($C_{1-2}$ alkyl), S($C_{1-2}$ alkyl), SO($C_{1-2}$ alkyl) or $SO_2$($C_{1-2}$ alkyl), any of which may optionally be substituted with 1, 2 or 3 halo substituents and/or one $R^8$ substituent; and
    - $NR^aC(O)R^c$, and $NR^aC(O)NR^bR^c$, wherein each $R^a$ and $R^b$ is independently selected from hydrogen and unsubstituted $C_{1-2}$ alkyl and each $R^c$ is unsubstituted $C_{1-2}$ alkyl;
- each $R^8$ is independently selected from CN, OH, -$C(O)NR^fR^g$, and -$NR^fR^g$; wherein each of $R^f$ and $R^g$ is independently H or unsubstituted $C_{1-2}$ alkyl.
- *n* is 0; or *n* is 1 and $R^3$ is H
- Z is selected from -$NR^{10}C(O)$-, -$C(O)NR^{10}$-, -$NR^{10}C(O)NR^{11}$-, -$NR^{10}C(O)O$-, -$OC(O)NR^{10}$, -$NR^{10}C(O)S$-, -$SC(O)NR^{10}$, -$NR^{10}C(NR^{11})$-, -$C(NR^{10})NR^{11}$-, and -$NR^{10}C(NR^{11})NR^{12}$-;
- L is a bond or is selected from $C_{1-4}$ alkylene, $C_{2-4}$ alkenylene and $C_{2-4}$ alkynylene; or L is -$C(R^{10})$=N-;
- X is a bond;
-

    i) *p* is 0;
    $R^4$ is H and $R^5$ is selected from H, -CN and $C_1$ to $C_2$ alkyl which is unsubstituted or is substituted with 1, 2 or 3 halo substituents and/or one - $NR^{10}R^{11}$ substituent; or $R^4$ is joined together with $R^5$ to form, together with the atoms to which they are attached, an unsubstituted 5- to 6- membered heterocyclic group comprising at least one saturated carbon atom in the ring; and
    $R^6$ is H or methyl;

  or

    ii) *p* is 1; and
    $R^4$ is H; $R^5$ is selected from H, -CN and $C_1$ to $C_2$ alkyl which is unsubstituted or is substituted with 1, 2 or 3 halo substituents and/or one - $NR^{10}R^{11}$ substituent; $R^6$ is H or methyl and $R^7$ is H or methyl; or $R^4$ is joined together with $R^5$ to form, together with the atoms to which they are attached, an unsubstituted 5- to 6- membered heterocyclic group comprising at least one saturated carbon atom in the ring; $R^6$ is H or methyl and $R^7$ is H;

**18.** A compound according to any one of claims 1 to 14 or 16 to 17 wherein:

- $R^1$ is H;
- Ⓐ is selected from phenyl, cyclohexane, piperidine, pyridazine, pyridine and thiazole;
- *m* is 1 or 2;
- each $R^2$ is independently selected from:

  ◦ halo, CN, OH, -C(O)$NR^fR^g$, -$NR^fR^g$; wherein each of $R^f$ and $R^g$ is independently H or methyl; and
  o $C_{1-2}$ alkyl, O($C_{1-2}$ alkyl), S($C_{1-2}$ alkyl), SO($C_{1-2}$ alkyl) any of which may optionally be substituted with 1, 2 or 3 substituents selected from halo, CN, OH;

- *n* is 0;
- Z is selected from -$NR^{10}$C(O)-, -C(O)$NR^{10}$-, and -$NR^{10}$C(O)$NR^{11}$-;
- L is selected from $C_{1-3}$ alkylene and $C_{2-3}$ alkenylene.
- X is a bond;
- *p* is 0; or *p* is 1 and $R^7$ is H;
- $R^4$ is H;
- $R^5$ is selected from H, -CN and $C_1$ to $C_2$ alkyl which is unsubstituted or is substituted with 1, 2 or 3 halo substituents and/or one -$NR^{10}R^{11}$ substituent H; and
- $R^6$ is H.

**19.** A compound according to any one of claims 1 to 14 or 16 to 18 wherein $R^4$, $R^5$, $R^6$ and $R^7$ if present are each hydrogen.

**20.** A compound according to claim 1, which compound is selected from:

- 5-[[4-[(2-guanidinoacetyl)amino]-3-(trifluoromethoxy)phenyl]sulfonylamino] thiazole-4-carboxylic acid;
- 5-[[3-fluoro-4-[[(2-guanidinoacetyl)amino]methyl]phenyl]sulfonylamino]thiazole-4-carboxylic acid;
- 5-[[3-fluoro-4-(guanidinomethyl)phenyl]sulfonylamino]thiazole-4-carboxylic acid;
- 5-[[3-fluoro-4-(2-guanidinoethylsulfanylcarbonylamino)phenyl]sulfonylamino] thiazole-4-carboxylic acid;
- 5-[[4-[2-[(2-amino-2-imino-ethyl)amino]-2-oxo-ethyl]-3-fluoro-phenyl] sulfonylamino]thiazole-4-carboxylic acid;
- 5-[[3-carbamoyl-4-[(2-guanidinoacetyl)amino]phenyl]sulfonylamino]thiazole-4-carboxylic acid;
- 5-[[3-cyano-4-[(2-guanidinoacetyl)amino]phenyl]sulfonylamino]thiazole-4-carboxylic acid;
- 5-[[3-fluoro-4-(2-guanidinoethoxycarbonylamino)phenyl]sulfonylamino]thiazole-4-carboxylic acid;
- 5-[(4-guanidinophenyl)sulfonylamino]thiazole-4-carboxylic acid;
- 5-[[4-[2-(2-carbamimidoylhydrazino)-2-oxo-ethyl]-3-fluoro-phenyl]sulfonylamino] thiazole-4-carboxylic acid;
- 5-[[3-chloro-4-[(2-guanidinoacetyl)amino]phenyl]sulfonylamino]thiazole-4-carboxylic acid;
- 5-[[4-[(2-guanidinoacetyl)amino]-3-methoxy-phenyl]sulfonylamino]thiazole-4-carboxylic acid;
- 5-[[4-[[2-(2-carbamimidoylhydrazino)acetyl]amino]-3-fluorophenyl]sulfonylamino] thiazole-4-carboxylic acid;
- 5-[[4-[[(2E)-2-(carbamimidoylhydrazono)acetyl]amino]-3-fluoro-phenyl] sulfonylamino]thiazole-4-carboxylic acid;
- 5-[[4-[[2-(4,5-dihydro-1H-imidazol-2-ylamino)acetyl]amino]-3,5-difluoro-phenyl] sulfonylamino]thiazole-4-carboxylic acid;
- 5-[[6-[(2-guanidinoacetyl)amino]pyridazin-3-yl]sulfonylamino]thiazole-4-carboxylic acid;
- 5-[[4-[(2-amino-2-imino-ethyl)carbamoylamino]-3-fluoro-phenyl]sulfonylamino] thiazole-4-carboxylic acid;
- 5-[[3,5-difluoro-4-(guanidinocarbamoylamino)phenyl]sulfonylamino]thiazole-4-carboxylic acid;
- 5-[[4-[(3-amino-3-imino-propanoyl)amino]-3,5-difluoro-phenyl]sulfonylamino] thiazole-4-carboxylic acid;
- 5-[[4-[[3-(dimethylamino)-3-imino-propanoyl]amino]-3-fluoro-phenyl] sulfonylamino]thiazole-4-carboxylic acid;
- 5-[[3-fluoro-4-[(2-guanidinooxyacetyl)amino]phenyl]sulfonylamino]thiazole-4-carboxylic acid;
- 5-[[3-fluoro-4-[[3-imino-3-(methylamino)propanoyl]amino]phenyl]sulfonylamino] thiazole-4-carboxylic acid;
- 5-[[4-[3-(4,5-dihydro-1H-imidazol-2-yl)propanoylamino]-3-fluoro-phenyl] sulfonylamino]thiazole-4-carboxylic acid ;
- 5-[[2-[(2-guanidinoacetyl)amino]thiazol-5-yl]sulfonylamino]thiazole-4-carboxylic acid;
- 5-[[4-[[2-[(N-cyanocarbamimidoyl)amino]acetyl]amino]-3-fluoro-phenyl] sulfonylamino]thiazole-4-carboxylic acid;
- 5-[[3-fluoro-4-(guanidinocarbamoylamino)phenyl]sulfonylamino]thiazole-4-carboxylic acid;
- 5-[[3-fluoro-4-[[2-(morpholine-4-carboximidoylamino)acetyl]amino]phenyl] sulfonylamino]thiazole-4-carboxylic acid;
- 5-[[4-[(3-amino-3-imino-2-methyl-propanoyl)amino]-3-fluoro-phenyl] sulfonylamino]thiazole-4-carboxylic acid;
- 5-[[4-[[2-(4,5-dihydro-1H-imidazol-2-yl)acetyl]amino]-3-fluoro-phenyl] sulfonylamino]thiazole-4-carboxylic acid;
- 5-[[4-(carbamimidoylcarbamoylamino)-3-fluoro-phenyl]sulfonylamino]thiazole-4-carboxylic acid;
- 5-[[4-[[(2R)-2-guanidinopropanoyl]amino]phenyl]sulfonylamino]thiazole-4-carboxylic acid;
- 5-[[3,5-difluoro-4-[(2-guanidinoacetyl)amino]phenyl]sulfonylamino]thiazole-4-carboxylic acid;
- 5-[[4-[(4-amino-4-imino-butanoyl)amino]-3-fluoro-phenyl]sulfonylamino]thiazole-4-carboxylic acid;
- 5-[[4-[[2-(4,5-dihydro-1H-imidazol-2-ylamino)acetyl]amino]-2,5-difluoro-phenyl] sulfonylamino]thiazole-4-carboxylic acid;
- 5-[[2,5-difluoro-4-[(2-guanidinoacetyl)amino]phenyl]sulfonylamino]thiazole-4-carboxylic acid;
- 5-[[3-fluoro-4-[[2-[(N-methylcarbamimidoyl)amino]acetyl]amino]phenyl] sulfonylamino]thiazole-4-carboxylic acid;
- 5-[[3-fluoro-4-[[2-(2-iminoimidazolidin-1-yl)acetyl]amino]phenyl]sulfonylamino] thiazole-4-carboxylic acid;
- 5-[[4-[[2-[carbamimidoyl(methyl)amino]acetyl]amino]-3-fluoro-phenyl] sulfonylamino]thiazole-4-carboxylic acid;
- 5-[[4-[[2-[[N-(2-aminoethyl)carbamimidoyl]amino]acetyl]amino]-3-fluoro-phenyl] sulfonylamino]thiazole-4-carboxylic acid;
- 5-[[5-fluoro-6-[(2-guanidinoacetyl)amino]-3-pyridyl]sulfonylamino]thiazole-4-carboxylic acid;
- 5-[[3-fluoro-4-(3-guanidinopropanoylamino)phenyl]sulfonylamino]thiazole-4-carboxylic acid;
- 5-[[4-[(3-amino-3-imino-propanoyl)amino]-3-fluorophenyl]sulfonylamino]thiazole-4-carboxylic acid;
- 5-[[3,5-difluoro-4-(guanidinocarbamoylamino)phenyl]sulfonylamino]thiazole-4-carboxylic acid;
- 5-[[3-fluoro-4-[(2-guanidinoacetyl)amino]phenyl]sulfonylamino]thiazole-4-carboxylic acid; and
- 5-[[4-[(2-guanidinoacetyl)amino]phenyl]sulfonylamino]thiazole-4-carboxylic acid; and pharmaceutically acceptable salts thereof.

**21.** A pharmaceutical composition comprising a compound according to any one of claims 1 to 14 or 16 to 20 together with at least one pharmaceutically acceptable carrier or diluent and optionally further comprising an antibiotic agent.

**22.** A product comprising a compound according to any one of claims 1 to 14 or 16 to 20 in combination with an antibiotic agent.

**23.** A composition according to claim 21 or a product according to claim 22 wherein the antibiotic agent is a β-lactam antibiotic.

**24.** A composition or product according to claim 23 wherein the β-lactam antibiotic is selected from carbapenems, penicillins, cephalosporins and penems.

**25.** A compound according to any one of claims 1 to 14 or 16 to 20, or a composition or product according to any one of claims 21 to 24 for use in medicine.

**26.** A compound according to any one of claims 1 to 14 or 16 to 20, or a composition or product according to any one of claims 21 to 24 for use in the removal or reduction of antibiotic resistance in Gram-negative bacteria.

**27.** A compound according to any one of claims 1 to 14 or 16 to 20, or a composition or product according to any one of claims 21 to 24 for use in the treatment or prevention of bacterial infection.

**28.** A compound, composition or product for use according to claim 26 or 27 wherein the Gram-negative bacteria are selected from Enterobacteriaceae, Pseudomonadaceae and Moraxellaceae, or the bacterial infection is caused by bacteria selected from Enterobacteriaceae, Pseudomonadaceae and Moraxellaceae.

**29.** A compound, composition or product for use according to claim 28 wherein the bacteria selected from Enterobacteriaceae, Pseudomonadaceae and Moraxellaceae are selected from *Klebsiella pneumonia, Escherichia coli, Pseudomonas aeruginosa, Burkholderia cepacia* and *Acinetobacter baumannii.*

**30.** A compound, composition or product for use according to claim 27 wherein the bacterial infection is caused by Carbapenem Resistant Enterobacteriaceae.

Europäisches Patentamt
European Patent Office
Office européen des brevets

# EUROPEAN SEARCH REPORT

Application Number
EP 17 30 5973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2014/198849 A1 (ANTABIO SAS [FR]) 18 December 2014 (2014-12-18) * claims; examples * ----- | 1 | INV. C07D417/12 C07D277/30 A61K31/426 A61K31/427 A61P31/04 |
| | | | TECHNICAL FIELDS SEARCHED (IPC) |
| | | | C07D |

The present search report has been drawn up for all claims

1

| Place of search | Date of completion of the search | Examiner |
|---|---|---|
| Berlin | 24 April 2018 | Beyss-Kahana, Ellen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 30 5973

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2018

| Patent document cited in search report | Publication date | Patent family member(s) | Publication date |
|---|---|---|---|
| WO 2014198849 A1 | 18-12-2014 | CN 105658631 A | 08-06-2016 |
| | | EP 3008045 A1 | 20-04-2016 |
| | | JP 2016520658 A | 14-07-2016 |
| | | US 2016272601 A1 | 22-09-2016 |
| | | WO 2014198849 A1 | 18-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014198849 A
- WO 2013123444 A, K. Ashton


### Non-patent literature cited in the description


- **P. PATEL et al.** *Bioorg Med Chem Lett,* 2007, vol. 17, 6610
- **C-M. PARK et al.** *J Med Chem,* 2008, vol. 51, 6902